# EUROPEAN PATENT SPECIFICATION

(11) **EP 1 866 323 B1**
(45) Date of publication and mention of the grant of the patent: **31.05.2017**
(21) Application number: 06719897.8
(22) Date of filing: 30.01.2006
(51) Int. Cl.: C07H 7/033, A61K 31/7012, A61K 31/7048, A61P 9/00, A61P 9/08

(54) **GLUCURONIDATED NEBIVOLOL**
GLUCURONIDIERTES NEBIVOLOL
DERIVE GLUCURONIDE DE NEBIVOLOL

(30) Priority: 31.01.2005 US 648552 P; 03.01.2006 US 755755 P
(43) Date of publication of application: 19.12.2007
(73) Proprietor: MYLAN LABORATORIES, INC, Morgantown, WV 26504 (US)
(72) Inventor: O'DONNELL, John, P., Morgantown, WV 26505 (US); OWENS, Walter, Morgantown, WV 26508 (US); DUNCAN, Joseph, Morgantown, WV 26508 (US); SHAW, Andrew, Morgantown, WV 26508 (US)
(74) Representative: Samson & Partner Patentanwälte mbB
(86) International application number: PCT/US2006/003253
(87) International publication number: WO 2006/083780

(56) References cited:
- EP-A- 0 334 429
- COCKCROFT JOHN: "Nebivolol: a review." EXPERT OPINION ON PHARMACOTHERAPY. APR 2004, vol. 5, no. 4, April 2004 (2004-04), pages 893-899, XP009070520 ISSN: 1465-6566
- RITTER JOSEPH K: "Roles of glucuronidation and UDP-glucuronosyltransferases in xenobiotic bioactivation reactions" CHEMICO-BIOLOGICAL INTERACTIONS, vol. 129, no. 1-2, 1 December 2000 (2000-12-01), pages 171-193, XP002393723 ISSN: 0009-2797
- BROEDERS MARTIJN A W ET AL: "Nebivolol: A third-generation beta-blocker that augments vascular nitric oxide release: Endothelial beta2-adrenergic receptor-mediated nitric oxide production" CIRCULATION, vol. 102, no. 6, 8 August 2000 (2000-08-08), pages 677-684, XP002393075 ISSN: 0009-7322
- SHAW A A ET AL: "Pharmacokinetic disposition of nebivolol in extensive and poor CYP2D6 metabolizers" CLINICAL PHARMACOLOGY & THERAPEUTICS, MOSBY-YEAR BOOK, ST LOUIS, MO, US, vol. 77, no. 2, February 2005 (2005-02), page P77, XP004802605 ISSN: 0009-9236

## Description

This application claims priority under 35 USC §119 to U.S. Provisional Application No. 60/648,552, filed January 31,2005 and to U.S. Provisional Application No. 60/755,755, filed January 3, 2006.

### FIELD OF THE INVENTION

Metabolites of nebivolol possess pharmacological properties that may be beneficial in treating a variety of diseases, such as cardiovascular diseases, which could include hypertension, atherosclerosis, and congestive heart failure. In particular, nebivolol and its key metabolites have effects on NO metabolism, independent of β₁-selective interactions and the favorable effects of nebivolol metabolites on endothelial NO release has implications for its use in the treatment of cardiovascular disorders. This invention describes glucuronidated nebivolol metabolites, compositions comprising at least one glucuronidated nebivolol metabolite and methods of using such compositions to treat and/or prevent cardiovascular diseases or disorders.

### BACKGROUND OF THE INVENTION

The normal production of endothelial nitric oxide is important for maintenance of vascular function. Increasing endothelial NO release results in a reduction in smooth muscle cell contractility and decreased vascular resistance. Under conditions of hypertension and hyperlipidemia, however, endothelial dysfunction produces a marked decrease in NO release, resulting in: 1) increased vasoconstriction and vasospasm; 2) greater monocyte and LDL infiltration; 3) proliferation of vascular smooth cells; 4) increased oxidative stress; and 5) increased platelet aggregation. Substances that restore endothelial function and NO metabolism may have benefit in the treatment of various cardiovascular disorders, including coronary artery disease and heart failure.

EP 0 334 429 A1 discloses the compound nebivolol. Ritter, Joseph K., Chemico-biological Interactions Vol. 129, No. 1-2 (2000) 171-193, discloses glucuronide derivatives of a number of drugs which show a pharmaceutical activity.

### SUMMARY OF THE INVENTION

Glucuronidated nebivolol metabolites increase NO release from human endothelial cell preparations in a concentration dependent fashion following acute and also possibly chronic administration. This invention provides glucuronidated nebivolol metabolites and compositions comprising at least one glucuronidated metabolite of nebivolol and optionally at least one other active compound in a pharmaceutically acceptable carrier. In addition, the present disclosure provides methods of treating and/or preventing vascular diseases, by administering at least one glucuronidated metabolite of nebivolol that is capable of releasing a therapeutically effective amount of nitric oxide to a targeted site affected by the vascular disease.

### BRIEF DESCRIPTION OF THE FIGURES:

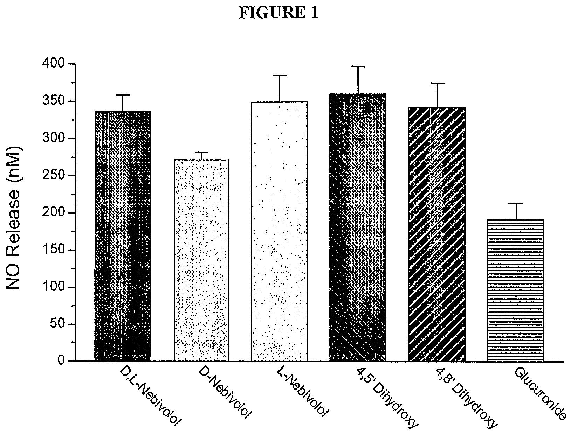

FIGURE 1: The effects of nebivolol and metabolites on peak NO release from human endothelial cells following acute treatment (50 µM treatment).

In particular, the invention comprises the following aspects and embodiments
In a first aspect the invention relates to a pharmaceutical composition comprising at least one diastereoisomer of a glucuronidated nebivolol metabolite having a formula below: 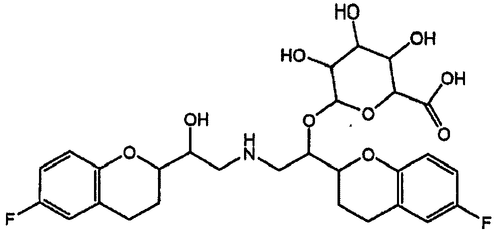 or a pharmaceutically acceptable salt thereof and a pharmaceutically-acceptable carrier.

In a further aspect the invention relates to at least one diastereomer of a glucuronidated nebivolol metabolite for use as a medicament in treating and/or preventing cardiovascular disease as it is defined in claim 1.

The cardiovascular disease may be selected from the group consisting of congestive heart failure, hypertension, pulmonary hypertension, myocardial and cerebral infarctions, atherosclerosis, atherogenesis, thrombosis, ischemic heart disease, post- angioplasty restenosis, coronary artery diseases, renal failure, stable, unstable and variant (Prinzmetal) angina, cardiac edema, renal insufficiency, nephrotic edema, hepatic edema, stroke, transient ischemic attacks, cerebrovascular accidents, restenosis, controlling blood pressure in hypertension, platelet adhesion, platelet aggregation, smooth muscle cell proliferation, pulmonary edema, vascular complications associated with the use of medical devices including stents, wounds associated with the use of medical devices, pulmonary thromboembolism, cerebral thromboembolism, thrombophlebitis, thrombocytopenia and bleeding disorders.

In particular the cardiovascular disease may be selected from the group consisting of congestive heart failure, hypertension, restenosis and atherosclerosis and the medicament may be administered intravenously, orally, bucally, parenterally, by inhalation or transdermally.

In a further aspect the invention relates to a pharmaceutical composition comprising at least one diastereomer of a glucuronidated nebivolol metabolite having a formula below: 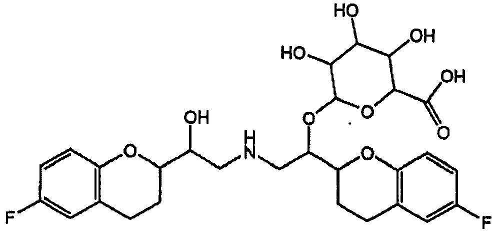 or a pharmaceutically acceptable salt thereof, and at least one additional cardiovascular agent.

The cardiovascular agent may be an ARB.

The ARB may be selected from the group consisting of olmesartan, candesartan, eprosartan, irbesartan,
losartan, valsartan, and mixtures thereof.

In a further aspect the invention relates to a pharmaceutical composition comprising at least one diastereomer of a glucuronidated nebivolol metabolite having a formula below: 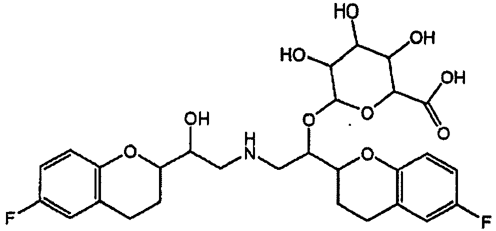 or a pharmaceutically acceptable salt thereof and at least one additional cardiovascular agent, and a pharmaceutically-acceptable carrier.

The cardiovascular agent may be an ARB.

The ARB may be selected from the group consisting of olmesartan, candesartan, eprosartan, irbesartan, losartan, valsartan, and mixtures thereof.

In a further aspect the invention relates to the above pharmaceutical compositions for use as a medicament in treating and/or preventing a cardiovascular disease. The cardiovascular disorder may be selected from the group consisting of congestive heart failure, hypertension, pulmonary hypertension, myocardial and cerebral infarctions, atherosclerosis, atherogenesis, thrombosis, ischemic heart disease, post-angioplasty restenosis, coronary artery diseases, renal failure, stable, unstable and variant (Prinzmetal) angina, cardiac edema, renal insufficiency, nephrotic edema, hepatic edema, stroke, transient ischemic attacks, cerebrovascular accidents, restenosis, controlling blood pressure in hypertension, platelet adhesion, platelet aggregation, smooth muscle cell proliferation, pulmonary edema, vascular complications associated with the use of medical devices including stents, pulmonary thromboembolism, cerebral thromboembolism, thrombophlebitis, thrombocytopenia and bleeding disorders. In particular the cardiovascular disease may be selected from the group consisting of congestive heart failure, hypertension, restenosis and atherosclerosis and the medicament is in dosage unit form, wherein each unit contains a predetermined quantity of the nebivolol metabolite or metabolites to produce the desired therapeutic effect, in association with a pharmaceutical carrier, wherein the composition is administered intravenously, orally bucally, parenterally, by inhalation or transdermally.

### BRIEF DESCRIPTION OF THE FIGURES:

FIGURE 1: The effects of nebivolol and metabolites on peak NO release from human endothelial cells following acute treatment (50 µM treatment).
FIGURE 2: Shows the concentration-response following acute treatment with nebivolol metabolites.
FIGURE 3: Comparison of the effects of acute treatment with nebivolol and its metabolites on NO release from human endothelial cells (500 nM) in a dose-dependency relationship.
FIGURE 4: Shows the amount of NO released as measured following acute drug treatment (1.0 µM) of HUVECs isolated from White (open bars) and Black American (solid bars) donors. The amount of NO released was measured following acute drug treatment (1.0 µM) of HUVEC isolated from White (open bars) and Black American (solid bars) donors. The drugs used in this study were nebivolol, *d*-nebivolol, *l*-nebivolol and four stereoselective glucuronide metabolites of nebivolol. Values are mean ± S.D. (n=5).
FIGURE 5: Is the synthetic scheme for the preparation of the glucuronidated nebivolol metabolites

### DETAILED DESCRIPTION OF THE INVENTION

### Definitions

For convenience, before further description of the present invention, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

The term "nebivolol metabolite" includes all possible diastereomers of the glucuronidated nebivolol.

The articles "a" and "an" are used herein to refer to one or to more than one (i.e., to at least one) of the grammatical object of the article. By way of example, "an element" means one element or more than one element.

"About the same time" means that within about thirty minutes of administering one compound (nebivolol) to the patient, the other active compound(s) is/are administered to the patient. "About the same time" also includes simultaneous administration of the compounds.

The phrase "angiotensin converting enzyme inhibitor" or "ACE inhibitor" as used herein refers to a compound that inhibits any enzyme from converting angiotensin to any other form. ACE inhibitors are effective antihypertensive agents that competitively inhibit angiotensin converting enzymes and prevent the conversion of angiotensin I to angiotensin II. Non-limiting examples of ACE inhibitors include alacepril, benazepril, captopril, ceronapril, cilazapril, delapril, enalapril, enalaprilat, fosinopril, imidapril, lisinopril, perindopril, quinapril, ramiprilat, ramipril, spirapril, temocapril, and trandolapril. Other ACE inhibitors may be identified using standard assaying techniques known to one of ordinary skill in the art.

The phrase "angiotensin II receptor antagonist" or "ARB" refers to a compound that binds to a receptor site on angiotensin II but does not cause any physiological changes unless another receptor ligand is present. ARBs are effective antihypertensive agents that have a very favorable side effect profile. Their mechanism of action is related to the selective blocking of the vasoconstrictor and aldosterone-secreting effects of angiotensin. Non-limiting examples of ARBs include candesartan, eprosartan, irbesartan, losartan, and valsartan. Other ARBs may be identified using standard assaying techniques known to one of ordinary skill in the art.

The term "antagonist" is art-recognized and refers to a compound that binds to a receptor site, but does not cause a physiological change unless another receptor ligand is present.

The term "bioavailable" is art-recognized and refers to a form of the subject invention that allows for it, or a portion of the amount administered, to be absorbed by, incorporated to, or otherwise physiologically available to a subject or patient to whom it is administered.

"Black" refers to a person of African descent or an African-American person but is not necessarily limited to those of African origin (e.g., Carribean).

"Therapeutically effective amount" refers to the amount of the compound and/or composition that is effective to achieve its intended purpose.

The phrase "cardiovascular agent" or "cardiovascular drug" refers to a therapeutic compound that is useful for treating or preventing a cardiovascular disease. Non-limiting examples of suitable compounds include (1) thiazide diuretics (including chlorothiazide (Diuril), chlorthalidone, hydrochlorothiazide, polythiazide (Renese), indapamide (Lozol) and metolazone (Mykrox, Zaroxolyn)); (2) loop diuretics (including bumetanide (Bumex) and furosemide (Lasix), torsemide (Demadex)); (3) potassium-sparing diuretics (including amiloride (Midamor) and triamterene (Dyrenium)); (4) aldosterone receptor blockers (including eplerenone (Inspra) and spironolactone (Aldactone)); (5) beta blockers (including atenolol (Tenormin), betaxolol (Kerlone), bisoprolol (Zebeta), metoprolol (Lopressor), metoprolol extended release (Toprol XL), nadolol (Corgard), propranolol (Inderal), propranolol long acting (Inderal LA) and timolol (Blocadren)); (6) beta blockers with intrinsic sympathomimetic activity (including acebutolol (Sectral), penbutolol (Levatol) and pindolol); (7) combined alpha and beta blockers (including carvedilol (Coreg) and labetalol (Normodyne, Trandate)); (8) ACE inhibitors (including benazepril (Lotensin), captopril (Capoten), enalapril (Vasotec), fosinopril (Monopril), lisinopril (Prinivil, Zestril), moexipril (Univasc), perindopril (Aceon), quinapril (Accupril), ramipril (Altace) and trandolapril (Mavik)); (9) angiotensin II antagonists (candesartan (Atacand), eprosartan (Teveten), irbesartan (Avapro), losartan (Cozaar), olmesartan (Benicar), (Micardis) and valsartan (Diovan); (10) calcium channel blockers- non-dihydropyridines (including diltiazem extended release (Cardizem CD, Dilacor XR, Tiazac), diltiazem extended release (Cardizem LA), verapamil, immediate release (Calan, Isoptin), verapamil long acting (Calan SR, Isoptin SR) and verapamil- Coer (Covera HS, Verelan PM)); (11) calcium channel blockers- dihydropyridines (including amlodipine (Norvasc), felodipine (Plendil), isradipine (DynaCirc CR), nicardipine sustained release (Cardene SR), nifedipine long-acting (Adalat CC, Procardia XL) and nisoldipine (Sular)); (12) alpha₁-blockers (including doxazosin (Cardura), prazosin (Minipress) and terazosin (Hytrin)); (13) central alpha₂-agonists and other centrally acting drugs (including clonidine (Catapres), clonidine patch (Catapres-TTS), methyldopa (Aldomet), reserpine, and guanfacine); (14) direct vasodilators (including hydralazine (Apresoline) and minoxidil (Loniten); (15) ACEIs and CCBs (including amlodipine/benazepril hydrochloride, enalapril maleate/felodipine and trandolapril/verapamil); (16) ACEIs and diuretics (including benazepril/hydrochlorothiazide, captopril/hydrochlorothiazide, enalapril maleate/hydrochlorothiazide, lisinopril/hydrochlorothiazide, moexipril HCl/hydrochlorothiazide and quinapril/hydrochlorothiazide; (17) ARBs and diuretics (including candesartan cilexetil/hydrochlorothiazide, eprosartan mesylate/. hydrochlorothiazide, irbesartan/ hydrochlorothiazide, losartan potassium/ hydrochlorothiazide, telmisartan/ hydrochlorothiazide, valsartan/ hydrochlorothiazide; (18) beta blockers and diuretics (including atenolol/chlorthalidone, bisoprolol fumarate/ hydrochlorothiazide, metoprolol tartrate/ hydrochlorothiazide, nadolol/bendrofluthiazide and timolol maleate/ hydrochlorothiazide; (19) centrally acting drug and diuretic (including methyldopa/ hydrochlorothiazide, reserpine/chlorothiazide and reserpine/ hydrochlorothiazide); (20) diuretic and diuretic (amiloride HCl/ hydrochlorothiazide, spironolactone/ hydrochlorothiazide and triamterene/ hydrochlorothiazide.

Cardiovascular disease or disorder refers to any cardiovascular disease or disorder known in the art, including, but not limited to, wherein the cardiovascular disease is selected from the group consisting of congestive heart failure, hypertension, pulmonary hypertension, myocardial and cerebral infarctions, atherosclerosis, atherogenesis, thrombosis, ischemic heart disease, post-angioplasty restenosis, coronary artery diseases, renal failure, stable, unstable and variant (Prinzmetal) angina, cardiac edema, renal insufficiency, nephrotic edema, hepatic edema, stroke, transient ischemic attacks, cerebrovascular accidents, restenosis, controlling blood pressure in hypertension, platelet adhesion, platelet aggregation, smooth muscle cell proliferation, pulmonary edema, and vascular complications associated with the use of medical devices.

The term "combination" refers to two or more different active agents which are administered at roughly about the same time (for example, where the active agents are in a single pharmaceutical preparation) or at different times (for example, one agent is administered to the subject before the other).

The terms "drug," "pharmaceutically active agent," "bioactive agent," "therapeutic agent," and "active agent" may be used interchangeably and refer to a substance, such as a chemical compound or complex, that has a measurable beneficial physiological effect on the body, such as a therapeutic effect in treatment of a disease or disorder, when administered in an effective amount. Further, when these terms are used, or when a particular active agent is specifically identified by name or category, it is understood that such recitation is intended to include the active agent per se, as well as pharmaceutically acceptable, pharmacologically active derivatives thereof, or compounds significantly related thereto, including without limitation, salts, pharmaceutically acceptable salts, N-oxides, prodrugs, active metabolites, isomers, fragments, analogs, solvates hydrates, radioisotopes, etc.

The phrase "effective amount" refers to that amount of a substance that produces some desired local or systemic effect at a reasonable benefit/risk ratio applicable to any treatment. The effective amount of such substance will vary depending upon the subject and disease condition being treated, the weight and age of the subject, the severity of the disease condition, the manner of administration and the like, which can readily be determined by one of ordinary skill in the art.

"Endothelial dysfunction" refers to the impaired ability of in any physiological processes carried out by the endothelium, in particular, production of nitric oxide regardless of cause. It may be evaluated by, such as, for example, invasive techniques, such as, for example, coronary artery reactivity to acetylcholine or methacholine, and the like, or by noninvasive techniques, such as, for example, blood flow measurements, brachial artery flow dilation using cuff occlusion of the arm above or below the elbow, brachial artery ultrasonography, imaging techniques, measurement of circulating biomarkers, such as, asymmetric dimethylarginine (ADMA), and the like. For the latter measurement the endothelial-dependent flow-mediated dilation will be lower in patients diagnosed with an endothelial dysfunction.

The phrase "endothelial nitric oxide synthase" or "eNOS" refers to enzymes that produce nitric oxide.

The phrase "nebivolol composition" refers to a composition comprising nebivolol and the two are used interchangeably. Nebivolol is a mixture of *d* and *l* isomers of α,α'-[iminobismethylene]bis[6-fluoro-3,4-dihydro-2H-1-benzopyran-2-methanol]. The composition may include at least one other cardiovascular agent or at least one pharmaceutically acceptable carrier or both.

The term "pharmaceutically acceptable salts" is art-recognized and refers to the relatively non-toxic, inorganic and organic acid addition salts of compounds, including, for example, those contained in compositions of the present invention.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be acceptable in the sense of being compatible with the subject composition and its components and not injurious to the patient. Some examples of materials which may serve as pharmaceutically acceptable excipients include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) IV fluids, including but not limited to Ringer's solution, 5% dextrose in water, and half normal saline; (19) ethyl alcohol; (20) phosphate buffer solutions; and (21) other non-toxic compatible substances employed in pharmaceutical formulations.

"Patient" refers to animals, preferably mammals, most preferably humans, and includes males and females.

"Quality of life" refers to one or more of a person's ability to walk, climb stairs, do errands, work around the house, participate in recreational activities, and/or not requiring rest during the day, and/or the absence of sleeping problems or shortness of breath.

The term "prophylactic" or "therapeutic" treatment is art-recognized and refers to administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The term "structure-activity relationship" or "(SAR)" is art-recognized and refers to the way in which altering the molecular structure of a drug or other compound alters its interaction with a receptor, enzyme, nucleic acid or other target and the like.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

The term "synthetic" is art-recognized and refers to production by in vitro chemical or enzymatic synthesis.

The phrase "therapeutic effect" is art-recognized and refers to a local or systemic effect in animals, particularly mammals, and more particularly humans caused by a pharmacologically active substance. The term thus means any substance intended for use in the diagnosis, cure, mitigation, treatment or prevention of disease or in the enhancement of desirable physical or mental development and/or conditions in an animal or human. The phrase "therapeutically-effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art.

The term "treating" is art-recognized and refers to curing as well as ameliorating at least one symptom of any condition or disease.

The parent nebivolol compound and its glucuronidated metabolites can be synthesized by one skilled in the art following the methods described in, for example, U.S. Pat. Nos. 4,654,362, 5,759,580, 6,075,046, 6,545,040 and in EP 0 145 067, EP 0 334 429, and in WO 95/22325 and WO 96/19987; Van Lommen et al., J. Pharm. Belg., 45(6): 355-360 (1990); Chandrasekhar, S. et al., Tetrahedron, 56(34): 6339-6344 (2000); and Kendrick et al., J. Chromatogram. A., 729: 341-354 (1996).

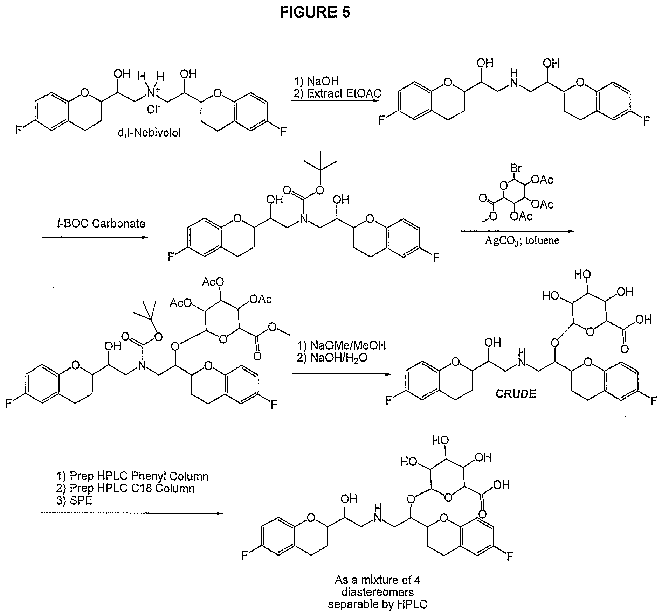

The glucuronidated metabolites may also be prepared by the synthetic procedures found in Figure 5. Reactions are performed in solvents appropriate to the reagents, and materials used are suitable for the transformations being effected. It is understood by one skilled in the art of organic synthesis that the functionality present in the molecule must be consistent with the chemical transformation proposed. This will, on occasion, necessitate judgment by the scientist as to the order of synthetic steps, protecting groups required, and deprotection conditions. Substituents on the starting materials may be incompatible with some of the reaction conditions required in some of the methods described, but alternative methods and substituents compatible with the reaction conditions will be readily apparent to one skilled in the art. The use of sulfur and oxygen protecting groups is known in the art for protecting thiol and alcohol groups against undesirable reactions during a synthetic procedure and many such protecting groups are known, e.g., T. H. Greene and P. G. M. Wuts, Protective Groups in Organic Synthesis, John Wiley & Sons, New York (1999).

The glucuronidated metabolites have basic properties and, consequently, they may be converted to their therapeutically active non-toxic acid addition salt forms by treatment with appropriate acids, such as, for example, inorganic acids, such as hydrohalic acid, e.g. hydrochloric, hydrobromic and the like, and sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propane-tricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely, the salt form can be converted by treatment with alkali into the free base form.

### Glucuronidated Nebivolol Metabolites

Glucuronidation is a pathway that the body utilizes to make a xenobiotic moiety more hydrophilic and more easily eliminated from the body. Typically, glucuronidation is utilized by the body as a detoxification step. There are only a few instances where glucuronidation produces a positive pharmacologically active species. Such examples include glucuronidation of ezetimibe, morphine and retinoids. Nebivolol glucuronides fall into this rare instance where glucuronidation does not produce an inactive or toxic substance. The nebivolol glucuronides of the present invention are capable of releasing NO from human umbilical vein endothelial cells. Figures 1-3 depict nebivolol glucuronide concentration versus amount of nitric oxide being released. Nebivolol glucuronide metabolites are also thought to function as anti-oxidants and prevent lipid peroxidation. Since these compounds have the capacity to increase endothelial nitric oxide concentrations, they may have added benefits in special populations such as diabetics, chronic smokers, African-Americans and people suffering from obesity.

The glucuronidated nebivolol metabolites may be used for the treatment and/or prevention of cardiovascular diseases associated with endothelial dysfunction. These disorders include, but are not limited to hypertension, coronary artery disease, congestive heart failure, and atherosclerosis. Glucuronidated nebivolol metabolites could be used as a mono-therapy to treat or prevent the aforementioned disorders or in combination with other appropriate pharmaceutical agents either in the same dosage form/product or concomitantly administered as separate dosage forms/products to adequately treat/prevent the aforementioned disorders.

The parent nebivolol compound and its metabolites can be glucuronidated through one or more sites such as oxygen sites (via hydroxyl condensation).

The glucuronidated nebivolol metabolites of this invention exist as four different diastereomers. It is to be understood that the invention envisions and includes within its scope the pure diastereomers and mixtures thereof. The structures of the metabolites are as follows:

| Metabolite Code | Structure |
|---|---|
| **G-UDa** (*d*-Nebivolol-11-*O-*β-D-glucuronide) | 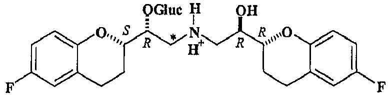 |
| **G-UDb** (*l*-Nebivolol-11-*O*-β-D-glucuronide | 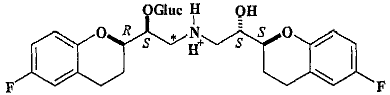 |
| **G-UDc** (*d*-Nebivolol-11'-*O*-β-D-glucuronide | 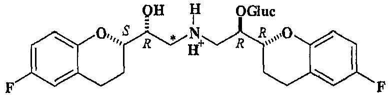 |
| **G-UDd** (*l*-Nebivolol-11'-*O-*β-D-glucuronide | 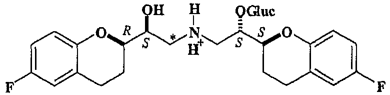 |

### Compositions

In part, the present disclosure features compositions comprising a nebivolol metabolite and at least one other active agent, for instance, a cardiovascular agent. The amount of each cardiovascular agent present in the compositions may vary depending on a number of variables such as age, weight, gender, and health related issues. In general, the dosage of the cardiovascular agents will generally be in the range of about 0.01 ng to about 10 g per kg body weight, specifically in the range of about 1 ng to about 0.1 g per kg, and more specifically in the range of about 100 ng to about 10 mg per kg. In another embodiment, the amount of nebivolol in the compositions of the present invention may be anywhere from about 0.125 mg to about 40 mg. When the other cardiovascular agent is an ACE inhibitor, the amount of the ACE inhibitor may be anywhere from 0.5 mg to about 80 mg. When the other cardiovascular agent is an ARB, the amount of ARB may be anywhere from about 1 mg to about 1200 mg. The amount of the other cardiovascular agent will depend in part on the particular cardiovascular agent used.

In addition to ACE inhibitors and ARBs, additional cardiovascular agents include, but are not limited to adrenergic blockers, adrenergic agonists, agents for pheochromocytoma, antianginal agents, antiarrhythmics, antiplatelet agents, anticoagulants, antihypertensives, antilipemic agents, antidiabetics, antiinflammatory agents, calcium channel blockers, CETP inhibitors, COX-2 inhibitors, direct thrombin inhibitors, diuretics, endothelin receptor antagonists, HMG Co-A reductase inhibitors, inotropic agents, rennin inhibitors, vasodilators, vasopressors, AGE crosslink breakers (advanced glycosylation end-product crosslink breakers, such as alagebrium, see USP 6,458,819), and AGE formation inhibitors (advanced glycosylation end-product formation inhibitors, such as pimagedine). Cardiovascular agents falling within these general categories are exemplified by the following:
*"Angiotensin I Converting Enzymes (ACE's) and Angiotensin II Receptor Antagonists (ARB's)"*

"Angiotensin II receptor antagonists"(ARB's) are compounds which interfere with the activity of angiotensin II by binding to angiotensin II receptors and interfering with its activity. Angiotensin I and angiotensin II are synthesized by the enzymatic renin-angiotensin pathway. The synthetic process is initiated when the enzyme renin acts on angiotensinogen, a pseudoglobulin in blood plasma, to produce the decapeptide angiotensin I. Angiotensin I is converted by angiotensin converting enzyme (ACE) to angiotensin II (angiotensin-[1-8] octapeptide). The latter is an active pressor substance which has been implicated as a causative agent in several forms of hypertension in various mammalian species, e.g., humans.

Angiotensin II receptor antagonists (ARB's) are well known and include peptide compounds and non-peptide compounds. Most angiotensin II receptor antagonists are slightly modified congeners in which agonist activity is attenuated by replacement of phenylalanine in position 8 with some other amino acid; stability can be enhanced by other replacements that slow degeneration in vivo.

Examples of angiotensin II receptor antagonists include: telmisartan; olmesartan; peptidic compounds (e.g., saralasin and related analogs); N-substituted imidazole-2-one (U.S. Pat. No. 5,087,634); imidazole acetate derivatives including 2-N-butyl-4-chloro-1-(2-chlorobenzile) imidazole-5-acetic acid (see Long et al., J. Pharmacol. Exp. Ther. 247(1), 1-7 (1988)); 4,5,6,7-tetrahydro-1H-imidazo [4,5-c]pyridine-6-carboxylic acid and analog derivatives (U.S. Pat. No. 4,816,463); N2-tetrazole beta-glucuronide analogs (U.S. Pat. No. 5,085,992); substituted pyrroles, pyrazoles, and tryazoles (U.S. Pat. No. 5,081,127); phenol and heterocyclic derivatives such as 1,3-imidazoles (U.S. Pat. No. 5,073,566); imidazo-fused 7-member ring heterocycles (U.S. Pat. No. 5,064,825); peptides (e.g., U.S. Pat. No. 4,772,684); antibodies to angiotensin II (e.g., U.S. Pat. No. 4,302,386); and aralkyl imidazole compounds such as biphenyl-methyl substituted imidazoles (e.g., EP 253,310, Jan. 20, 1988); ES8891 (N-morpholinoacetyl-(-1-naphthyl)-L-alanyl-(4, thiazolyl)-L-alanyl (35, 45)-4-amino-3-hydroxy-5-cyclo-hexapentanoyl-N-hexylamide, Sankyo Company, Ltd., Tokyo, Japan); SKF108566 (E-alpha-2-[2-butyl-1-(carboxyphenyl) methyl] 1H-imidazole-5-yl[methylane]-2-thiophenepropanoic acid, Smith Kline Beecham Pharmaceuticals, Pa.); Losartan (DUP753/MK954, DuPont Merck Pharmaceutical Company); Remikirin (RO42-5892, F. Hoffman LaRoche A G); A.sub.2 agonists (Marion Merrill Dow) and certain non-peptide heterocycles (G. D. Searle and Company). Other non-limiting examples of ARBs include candesartan, eprosartan, irbesartan, losartan, and valsartan. Other ARBs may be identified using standard assaying techniques known to one of ordinary skill in the art.

"Angiotensin converting enzyme" (ACE) is an enzyme which catalyzes the conversion of angiotensin I to angiotensin II. ACE inhibitors include amino acids and derivatives thereof, peptides, including di- and tri-peptides and antibodies to ACE which intervene in the renin-angiotensin system by inhibiting the activity of ACE thereby reducing or eliminating the formation of pressor substance angiotensin II. ACE inhibitors have been used medically to treat hypertension, congestive heart failure, myocardial infarction and renal disease. Classes of compounds known to be useful as ACE inhibitors include acylmercapto and mercaptoalkanoyl prolines such as captopril (U.S. Pat. No. 4,105,776) and zofenopril (U.S. Pat. No. 4,316,906), carboxyalkyl dipeptides such as enalapril (U.S. Pat. No. 4,374,829), lisinopril (U.S. Pat. No. 4,374,829), quinapril (U.S. Pat. No. 4,344,949), ramipril (U.S. Pat. No. 4,587,258), and perindopril (U.S. Pat. No. 4,508,729), carboxyalkyl dipeptide mimics such as cilazapril (U.S. Pat. No. 4,512,924) and benazepril (U.S. Pat. No. 4,410,520), phosphinylalkanoyl prolines such as fosinopril (U.S. Pat. No. 4,337,201) and trandolapril. Other non-limiting examples of ACE inhibitors include, but are not limited to, alacepril, benazepril, captopril, ceronapril, cilazapril, delapril, enalapril, enalaprilat, fosinopril, imidapril, lisinopril, perindopril, quinapril, ramipril, ramiprilat, spirapril, temocapril, trandolapril.

### Other classes of co-actives include the following:

### Adrenergic Blockers

Non-limiting examples of adrenergic blockers, both α- and β-adrenergic blockers, that may be used in the compositions of the present invention include Beta-adrenergic receptor blockers include, but are not limited to, atenolol, acebutolol, alprenolol, befunolol, betaxolol, bunitrolol, carteolol, celiprolol, hedroxalol, indenolol, labetalol, levobunolol, mepindolol, methypranol, metindol, metoprolol, metrizoranolol, oxprenolol, pindolol, propranolol, practolol, sotalolnadolol, tiprenolol, tolamolol, timolol, bupranolol, penbutolol, trimepranol, yohimbine, 2-(3-(1,1-dimethylethyl)-amino-2-hydroxypropoxy)-3-pyridenecarbonitrilHCl, 1-butylamino-3-(2,5-dichlorophenoxy)-2-propanol, 1-isopropylamino-3-(4-(2-cyclopropylmethoxyethyl)phenoxy)-2-propanol, 3-isopropylamino-1-(7-methylindan-4-yloxy)-2-butanol, 2-(3-t-butylamino-2-hydroxy-propylthio)-4-(5-carbamoyl-2-thienyl)thiazole, 7-(2-hydroxy-3-t-butylaminpropoxy)phthalide. The above-identified compounds can be used as isomeric mixtures, or in their respective levorotating or dextrorotating form.

### Adrenergic Agonists

Non-limiting examples of adrenergic agonists, both α- and β-adrenergic agonists, that may be used in the compositions of the present invention include adrafinil, adrenalone, albuterol, amidephrine, apraclonidine, bitolterol, budralazine, carbuterol, clenbuterol, clonidine, clorprenaline, clonidine, cyclopentamine, denopamine, detomidine, dimetofrine, dioxethedrine, dipivefrin, dopexamine, ephedrine, epinephrine, etafedrine, ethylnorepinephrine, fenoterol, fenoxazoline, formoterol, guanabenz, guanfacine, hexoprenaline, hydroxyamphetamine, ibopamine, indanazoline, isoetharine, isometheptene, isoproterenol, mabuterol, mephentermine, metaproterenol, metaraminol, metizoline, methoxamine, methylhexaneamine, methoxyphenamine, midodrine, modafinil, moxonidine, naphazoline, norepinephrine norfenefrine, octodrine, octopamine, oxyfedrine, oxymetazoline, phenylephrine hydrochloride, phenylpropanolamine hydrochloride, phenylpropylmethylamine, pholedrine, pirbuterol prenalterol, procaterol, propylhexedrine, protokylol, pseudoephedrine, reproterol, rilmenidine, rimiterol, ritodrine, salmeterol, solterenol, synephrine, talipexole, terbutaline, tetrahydrozoline, tiamenidine, tramazoline, tretoquinol, tuaminoheptane, tulobuterol, tymazoline, tyramine, xamoterol, xylometazoline, and mixtures thereof.

### Agents for Pheochromocytoma

Include but are not limited to chemotherapeutics.

### Antianginal agents

Include but are not limited to amlodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, nitrates (including but not limited to glyceryl trinitrate (GTN, nitroglycerin, Nitro-Bid), isosorbide-5-mononitrate (5-ISMN, Ismo), amyl nitrate and nicorandil (Icorel)), primidolol, ranolazine hydrochloride, tosifen, verapamil hydrochloride).

### Antiarrhythmics

Non-limiting examples of antiarrhythmics that may be used in the compositions of the present invention include acebutolol, acecainide, adenosine, ajmaline, alprenolol, amiodarone, amoproxan, aprindine, aprotinolol, atenolol, azimilide, bevantolol, bidisomide, bretylium tosylate, bucumolol, butetolol, bunaftine, bunitrolol, bupranolol, butidrine hydrochloride, butobendine, capobenic acid, carazolol, carteolol, cifenline, cloranolol, disopyramide, dofetilide, encainide, esmolol, flecainide, hydroquinidine, ibutilide, indecainide, indenolol, ipratropium bromide, lidocaine, lorajmine, lorcainide, meobentine, mexiletine, moricizine, nadoxolol, nifenaolol, oxprenolol, penbutolol, pentisomide, pilsicainide, pindolol, pirmenol, practolol, prajmaline, procainamide hydrochloride, pronethalol, propafenone, propranolol, pyrinoline, quinidine, sematilide, sotalol, talinolol, tilisolol, timolol, tocainide, verapamil, viquidil, xibenolol, and mixtures thereof.

### Antiplatelet Agents

Non-limiting examples of antiplatelet agents that may be used in the compositions of the present invention include clopidogrel, dipyridamole, abciximab, and ticlopidine.

### Anticoagulants

Anti-coagulant agents are agents which inhibit the coagulation pathway by impacting negatively upon the production, deposition, cleavage and/or activation of factors essential in the formation of a blood clot. Non-limiting examples of anticoagulants (i.e. coagulation-related therapy) that may be used in the compositions of the present invention include Aggrenox, Agrylin, Amicar, Anturane, Arixtra, Coumadin, Fragmin, Heparin Sodium, Lovenox, Mephyton, Miradon, Persantine, Plavix, Pletal, Ticlid, Trental, Warfarin. Other "anti-coagulant" and/or "fibrinolytic" agents include Plasminogen (to plasmin via interactions of prekallikrein, kininogens, Factors XII, XIIIa, plasminogen proactivator, and tissue plasminogen activator[TPA]) Streptokinase; Urokinase: Anisoylated Plasminogen-Streptokinase Activator Complex; Pro-Urokinase; (Pro-UK); rTPA (alteplase or activase; r denotes recombinant); rPro-UK; Abbokinase; Eminase; Streptase Anagrelide Hydrochloride; Bivalirudin; Dalteparin Sodium; Danaparoid Sodium; Dazoxiben Hydrochloride; Efegatran Sulfate; Enoxaparin Sodium; Ifetroban; Ifetroban Sodium; Tinzaparin Sodium; reteplase; Trifenagrel; Warfarin; Dextrans.

Still other anti-coagulant agents include, but are not limited to, Ancrod; Anticoagulant Citrate Dextrose Solution; Anticoagulant Citrate Phosphate Dextrose Adenine Solution; Anticoagulant Citrate Phosphate Dextrose Solution; Anticoagulant Heparin Solution; Anticoagulant Sodium Citrate Solution; Ardeparin Sodium; Bivalirudin; Bromindione; Dalteparin Sodium; Desirudin; Dicumarol; Heparin Calcium; Heparin Sodium; Lyapolate Sodium; Nafamostat Mesylate; Phenprocoumon; Tinzaparin Sodium.

Inhibitors of platelet function are agents that impair the ability of mature platelets to perform their normal physiological roles (i.e., their normal function). Platelets are normally involved in a number of physiological processes such as adhesion, for example, to cellular and non-cellular entities, aggregation, for example, for the purpose of forming a blood clot, and release of factors such as growth factors (e.g., platelet-derived growth factor (PDGF)) and platelet granular components. One subcategory of platelet function inhibitors are inhibitors of platelet aggregation which are compounds which reduce or halt the ability of platelets to associate physically with themselves or with other cellular and non-cellular components, thereby precluding the ability of a platelet to form a thrombus.

Examples of useful inhibitors of platelet function include but are not limited to acadesine, anagrelide (if given at doses exceeding 10 mg/day), anipamil, argatroban, aspirin, clopidogrel, cyclooxygenase inhibitors such as nonsteroidal anti-inflammatory drugs and the synthetic compound FR-122047, danaparoid sodium, dazoxiben hydrochloride, diadenosine 5',5"'-P1,P4-tetraphosphate (Ap4A) analogs, difibrotide, dilazep dihydrochloride, 1,2- and 1,3-glyceryl dinitrate, dipyridamole, dopamine and 3-methoxytyramine, efegatran sulfate, enoxaparin sodium, glucagon, glycoprotein IIb/IIIa antagonists such as Ro-43-8857 and L-700,462, ifetroban, ifetroban sodium, iloprost, Integrilin (eptifibatide), isocarbacyclin methyl ester, isosorbide-5-mononitrate, itazigrel, ketanserin and BM-13.177, lamifiban, lifarizine, molsidomine, nifedipine, oxagrelate, PGE, platelet activating factor antagonists such as lexipafant, prostacyclin (PGI₂), pyrazines, pyridinol carbamate, ReoPro (i.e., abciximab), sulfinpyrazone, synthetic compounds BN-50727, BN-52021, CV-4151, E-5510, FK-409, GU-7, KB-2796, KBT-3022, KC-404, KF-4939, OP-41483, TRK-100, TA-3090, TFC-612 and ZK-36374, 2,4,5,7-tetrathiaoctane, 2,4,5,7-tetrathiaoctane 2,2-dioxide, 2,4,5-trithiahexane, theophylline, pentoxifylline, thromboxane and thromboxane synthetase inhibitors such as picotamide and sulotroban, ticlopidine, tirofiban, trapidil and ticlopidine, trifenagrel, trilinolein, 3-substituted 5,6-bis(4-methoxyphenyl)-1,2,4-triazines, and antibodies to glycoprotein IIb/IIIa as well as those disclosed in U.S. Pat. No. 5,440,020, and anti-serotonin drugs, Clopidogrel; Sulfinpyrazone; Aspirin; Dipyridamole; Clofibrate; Pyridinol Carbamate; PGE; Glucagon; Antiserotonin drugs; Caffeine; Theophylline Pentoxifylline; Ticlopidine.

### Antihypertensives

Non-limiting examples of antihypertensives that may be used in the compositions of the present invention include amlodipine, benidipine, benazepril, candesartan, captopril, darodipine, diltiazem HCl, diazoxide, doxazosin HCl, enalapril, eprosartan, losartan mesylate, felodipine, fenoldopam, fosinopril, guanabenz acetate, irbesartan, isradipine, lisinopril, mecamylamine, minoxidil, nicardipine HCl, nifedipine, nimodipine, nisoldipine, phenoxybenzamine HCl, prazosin HCl, quinapril, reserpine, terazosin HCl, telmisartan, and valsartan.

### Antilipemic Agents

Non-limiting examples of antilipemic agents that may be used in the compositions of the present invention include acipimox, aluminum nicotinate, atorvastatin, cholestyramine resin, colestipol, polidexide, beclobrate, fluvastatin, gemfibrozil, lovastatin, lysosomal acid lipase, icofibrate, niacin; PPARα agonist such as fibrates, which include, but are not limited to fenofibrate, clofibrate, pirifibrate, ciprofibrate, bezafibrate, clinofibrate, ronifibrate, theofibrate, clofibric acid, etofibrate,and gemfibrozil; pravastatin sodium, simfibrate, simvastatin, niceritrol, nicoclonate, nicomol. oxiniacic acid, etiroxate, thyropropic acid, thyroxine, acifran, azacosterol, benfluorex, beta-benzalbutyramide, carnitine, chondroitin sulfate clomestrone, detaxtran, dextran sulfate sodium, 5, 8, 11, 14, 17-eicosapentaenoic acid, eritadenine, furazabol, meglutol, melinamide, mytatrienediol, ornithine, gamma-oryzanol, pantethine, pentaerythritol tetraacetate, alpha-phenylbutyramide, pirozadil, probucol, beta-sitosterol, sultosilic acid (piperazine salt), tiadenol, triparanol, xenbucin, and mixtures thereof.

### Antidiabetics

Non-limiting examples of antidiabetics that may be used in the compositions of the present invention include biguanides such as buformin, metformin, and phenformin; hormones such as insulin; sulfonylurea derivatives such as acetohexamide, 1-butyl-3-metanilylurea, carbutamide, chlorpropamide, glibornuride, gliclazide, glimepiride, glipizide, gliquidone, glisoxepid, glyburide, glybuthiazole, glybuzole, glyhexamide, glymidine, glypinamide, phenbutamide, tolazamide, tolbutamide, tolcyclamide; HDL agonists; PPARγ agonists such as thiazolidinediones such as pioglitazone, rosiglitazone, and troglitazone; and others including acarbose, calcium mesoxalate, miglitol, and repaglinide.

### Antiinflammatory Agents

Non-limiting examples of antiinflammatory agents that may be used in the compositions of the present invention include Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lomoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salicylates; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Glucocorticoids; Zomepirac Sodium. One preferred antiinflammatory agent is aspirin.

### Calcium Channel Blockers

Calcium channel blockers are a chemically diverse class of compounds having important therapeutic value in the control of a variety of diseases including several cardiovascular disorders, such as hypertension, angina, and cardiac arrhythmias (Fleckenstein, Cir. Res. v. 52, (suppl. 1), p. 13-16 (1983); Fleckenstein, Experimental Facts and Therapeutic Prospects, John Wiley, New York (1983); McCall, D., Curr Pract Cardiol, v. 10, p. 1-11 (1985)). Calcium channel blockers are a heterogeneous group of drugs that prevent or slow the entry of calcium into cells by regulating cellular calcium channels. (Remington, The Science and Practice of Pharmacy, Nineteenth Edition, Mack Publishing Company, Eaton, Pa., p.963 (1995)). Most of the currently available calcium channel blockers, and useful according to the present invention, belong to one of three major chemical groups of drugs, the dihydropyridines, such as nifedipine, the phenyl alkyl amines, such as verapamil, and the benzothiazepines, such as diltiazem. Non-limiting examples of calcium channel blockers that may be used in the compositions of the present invention include bepridil, clentiazem, diltiazem, fendiline, gallopamil, mibefradil, prenylamine, semotiadil, terodiline, verapamil, amlodipine, aranidipine, barnidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, cinnarizine, flunarizine, lidoflazine, lomerizine, bencyclane, etafenone, fantofarone, perhexiline, and mixtures thereof.

### CETP Inhibitors

Non-limiting examples of CETP inhibitors that may be used in the compositions of the present invention include JT7-705 and torcetrapib.

### COX-2 Inhibitors

Non-limiting examples of COX-2 inhibitors that may be used in the compositions of the present invention include compounds according to the following: all of the compounds and substances beginning on page 8 of Winokur WO99/2011 as members of three distinct structural classes of selective COX-2 inhibitor compounds, and the compounds and substances which are selective COX-2 inhibitors in Nichtberger, U.S. Pat. No. 6,136,804, Oct. 24, 2000, entitled "Combination therapy for treating, preventing, or reducing the risks associated with acute coronary ischemic syndrome and related conditions", and the compounds and substances which are selective COX-2 inhibitors in Isakson et al, PCT application WO/09641645 published Dec. 27, 1996, filed as PCT/US 9509905 on Jun. 12, 1995, entitled "Combination of a Cyclooxygenase-2 Inhibitor and a Leukotriene B4 Receptor Antagonist for the Treatment of Inflammations." The meaning of COX-2 inhibitor in this invention shall include the compounds and substances referenced and incorporated into Winokur WO99/2011 by reference to art therein, the compounds and substances referenced and incorporated into Nichtberger, U.S. Pat. No. 6,136,804, Oct. 24, 2000, by reference to art therein, and the compounds and substances which are COX-2 inhibitors referenced and incorporated into Isakson et al, PCT application WO/09641645 published Dec. 27, 1996, filed as PCT/US 9509905 on Jun. 12, 1995, entitled "Combination of a Cyclooxygenase-2 Inhibitor and a Leukotriene B4 Receptor Antagonist for the Treatment of Inflammations." The meaning of COX-2 inhibitor in this invention also includes rofecoxib, and celecoxib, marketed as VIOXX and CELEBREX by Merck and Searle/Pfizer respectively. Rofecoxib is discussed in Winokur, WO99/2011 as compound 3, on p.9. Celecoxib is discussed as SC-58635 in the same reference, and in T. Penning, Synthesis and biological evaluation of the 1,5-diarylpyrazole class of cyclooxygenase-2 inhibitors: identification of 4-[5-(4-methylphenyl)-3-(trifluoromethyl)-1H-pyrozol-1-yl]benzenesulfonami de (SC-58635, celecoxib)", J. Med. Chem. Apr. 25, 1997: 40(9): 1347-56. The meaning of COX-2 inhibitor in this invention also includes SC299 referred to as a fluorescent diaryloxazole. C. Lanzo et al, "Fluorescence quenching analysis of the association and dissociation of a diarylheterocycle to cyclooxygenasel-1 and cyclooxygenase-2: dynamic basis of cycloxygenase-2 selectivity", Biochemistry May 23, 2000, vol. 39(20):6228-34, and in J. Talley et al, "4,5-Diaryloxazole inhibitors of cyclooxygenase-2 (COX-2)", Med. Res. Rev. May 1999; 19(3): 199-208. The meaning of COX-2 inhibitor in this invention also includes valdecoxib, See, "4-[5-Methyl-3-phenylisoxazol-1-yl]benzenesulfonamide, Valdecoxib: A Potent and Selective Inhibitor of COX-2", J. Med. Chem. 2000, Vol. 43: 775-777, and parecoxib, sodium salt or parecoxib sodium, See, N-[[(5-methyl-3-phenylixoxazol-4yl)-phenyl]sulfonyl]propanimide, Sodium Salt, Parecoxib Sodium: A Potent and Selective Inhibitor of COX-2 for Parenteral Administration", J. Med. Chem. 2000, Vol. 43: 1661-1663. The meaning of COX-2 inhibitor in this invention also includes the substitution of the sulfonamide moiety as a suitable replacement for the methylsulfonyl moiety. See, J. Carter et al, Synthesis and activity of sulfonamide-substituted 4,5-diaryl thiazoles as selective cyclooxygenase-2 inhibitors." Bioorg. Med. Chem. Lett Apr. 19, 1999: Vol. 9(8): 1171-74, and compounds referenced in the article "Design and synthesis of sulfonyl-substituted 4,5-diarylthiazoles as selective cyclooxygenase-2 inhibitors", Bioorg. Med. Chem. Lett Apr. 19, 1999: Vol. 9(8): 1167-70. The meaning of this invention includes a COX-2 inhibitor, NS398 referenced in two articles: Attiga et al, "Inhibitors of Prostaglandin Synthesis Inhibit Human Prostate Tumor Cell Invasiveness and Reduce the Release of Matrix Metalloproteinases", 60 Cancer Research 4629-4637, Aug. 15, 2000, and in "The cyclooxygenase-2 inhibitor celecoxib induces apoptosis by blocking Akt activation in human prostate cancer cells independently of Bcl-2," Hsu et al, 275(15) J. Biol. Chem. 11397-11403 (2000). The meaning of COX-2 inhibitor in this invention includes the cyclo-oxygenase-2 selective compounds referenced in Mitchell et al, "Cyclo-oxygenase-2: pharmacology, physiology, biochemistry and relevance to NSAID therapy", Brit. J. of Pharmacology (1999) vol.128: 1121-1132, see especially p. 1126. The meaning of COX-2 inhibitor in this invention includes so-called NO-NSAIDs or nitric oxide-releasing-NSAIDs referred to in L. Jackson et al, "COX-2 Selective Nonsteroidal Anti-Inflammatory Drugs: Do They Really Offer Any Advantages?", Drugs, June, 2000 vol. 59(6): 1207-1216 and the articles at footnotes 27, and 28. Also included in the meaning of COX-2 inhibitor in this invention includes any substance that selectively inhibits the COX-2 isoenzyme over the COX-1 isoenzyme in a ratio of greater than 10 to 1 and preferably in ratio of at least 40 to 1 as referenced in Winokur WO 99/20110, and has one substituent having both atoms with free electrons under traditional valence-shell-electron-pair-repulsion theory located on a cyclic ring (as in the sulfylamine portion of celecoxib), and a second substituent located on a different ring sufficiently far from said first substituent to have no significant electron interaction with the first substituent. The second substituent should have an electronegativity within such substituent greater than 0.5, or the second substituent should be an atom located on the periphery of the compound selected from the group of a halogen F, Cl, Br or I, or a group VI element, S or O. Thus for purposes of this last included meaning of a COX-2 inhibitor, one portion of the COX-2 inhibitor should be hydrophilic and the other portion lipophilic. Also included as a COX-2 inhibitor are compounds listed at page 553 in Pharmacotherapy: A Pathophysiologic Approach, Depiro et al (McGraw Hill 1999) including nabumetone and etodolac. Recognizing that there is overlap among the selective COX-2 inhibitors set out in this paragraph, the intent of the term COX-2 inhibitor is to comprehensively include all selective COX-2 inhibitors, selective in the sense of inhibiting COX-2 over COX-1. The inventors add to the class of COX-2 inhibitors useful in the invention the drug bearing the name etoricoxib referenced in the Wall Street Journal, Dec. 13, 2000, manufactured by Merck. See, also, Chauret et al., "In vitro metabolism considerations, including activity testing of metabolites, in the discovery and selection of the COX-2 inhibitor etoricoxib (MK-0663)," Bioorg. Med. Chem. Lett. 11(8): 1059-62 (Apr. 23, 2001). Another selective COX-2 inhibitor is DFU [5,5-dimethyl-3-(3-fluorophenyl)-4-(4-methylsulphonyl) phenyl-2(5H)-furanone] referenced in Yergey et al, Drug Metab. Dispos. 29(5):638-44 (May 2001). The inventors also include as a selective COX-2 inhibitor the flavonoid antioxidant silymarin, and an active ingredient in silymarin, silybinin, which demonstrated significant COX-2 inhibition relative to COX-1 inhibition. The silymarin also showed protection against depletion of glutathione peroxidase. Zhao et al, "Significant Inhibition by the Flavonoid Antioxidant Silymarin against 12-O-tetracecanoylphorbol 13-acetate-caused modulation of antioxidant and inflammatory enzymes, and cyclooxygenase 2 and interleukin-1 alpha expression in SENCAR mouse epidermis: implications in the prevention of stage I tumor promotion," Mol. Carcinog. December 1999, Vol 26(4):321-33 PMID 10569809. Silymarin has been used to treat liver diseases in Europe.

A number of the above-identified COX-2 inhibitors are prodrugs of selective COX-2 inhibitors, and exert their action by conversion in vivo to the active and selective COX-2 inhibitors. The active and selective COX-2 inhibitors formed from the above-identified COX-2 inhibitor prodrugs are described in detail in WO 95/00501, published Jan. 5, 1995, WO 95/18799, published Jul. 13, 1995 and U.S. Pat. No. 5,474,995, issued Dec. 12, 1995. Given the teachings of U.S. Pat. No. 5,543,297, entitled: "Human cyclooxygenase-2 cDNA and assays for evaluating cyclooxygenase-2 activity," a person of ordinary skill in the art would be able to determine whether an agent is a selective COX-2 inhibitor or a precursor of a COX-2 inhibitor, and therefore part of the present invention.

### "Direct thrombin inhibitors"

Non limiting examples of direct thrombin inhibitors include hirudin, hirugen, Hirulog, argatroban, PPACK, and thrombin aptamers.

### Diuretics

Non-limiting examples of diuretics that may be used in the compositions of the present invention include althiazide, bendroflumethiazide, benzthiazide, buthiazide, chlorthalidone, cyclopenthiazide, cyclothiazide, epithiazide, ethiazide, fenquizone, indapamide, hydroflumethiazide, methyclothiazide, meticrane, metolazone, paraflutizide, polythiazide, quinethazone, teclothiazide, trichloromethiazide, chlormerodrin, meralluride, mercamphamide, mercaptomerin sodium, mercumatilin sodium, mercurous chloride, mersalyl, acefylline, 7-morpholinomethyl-theophylline, pamabrom, protheobromine, theobromine, canrenone, oleandrin, spironolactone, acetazolamide, ambuside, azosemide, bumetanide, butazolamide, clopamide, clorexolone, disulfamide, ethoxzolamide, furosemide, mefruside, methazolamide, piretanide, torsemide, tripamide, xipamide, aminometradine, amisometradine, amanozine, amiloride, arbutin, chlorazanil, ethacrynic acid, etozolin, hydracarbazine, isosorbide, mannitol, metochalcone, muzolimine, perhexiline, ticrynafen, triamterene, urea, and mixtures thereof. Depending on the diuretic employed, potassium may also be administered to the patient in order to optimize the fluid balance while avoiding hypokalemic alkalosis. The administration of potassium can be in the form of potassium chloride or by the daily ingestion of foods with high potassium content such as, for example, bananas or orange juice.

### Endothelin Receptor Antagonists

Non-limiting examples of an endothelin receptor antagonist that may be used in the compositions of the present invention include bosentan, sulfonamide endothelin antagonists, BQ-123, SQ 28608, and the like); and mixtures thereof.

### HMG-CoA Reductase Inhibitor (Statins)

HMG-CoA (3-hydroxy-3-methylglutaryl-coenzyme A) reductase is the microsomal enzyme that catalyzes the rate limiting reaction in cholesterol biosynthesis (HMG-CoA6Mevalonate). An HMG-CoA reductase inhibitor inhibits HMG-CoA reductase, and as a result inhibits the synthesis of cholesterol. A number of HMG-CoA reductase inhibitors have been used to treat individuals with hypercholesterolemia. More recently, HMG-CoA reductase inhibitors have been shown to be beneficial in the treatment of stroke (Endres M, et al., Proc Natl Acad Sci USA, 1998, 95:8880-5).

HMG-CoA reductase inhibitors useful for co-administration with the agents of the invention include, but are not limited to, simvastatin (U.S. Pat. No. 4,444,784), lovastatin (U.S. Pat. No. 4,231,938), pravastatin sodium (U.S. Pat. No. 4,346,227), fluvastatin (U.S. Pat. No. 4,739,073), atorvastatin (U.S. Pat. No. 5,273,995), cerivastatin, and numerous others described in U.S. Pat. Nos. 5,622,985; 5,135,935; 5,356,896; 4,920,109; 5,286,895; 5,262,435; 5,260,332; 5,317,031; 5,283,256; 5,256,689; 5,182,298; 5,369,125; 5,302,604; 5,166,171; 5,202,327; 5,276,021; 5,196,440; 5,091,386; 5,091,378; 4,904,646; 5,385,932; 5,250,435; 5,132,312; 5,130,306; 5,116,870; 5,112,857; 5,102,911; 5,098,931; 5,081,136; 5,025,000; 5,021,453; 5,017,716; 5,001,144; 5,001,128; 4,997,837; 4,996,234; 4,994,494; 4,992,429; 4,970,231; 4,968,693; 4,963,538; 4,957,940; 4,950,675; 4,946,864; 4,946,860; 4,940,800; 4,940,727; 4,939,143; 4,929,620; 4,923,861; 4,906,657; 4,906,624 and 4,897,402.

Other non-limiting examples of HMG-CoA reductase inhibitors that may be used in the compositions of the present invention include mevastatin, pitavastatin, rosuvastatin, gemcabene, and probucol.

### Inotropic Agents

Non-limiting examples of inotropic agents that may be used in the compositions of the present invention include acefylline, acetyldigitoxins, 2-amino-4-picoline, amrinone, benfurodil hemisuccinate, bucladesine, camphotamide, convallatoxin, cymarin, denopamine, deslanoside, digitalin, digitalis, digitoxin, digoxin, dobutamine, docarpamine, dopamine, dopexamine, enoximone, erythrophleine, fenalsomine, gitalin, gitoxin, glycocyamine, heptaminol, hydrastinine, ibopamine, lanatosides, loprinine, milrinone, nerifolin, oleandrin, ouabain, oxyfedrine, pimobendan, prenalterol, proscillaridin, resibufogenin, scillaren, scillarenin, strophanthin, sulmazole, theobromine, vesnarinone, xamoterol, and mixtures thereof.

### "Renin inhibitors"

Renin inhibitors are compounds which interfere with the activity of renin. Renin inhibitors include amino acids and derivatives thereof, peptides and derivatives thereof, and antibodies to renin. Examples of renin inhibitors that are the subject of United States patents are as follows: urea derivatives of peptides (U.S. Pat. No. 5,116,835); amino acids connected by nonpeptide bonds (U.S. Pat. No. 5,114,937); di- and tri-peptide derivatives (U.S. Pat. No. 5,106,835); amino acids and derivatives thereof (U.S. Pat. Nos. 5,104,869 and 5,095,119); diol sulfonamides and sulfinyls (U.S. Pat. No. 5,098,924); modified peptides (U.S. Pat. No. 5,095,006); peptidyl beta-aminoacyl aminodiol carbamates (U.S. Pat. No. 5,089,471); pyrolimidazolones (U.S. Pat. No. 5,075,451); fluorine and chlorine statine or statone containing peptides (U.S. Pat. No. 5,066,643); peptidyl amino diols (U.S. Pat. Nos. 5,063,208 and 4,845,079); N-morpholino derivatives (U.S. Pat. No. 5,055,466); pepstatin derivatives (U.S. Pat. No. 4,980,283); N-heterocyclic alcohols (U.S. Pat. No. 4,885,292); monoclonal antibodies to renin (U.S. Pat. No. 4,780,401); and a variety of other peptides and analogs thereof (U.S. Pat. Nos. 5,071,837, 5,064,965, 5,063,207, 5,036,054, 5,036,053, 5,034,512, and 4,894,437).

### Vasodilators

Non-limiting examples of vasodilators that may be used in the compositions of the present invention include bencyclane, cinnarizine, citicoline, cyclandelate, ciclonicate, diisopropylamine dichloroacetate, eburnamonine, fasudil, fenoxedil, flunarizine, ibudilast, ifenprodil, isosorbide dinitrate, isosorbide mononitrate, lomerixine, nafronyl, nicametate, nicergoline, nimodipine, papaverine, pentifylline, tinofedrine, vancamine, vinpocetine, viquidil, amotriphene, bendazol, benfurodil hemisuccinate, benziodarone, chloracizine, chromonar, clobenfurol, clonitrate, cloricromen, dilazep, dipyridamole, droprenilamine, efloxate, erythrityl tetranitrate, etafenone, fendiline, floredil, ganglefence, heart muscle extract, hexestrol bis(alpha-diethylaminoethyl ether), hexobendine, hydralazine compound, itramin tosylate khellin, lidoflazine, mannitol hexanitrate, medibazine, nitroglycerin, isosorbide mononitrate, isosorbide dinitrate, and other nitrates, pentaerythritol tetranitrate, pentrinitrol, perhexiline, pimefylline, prenylamine, propatyl nitrate, pyridofylline, trapidil, tricromyl, trimetazidine, trolnitrate phosphate, visnadine, aluminum nicotinate, bamethan, bencyclane, betahistine, bradykinin, brovincamine, bufeniode, buflomedil, butalamine, cetiedil, ciclonicate, cinepazide, cinnarizine, cyclandelate, diisopropylamine dichloroacetate, eledoisin, fenoxedil, flunazine, hepronicate, ifenprodil, iloprost, inositol niacinate, isoxsuprine, kallidin, kallikrein, moxisylyte, nafronyl, nicametate nicergoline, nicofuranose, nicotinyl alcohol, nylidrin, pentifylline, pentoxifylline, piribedil, prostaglandin E1, suloctidil, tolazoline, xanthinol niacinate, and mixtures thereof.

Note that "hydralazine compound" refers to a compound having the formula: 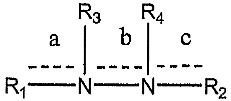 wherein a, b and c are each independently a single or a double bond; R₁ and R₂ are each independently a hydrogen, an alkyl, an ester or a heterocyclic ring; R₃ and R₄ are each independently a lone pair of electrons or a hydrogen, with the proviso that at least one of R₁, R₂, R₃ and R₄ is not a hydrogen. Examples of hydralazine compounds include, but are not limited to budralazine, cadralazine, dihydralazine, endralazine, hydralazine, pildralazine, todralazine and the like.

### Vasopressors

Non-limiting examples of vasopressors that may be used in the compositions of the present invention include amezinium methyl sulfate, angiotensin amide, dimetofrine, dopamine, etifelmin, etilefrin, gepefrine, metaraminol, methoxamine, midodrine, norepinephrine, pholedrine, synephrine, and mixtures thereof.

### AGE Crosslink Breakers (advanced glycosylation end-product crosslink breakers)

Non-limiting examples of AGE crosslink breakers that may be used in the compositions of the present invention include Alagebrium.

### AGE Formation Inhibitors (advanced glycosylation end-product formation inhibitors)

Non-limiting examples of AGE formation inhibitors that may be used in the compositions of the present invention include Pimagedine.

### Other actives:

Non-limiting examples of other active ingredients that may be combined with these nebivolol compositions include, but are not limited to, the following representative classes of compounds, as well as their pharmaceutically acceptable salts, isomers, esters, ethers and other derivatives:
*Analgesics and anti-inflammatory agents*, such as aloxiprin, auranofin, azapropazone, benorylate, capsaicin, celecoxib, diclofenac, diflunisal, etodolac, fenbufen, fenoprofen calcium, flurbiprofen, ibuprofen, indomethacin, ketoprofen, ketorolac, leflunomide, meclofenamic acid, mefenamic acid, nabumetone, naproxen, oxaprozin, oxyphenbutazone, phenylbutazone, piroxicam, rofecoxib, sulindac, tetrahydrocannabinol, tramadol and tromethamine;
*antihelminthics*, such as albendazole, bephenium hydroxynaphthoate, cambendazole, dichlorophen, ivermectin, mebendazole, oxamniquine, oxfendazole, oxantel embonate, praziquantel, pyrantel embonate and thiabendazole;
*anti-asthma agents*, such as zileuton, zafirlukast, terbutaline sulfate, montelukast, and albuterol;
*anti-bacterial agents*, such as alatrofloxacin, azithromycin, baclofen, benzathine penicillin, cinoxacin, ciprofloxacin HCl, clarithromycin, clofazimine, cloxacillin, demeclocycline, dirithromycin, doxycycline, erythromycin, ethionamide, furazolidone, grepafloxacin, imipenem, levofloxacin, lorefloxacin, moxifloxacin HCl, nalidixic acid, nitrofurantoin, norfloxacin, ofloxacin, rifampicin, rifabutin, rifapentine, sparfloxacin, spiramycin, sulphabenzamide, sulphadoxine, sulphamerazine, ulphacetamide, sulphadiazine, sulphafurazole, sulphamethoxazole, sulphapyridine, tetracycline, trimethoprim, trovafloxacin, and vancomycin;
*anti-viral agents*, such as abacavir, amprenavir, delavirdine, efavirenz, indinavir, lamivudine, nelfinavir, nevirapine, ritonavir, saquinavir, and stavudine;
*anti-depressants,* such as amoxapine, bupropion, citalopram, clomipramine, fluoxetine HCl, maprotiline HCl, mianserin HCl, nortriptyline HCl, paroxetine HCl, sertraline HCl, trazodone HCl, trimipramine maleate, and venlafaxine HCl;
*anti-epileptics*, such as beclamide, carbamazepine, clonazepam, ethotoin, felbamate, fosphenytoin sodium, lamotrigine, methoin, methsuximide, methylphenobarbitone, oxcarbazepine, paramethadione, phenacemide, phenobarbitone, phenytoin, phensuximide, primidone, sulthiame, tiagabine HCl, topiramate, valproic acid, and vigabatrin;
*anti-fungal agents*, such as amphotericin, butenafine HCl, butoconazole nitrate, clotrimazole, econazole nitrate, fluconazole, flucytosine, griseofulvin, itraconazole, ketoconazole, miconazole, natamycin, nystatin, sulconazole nitrate, oxiconazole, erbinafine HCl, terconazole, tioconazole and undecenoic acid;
*anti-gout agents*, such as allopurinol, probenecid and sulphinpyrazone;
*anti-malarials*, such as amodiaquine, chloroquine, chlorproguanil HCl, halofantrine HCl, mefloquine HCl, proguanil HCl, pyrimethamine and quinine sulfate;
*anti-migraine agents*, such as dihydroergotamine mesylate, ergotamine tartrate, frovatriptan, methysergide maleate, naratriptan HCl, pizotifen maleate, rizatriptan benzoate, sumatriptan succinate, and zolmitriptan;
*anti-muscarinic agents*, such as atropine, benzhexol HCl, biperiden, ethopropazine HCl, hyoscyamine, mepenzolate bromide, oxyphencyclimine HCl and tropicamide;
*anti-neoplastic agents and immunosuppressants*, such as aminoglutethimide, amsacrine, azathioprine, bicalutamide, bisantrene, busulfan, camptothecin, capecitabine, chlorambucil, cyclosporin, dacarbazine, ellipticine, estramustine, etoposide, irinotecan, lomustine, melphalan, mercaptopurine, methotrexate, mitomycin, mitotane, mitoxantrone, mofetil mycophenolate, nilutamide, paclitaxel, procarbazine HCl, sirolimus, tacrolimus, tamoxifen citrate, teniposide, testolactone, topotecan HCl, and toremifene citrate;
*anti-protozoal agents*, such as atovaquone, benznidazole, clioquinol, decoquinate, diiodohydroxyquinoline, diloxanide furoate, dinitolmide, furazolidone, metronidazole, nimorazole, nitrofurazone, ornidazole and tinidazole;
*anti-psychotics*, such as aripiprazole, clozapine, ziprasidone, haloperidol, molindone, loxapine, thioridazine, molindone, thiothixene, pimozide, fluphenazine, risperidone mesoridazine, quetiapine, trifluoperazine, chlorprothixene, chlorpromazine, perphenazine, trifluopromazine, olanzapine;
*anti-thyroid agents*, such as carbimazole, paricalcitol, and propylthiouracil;
*anti-tussives*, such as benzonatate;
*anxiolytics, sedatives, hypnotics and neuroleptics*, such as alprazolam, amylobarbitone, barbitone, bentazepam, bromazepam, bromperidol, brotizolam, butobarbitone, carbromal, chlordiazepoxide, chlormethiazole, chlorpromazine, chlorprothixene, clonazepam, clobazam, clotiazepam, clozapine, diazepam, droperidol, ethinamate, fluanisone, flunitrazepam, triflupromazine, flupenthixol decanoate, fluphenthixol decanoate, flurazepam, gabapentin, haloperidol, lorazepam, lormetazepam, medazepam, meprobamate, mesoridazine, methaqualone, methylphenidate, midazolam, molindone, nitrazepam, olanzapine, oxazepam, pentobarbitone, perphenazine pimozide, prochlorperazine, pseudoephedrine, quetiapine, risperidone, sertindole, sulpiride, temazepam, thioridazine, triazolam, zolpidem, and zopiclone;
*corticosteroids,* such as beclomethasone, betamethasone, budesonide, cortisone acetate, desoxymethasone, dexamethasone, fludrocortisone acetate, flunisolide, fluocortolone, fluticasone propionate, hydrocortisone, methylprednisolone, prednisolone, prednisone and triamcinolone;
*anti-parkinsonian agents*, such as apomorphine, bromocriptine mesylate, lisuride maleate, pramipexole, ropinirole HCl, and tolcapone;
*gastrointestinal agents*, such as bisacodyl, cimetidine, cisapride, diphenoxylate HCl, domperidone, famotidine, lansoprazole, loperamide, mesalazine, nizatidine, omeprazole, ondansetron HCL, rabeprazole sodium, ranitidine HCl and sulphasalazine;
*keratolytics*, such as acitretin, calcipotriene, calcifediol, calcitriol, cholecalciferol, ergocalciferol, etretinate, retinoids, Targretin, and tazarotene;
*lipid regulating agents*, such as atorvastatin, bezafibrate, cerivastatin, ciprofibrate, clofibrate, fenofibrate, fluvastatin, gemfibrozil, pravastatin, probucol, and simvastatin;
*muscle relaxants,* such as dantrolene sodium and tizanidine HCl;
*nutritional agents*, such as calcitriol, carotenes, dihydrotachysterol, essential fatty acids, non-essential fatty acids, phytonadiol, vitamin A, vitamin B.sub.2, vitamin D, vitamin E and vitamin K;
*opioid analgesics*, such as codeine, dextropropoxyphene, diamorphine, dihydrocodeine, fentanyl, meptazinol, methadone, morphine, nalbuphine and pentazocine;
*sex hormones,* such as clomiphene citrate, cortisone acetate, danazol, dehydroepiandrosterone, ethynyl estradiol, finasteride, fludrocortisone, fluoxymesterone, medroxyprogesterone acetate, megestrol acetate, mestranol, methyltestosterone, norethisterone, norgestrel, oestradiol, conjugated estrogens, progesterone, rimexolone, stanozolol, stilbestrol, testosterone and tibolone;
*stimulants,* such as amphetamine, dexamphetamine, dexfenfluramine, fenfluramine and mazindol;
*drugs for rheumatoid arthritis* such as methotrexate, auranofin, aurothioglucose and gold sodium thiomalate;
*drugs for osteoporosis* such as alendronate and raloxifene;
local anesthetics;
*anti-herpes drugs* such as acyclovir, valacyclovir and famciclovir;
*anti-emetics* such as ondansetron and granisetron;
*Flavonoids and Isoflavonoids* which include the anthocyanidins and anthocyanins; proanthocyanidins; flavan-3-ols; flavonols; flavones; flavanones; isoflavanones; salts and esters thereof. This development is however, not limited to flavonoid compounds isolated from plant, part of plant or extracts of *Astragalus Membranaceus,* but encompasses any suitable flavonoid compound isolated from different sources or chemically synthesized. In addition, any suitable known or not yet discovered flavonoid compound, and isoflavonoid compound, is within the scope of the present technology. A number of flavonoids and isoflavonoids are described in USDA-Iowa State University Database on the Isoflavone Content of Foods, Release 1.3-2002, and in USDA Database for the Flavonoid Content of Selected Foods--2003 (http://www.nal.usda. gov/fnic/foodcomp/Data/isoflav/isoflav.html) and (http://www.nal.usda.gov/fnic/foodcomp/Data/Flav/flav.html). It will be evident to any skilled person how to choose the suitable flavonoid and/or isoflavonoid compound for the purpose of the present development. For example, flavonoid compounds for the purpose of the present development may be, but are not limited to, (-)-epictechin, (+)-catechin, procyanidin B2, quercetin dehydrate, taxifolin, resveratrol, and the like.

*Carotenoids,* which generally are tetraterpenes originating from the mevalonate and deoxyxylulose phosphate pathways (older sources sometimes refer to their source as the 'isoprenoid' pathway). Two molecules of the C₂₀ compound geranylgeranyldiphosphate (GGDP) condense to form the symmetrical carotenoid skeleton.

Carotenoids are divided into two subclasses, i.e., more polar compounds called xanthophylls, or oxycarotenoids, and the nonpolar hydrocarbon carotenes.

Terms such as carotenoid analog and carotenoid derivative may generally refer to in some embodiments chemical compounds or compositions derived from a naturally occurring carotenoid or simply to synthetic carotenoids. In some embodiments, terms such as carotenoid analog and carotenoid derivative may generally refer to chemical compounds or compositions which are synthetically derived from non-carotenoid based parent compounds; however, which ultimately substantially resemble a carotenoid derived analog. In certain embodiments, terms such as carotenoid analog and carotenoid derivative may generally refer to a synthetic derivative of a naturally occurring carotenoid. Examples of carotenoids are provided in the book "Carotenoids Handbook," edited by G. Britton et al, 2004.

It will be evident to any skilled person how to choose the suitable carotenoid compound for the purpose of the present development.

Examples of carotenoids include astaxanthin, zeaxanthin, lutein, lycopene, beta-carotene.

Other non-limiting examples of naturally occurring carotenoids include:
Aaptopurpurin; Actinioerythrin; Actinioerythrol; Adonirubin; Adonixanthin; A.g.470; A.g.471; Agelaxanthin C; Aleuriaxanthin; Alloxanthin; Amarouciaxanthin A; Amarouciaxanthin B; Anchovyxanthin; 3',4'-Anhydrodiatoxanthin; Anhydrodeoxyflexixanthin; Anhydroeschscholtzxanthin; Anhydrolutein; Anhydroperidinin; Anhydrorhodovibrin; Anhydrosaproxanthin; Anhydrowarmingol; Anhydrowarmingone; Antheraxanthin; Aphanicin; Aphanicol; Aphanin; Aphanol; Aphanizophyll; 8'-Apo-beta-caroten-8'-al; 10'-Apo-beta-caroten-10'-al; 12'-Apo-beta-caroten-12'al; 14'-Apo-beta-caroten-14'-al; 6'-Apo-psi-caroten-6'-al; 8'-Apo-psi-caroten-8'-al; beta-Apo-2-carotenal; beta-Apo-3-carotenal; beta-Apo-4-carotenal; beta-Apo-2'-carotenal; beta-Apo-8'-carotenal; beta-Apo-10'-carotenal; beta-Apo-12'-carotenal; beta-Apo-14'-carotenal; Apo-8,8'-carotenedial; 8'-Apo-beta-carotene-3,8'-diol; 4'-Apo-beta-caroten-4'-oic acid; 8'-Apo-beta-caroten-8'-oic acid; 10'-Apo-beta-caroten-10'-oic acid; 12'-Apo-beta- caroten-12'-oic acid; beta-Apo-2'-carotenoic acid; beta-Apo-2'-carotenoic acid methylester; beta-Apo-8'-carotenoic acid; beta-Apo-10'-carotenoic acid; beta-Apo-12'-carotenoic acid; 8'-Apo-beta-caroten-3-ol; beta-Apo-2'-carotenol; Apo-7-fucoxanthinol; Apo-2-lycopenal; Apo-3-lycopenal; Apo-6' lycopenal; Apo-8'-lycopenal; Apo-10'-violaxanthal; Apo-12'-violaxanthal; Apoviolaxanthinal; Apo-2-zeaxanthinal; Apo-3-zeaxanthinal; Apo-4-zeaxanthinal; Astacein; Astacene; Astacene dipalmitate; Astaxanthin; Asterinic acid; Asteroidenone; Asym. zeta- carotene; Aurochrome; Auroxanthin; Azafrin; Azafrinaldehyde;
Bacterial phytoene; Bacterioerythrin alpha; Bacterioerythrin beta; Bacteriopurpurin alpha; Bacterioruberin; alpha-Bacterioruberin; Bacterioruberin diglycoside; Bacterioruberin monoglycoside; alpha-Bacterioruberin monomethyl ether; Bisanhydrobacterioruberin; 3,4,3',4'-Bisdehydro-beta-carotene; Bisdehydrolycopene; 2,2'-Bis(4-hydroxy-3-methyl-2-butenyl)-beta,beta- carotene; 2,2'-Bis[3-(glucosyloxy)-3-methylbutyl]-3,4,3',4'-tetradehydro-1,2,1',2'-tetrahydro-psi,psi-carotene-1,1'-diol; 2,2'-Bis[4-(beta,D- glucopyranosyloxy)-3-methyl-2-butenyl]-gamma,gamma-carotene; 2,2'-Bis(4-hydroxy-3-methyl-2-butenyl)-gamma,gamma-carotene; 2,2'-Bis(4- hydroxy-3-methyl-2-butenyl)-[epsi],[epsi]-carotene; 2,2'-Bis(3-hydroxy-3-methylbutyl-3,4,3',4'-tetradehydro-1,2,1',2'tetrahydro-psi,psi-carotene-1,1'-diol; 2,2'-Bis(3-methyl-2-butenyl)-[epsi],[epsi]-carotene; 2,2'-Bis(3-methyl-2-butenyl-3,4,3',4'-tetradehydro-1,2-dihydro-psi,psi -caroten-1-ol; 2,2'-Bis(3-methyl-2-butenyl)-3,4,3',4'-tetradehydro-1,2,1',2'-tetrahydro-psi,psi-carotene-1,1'-diol; 2,2'-Bis(3-methyl-2-butenyl)-1,2,1',2'-tetrahydro-psi,psi-carotene-1,1'-diol; 2,2'-Bis(O-methyl-5-C-methylpentosyloxy)-3,4,3',4'-tetradehydro-1,2,1',2'-tetrahydropsi, psi-carotene-1,1'-diol; 3,3'-Bis(rhamnosyloxy)-beta,beta-carotene; 2,2'-Bis(rhamnosyloxy)-3,4,3',4'-tetradehydro-1,2,1',2'-tetrahydro-psi, psi-carotene-1,1'-diol; Bixin;
Caloxanthin; Calthaxanthin; Canthaxanthin; Capsanthin; Capsanthin epoxide; Capsanthinone; Capsanthone; Capsochrome; Capsorubin; Capsorubindione; Capsorubone; Carangoxanthin; 16'-Carboxyl-3',4'-dehydro-gamma-carotene; Carcinoxanthin; Caricaxanthin; beta-Carotenal; psi, psi-Caroten-20-al; Carotene; Carotene X; alpha-Carotene; beta- Carotene; beta,beta-Carotene; beta,gamma-Carotene; beta,[epsi]- Carotene; beta,[phi]-Carotene; beta,psi-Carotene; gamma-Carotene; gamma,gamrna-Carotene; gamma,psi-Carotene; [delta]-Carotene; [epsi] -Carotene; [epsi] (1)-Carotene; [epsi],[epsi]-Carotene; [epsi],psi -Carotene; zeta-Carotene; zeta-Carotene, asym.; eta-Carotene; [theta]-Carotene; xi-Carotene; [phi]-Carotene; [phi],[phi]-Carotene; [phi],X-Carotene; [phi],psi-Carotene; X,X-Carotene; psi-Carotene; psi,alpha-Carotene; psi,psi-Carotene; [theta]-Carotene; beta- Carotene-5,6,5',6'-diepoxide; beta-Carotene 5,8,5',8'-diepoxide; beta, beta-Carotene-2,2'-diol; beta,beta-Carotene-2,3-diol; beta,beta- Carotene-3,4-diol; beta,beta-Carotene-3,3'-diol; beta,beta-Carotene-4,4'-diol; beta,[epsi]-Carotene-3,2'-diol; beta,[epsi]- Carotene-3,3'-diol; beta,psi-Carotene-2,3-diol; beta,psi-Carotene- 3,3'-diol; [epsi],[epsi]-Carotene-3,3'-diol; [phi],[phi]-Carotene-3,3'- diol; psi,psi-Carotene-16,16'-diol; beta,beta-Carotene-3,3'-diol dipalmitate; beta, [epsi]-Carotene-3,3'-diol dipalmitate; beta,beta-Carotene-2,2'-dione; beta,beta-Carotene-3,4-dione; beta,beta-Carotene-4,4'dione; beta,psi-Carotene-3,4-dione; [epsi],[epsi]-Carotene-3,3'-dione; beta,chi-Carotene-3',6'-dione; beta,X-Carotene- 3,4-dione; beta,psi-Carotene-4,4'-dione; beta,[phi]-Carotene-3,4-dione; psi,psi-Carotene-4,4'-dione; alpha-Carotene 5,6-epoxide; beta-Carotene 5,6-epoxide; zeta-Carotene epoxide; Carotene oxide; beta,beta-Carotene-3,4,3',4'-tetrol; beta,beta-Carotene-2,3,2',3'- tetrol; beta,beta-Carotene-3,4,3',4'-tetrone; chi,chi-Carotene-3, 6,3',6'-tetrone; beta,beta-Carotene-2,3,2'-triol; beta,beta-Carotene-2,3,3'-triol; beta,beta-Carotene-3,4,3'-triol; beta,beta- Carotene-3,4,4'-triol; beta,[epsi]-Carotene-3,4,3'-triol; beta,[epsi]-Carotene-3,19,3'-triol; beta,[epsi]-Carotene-3,20,3'-triol; beta, beta-Carotene-3,4,4'-trione; beta,beta-Caroten-2-ol; beta,beta- Caroten-3-ol; beta,beta-Caroten-4-ol; beta,[epsi]-Caroten-2-ol; beta,[epsi]-Caroten-3-ol; beta,[epsi]-Caroten-3'-ol; beta,[epsi]-Caroten-4-ol; beta,[phi]-Caroten-3-ol; beta,X-Caroten-3-ol; beta, psi-Caroten-3-ol; beta,psi-Caroten-4'-ol; [epsi],psi-Caroten-3-ol; [phi],[phi]-Caroten-3-ol; [phi],[phi]-Caroten-16-ol; [phi],[phi]-Caroten-20-ol; Carotenonaldehyd; beta-Carotenone; beta,beta-Caroten-2-one; beta,beta-Caroten-4-one; beta,[epsi]-Caroten-2-one; beta,[epsi]-Caroten-4-one; beta,psi-Caroten-4-one; gamma-Caroten-4-one; alpha- Carotone; Celaxanthin; Chiriquixanthin A; Chiriquixanthin B; Chlorellaxanthin; Chlorobactene; Chloroxanthin; Chrysanthemaxanthin; Citranaxanthin; alpha-Citraurin; beta-Citraurin; beta-Citraurinene; beta-Citraurinol; Citroxanthin; Compound X; C.p.: *Corynebacterium poinsettiae;* Corynexanthin; Corynexanthin glucoside; C.p.; C.p.; C.p.; Crocetin; gamma-Crocetin; Crocetindial(dehyde); Crocetin diglucosyl ester; Crocetin dimethyl ester; Crocetin gentiobiosyl glucosyl diester; Crocetin glucosyl methyl diester; Crocetin monogentiobiosyl ester; Crocetinsemialdehyde; Crocin; Crocoxanthin; Crustaxanthin; Cryptocapsin; Cryptocapsone; Cryptochrome; alpha-Cryptoeutreptiellanone; beta-Cryptoeutreptiellanone; Cryptoflavin; Cryptomonaxanthin; Cryptoxanthene; Cryptoxanthin; alpha-Cryptoxanthin; beta-Cryptoxanthin; Cryptoxanthin 5,6,5',6' diepoxide; Cryptoxanthin 5,6,5',8' diepoxide; Cryptoxanthin 5,8, 5',8' diepoxide; Cryptoxanthin 5,6-epoxide; Cryptoxanthin 5,8-epoxide; Cryptoxanthol; Cucurbitaxanthin; Cyclic zeta-carotene; Cynthiaxanthin; Decahydro-beta-carotene; 1,2,7,8,11,12,7',8',11',12'- Decahydro-psi,psi-carotene; 7,8,11,12,15,7',8',11',12',15' Decahydro- psi,psi-carotene; 1,2,7,8,11,12,7',8',11',12'-Decahydro-psi,psi-caroten-1-ol; Decahydrolycopene; Decaprenoxanthin; Decaprenoxanthin diglucoside; Decaprenoxanthin monoglucoside; Deepoxyneoxanthin; Dehydro- see also Bisdehydro-, Didehydro-, MonodehydroDehydroadonirubin; Dehydroadonixanthin; Dehydrocarotene II; Dehydrocarotene III; Dehydro-beta-carotene; 3,4-Dehydro-beta-carotene; 3',4'-Dehydro-gamma- carotene; 3',4'-Dehydrocryptoxanthin; Dehydrogenans-P; Dehydrogenans-P; Dehydrogenans-P; Dehydrogenans-P; Dehydrogenans-P 439 mono-OH; dehydrogenans-Phytoene; dehydrogenans-Phytofluene; Dehydrohydroxyechinenone; 3'-Dehydrolutein; 3,4-Dehydrolycopen-16-al; Dehydrolycopene; 3,4-Dehydrolycopene; 15,15'-Dehydrolycopersene; 7',8', 11',12'-Dehydrononapreno xanthin; 11',12'-Dehydrononaprenoxanthin; 3',4'-Dehydro-17'(or 18')-oxo-gamma-carotene; Dehydropapilioerythrin; 11,12-Dehydrophytoene; 11',12'-Dehydrophytoene; 2'-Dehydroplectaniaxanthin; Dehydroretrocarotene; 3,4-Dehydrorhodopin; Dehydrorhodovibrin; 3',4'-Dehydrorubixanthin; Dehydrosqualene; 7,8,7',8'-Dehydrozeaxanthin; 7,8-Dehydrozeinoxanthin; Demethyl(ated) spheroidene; Deoxyflexixanthin; Deoxylutein I; Deshydroxydecaprenoxanthin; Diadinochrome; Diadinoxanthin; Dianhydroeschscholtzxanthin; 4,4'-Diapo-zeta-carotene; 4,4'-Diapocaroten-4-al; 4,4'-Diapocarotene-4,4'-dial; 8,8'-Diapocarotene-8,8'-dial; 6,6'-Diapocarotene-6,6'-dioic acid; 8,8'-Diapocarotene-8,8'-dioic acid; 4,4'-Diapocaroten-4-oic acid; 4,4'-Diaponeurosporene; 4,4'- Diaponeurosporen-4-oic acid; 4,4'-Diapophytoene; 4,4'-Diapophytofluene; 4,4'-Diapo-7,8,11,12-tetrahydro lycopene; Diatoxanthin; Didehydro-, see also Dehydro-, Monodehydro 3',4'-Didehydro-2'-apo-beta-caroten-2'-al; 3',4'-Didehydro-2'-apo-beta-caroten-2'-ol; 7,8-Didehydroastaxanthin; 3', 4'-Didehydro-beta,psi-caroten-16'-al; 3,4-Didehydro-psi,psi-caroten-16-al; 3,4-Didehydro-beta,beta-carotene; 4,4'-Didehydro-beta-carotene; 3,4-Didehydro-beta,[epsi]-carotene; 3,4-Didehydro-beta, [phi]-carotene; 3,4-Didehydro-beta,X-carotene; 3',4'-Didehydro-beta, psi-carotene; 3',4'-Didehydro-gamma,psi-carotene; 7,8-Didehydro- [phi],[phi]-carotene; 7,8-Didehydro-[phi],X-carotene; 3,4-Didehydro-psi, psi-carotene; 7,8-Didehydro-beta,beta-carotene-3,3'-diol; 7,8-Didehydro-beta,[epsi]-carotene-3,3'-diol; 3,4-Didehydro-beta,beta-carotene-2,2'-dione; 3',4'-Didehydro-beta,psi-caroten-16'-oic acid; 3',4'-Didehydro-beta,beta-caroten-3-ol; 3',4'-Didehydro-beta,beta-caroten-4-ol; 7,8-Didehydro-beta,[epsi]-caroten-3-ol; 7,8-Didehydro-beta,[phi]-caroten-3-ol; 7,8-Didehydro-beta,X-caroten-3-ol; 3',4'- Didehydro-beta,psi-caroten-3-ol; 3',4' Didehydro-beta,psi-caroten-16'-ol; 3',4'-Didehydro-beta,psi-caroten-18'-ol; 3',4'-Didehydro- beta,beta-caroten-4-one; 3',4'-Didehydro-beta,psi-caroten-4-one; 7',8'-Didehydro-beta,beta-carotene 3,4,3'-triol; 3,4-Didehydro-1,2-dihydro-psi,psi-carotene; 3,4-Didehydro-1,2-dihydro-psi,psi-caroten-20-al; 6,7-Didehydro-5,6-dihydro-beta,beta-carotene-3,3'-diol; 3',4'-Didehydro-1',2'-dihydro-beta,psi-carotene-3,1'-diol; 3',4'-Didehydro-1',2'-dihydro-beta,psi-carotene-1',2'-diol; 3',4'-Didehydro-1',2'-dihydro-beta,psi-carotene-4,2'-dione; 3,4-Didehydro-1,2-dihydro-psi,psi-carotene-1,2-diol; 7',8'-Didehydro-5,6-dihydro-beta,beta-carotene-3,5,6,3'-tetrol; 6,7-Didehydro-5,6-dihydro-beta,beta-carotene-3,5,3'-triol; 7',8'-Didehydro-5,6-dihydro-beta,beta-carotene-3,5,3-triol; 3',4-Didehydro-1'2'-dihydro-beta,psi-carotene-2,1',2'-triol; 1',16'-Didehydro-1',2'-dihydro-beta,psi-caroten-2'-ol; 3',4'-Didehydro-1',2'-dihydro-beta,psi-caroten-1'-ol; 3',4'-Didehydro-1',2'-dihydro-beta,psi-caroten-2'-ol; 3,4-Didehydro-1,2-dihydro-psi,psi-caroten-1-ol; 3',4'-Didehydro-18'-hydroxy-gamma-carotene; 7,8- Didehydroisorenieratene; 3',4'-Didehydro-4-keto-gamma-carotene; 7,8- Didehydrorenieratene; 4',5'-Didehydro-4,5'-retro-beta,beta-carotene; 4',5'-Didehydro-4,5'-retro-beta,psi-carotene; Didehydroretro-gamma- carotene; 4',5'-Didehydro-4,5'-retro-beta,beta-carotene-3,3'-diol; 4',5'-Didehydro-4,5'-retro-beta,beta-carotene-3,3'-dione; 10',11'-Didehydro-5,8,11',12'tetrahydro-10'-apo-beta-carotene-3,5,8-triol; 6',7'-Didehydro-5,6,5',6'tetrahydro-beta,beta-carotene-3,5,6,3',5'-pentol; 6,7-Didehydro-5,6,5',6'-tetra hydro-beta,beta-carotene-3,5,3',5'tetrol; 3,4-Didehydro-1,2,7',8'-tetra hydro-psi,psi-caroten-1-ol; Didehydrotrikentriorhodin; 7,8-Didehydrozeaxanthin; Didemethylated spirilloxanthin; 1,2,1',2'-Diepoxy-2,2'-b is (3-hydroxy-3-methylbutyl)3,4-didehydro-1,2,1',2'-tetrahydro-psi,psi-carotene; Diepoxy-beta- carotene; 5,8,5',8'-Diepoxycryptoxanthin; 5,6,5',6'-Diepoxy-5,6,5',6'- tetrahydro-beta,beta-carotene; 5,6,5',8'-Diepoxy-5,6,5',8'tetrahydro-beta,beta-carotene; 5,8,5',8'-Diepoxy-5,8,5',8'tetrahydro-beta, beta-carotene; 5,6,5',6'-Diepoxy-5,6,5',6'-tetrahydro-beta,beta-carotene-3,3'-diol; 5,6,5',8'-Diepoxy-5,6,5',8'tetrahydro-beta,beta-carotene-3,3'-diol; 5,8,5',8'-Diepoxy-5,8,5',8'tetrahydro-beta,beta- carotene-3,3'-diol; 5,6,5',6'-Diepoxy-5,6,5',6'tetrahydro-beta,beta-caroten-3-ol; 5,6,5',8'-Diepoxy-5,6,5',8'tetrahydro-beta,beta-caroten- 3-ol; 5,8,5',8'-Diepoxy-5,8,5',8'tetrahydro-beta,beta-caroten-3-ol; 5,6,5',8'-Diepoxyzeaxanthin; 5,8,5',8'-Diepoxyzeaxanthin; Digentiobiosyl 8,8'-diapocarotene-8,8'-dioate; Di-(beta,D-glucopyranosyi)-4,4'-diapocarotene-4,4'-dioate; Diglucosyl 8,8'-diapocarotene-8,8'-dioate; Dihydroanhydrorhodovibrin; 9',10'-Dihydro-9'-apo-beta-carotene-3,9'-dione; 9',10'-Dihydro-9'-apo-[epsi]-carotene-3,9'-dione; 7',8'-Dihydro-7'-apo-beta-caroten-8'-one; 5',6'-Dihydro-5'-apo-18'-nor-beta-caroten-6'-one; 7,8-Dihydroastaxanthin; beta-Dihydrocarotene; 1,1'-Dihydro-beta- carotene; 3,4-Dihydro-beta-carotene; 7,7'-Dihydro-beta-carotene; 7',8'-Dihydro-beta,psi-carotene; 7',8'-Dihydro-gamma-carotene; 7',8'- Dihydro-gamma,psi-carotene; 7',8'-Dihydro-[delta]-carotene; 7',8'- Dihydro-[epsi],psi-carotene; 1,2-Dihydro-zeta-carotene; 1,2-Dihydro-psi,psi-carotene; 7,8-Dihydro-psi,psi-carotene; 7,8-Dihydro-beta,beta-carotene 3,3'-diol; 7',8'-Dihydro-beta,psi-carotene 3,17'- diol; 9',10'-Dihydro-beta,psi-carotene-3,17'-diol; 7',8'-Dihydro-[epsi],psi-carotene-3,17'-diol; 1,2-Dihydro-psi,psi-carotene-1,20-diol; 5,6-Dihydro-beta,beta-carotene 3,5,6,3'-tetrol; 5,6-Dihydro- beta,beta-carotene 3,5,3'-triol; 1',2'-Dihydro-beta,psi-caroten 1'-ol; 7',8'-Dihydro-beta,psi-caroten 3-ol; 1',2'-Dihydro-[phi],psi- caroten-1'-ol; 1,2-Dihydro-psi,psi-caroten-1-ol; 5,6-Dihydro-beta, beta-carotene-3,5,6,3'-tetrol; 5,6-Dihydro-beta,[epsi]-carotene-3,5,6, 3'-tetrol; 7,8 (or 7',8)-Dihydrodecaprenoxanthin monoglucoside; 1',2'-Dihydro-3',4'-dehydro-3,1'-dihydroxy-gamma-carotene; 1,2-Dihydro-3,4-dehydrolycopene; 1,2-Dihydro-3,4-dehydro-1-OH-lycopene; 7,8-Dihydro-4,4'-diapocarotene; 7',8'-Dihydro-4,4'-diapocaroten-4-al; 7',8'-Dihydro-4,4'-diapocaroten-4-oic acid; 1',2'-Dihydro-3',4'-didehydro-3,1'-dihydroxy-gamma-caroten-2'yl rhamnoside; 1',2'-Dihydro-1',2'-dihydroxy-4-ketotorulene; 1',2'-Dihydro-3,1'-dihydroxytorulene glucoside; 1',2'-Dihydro-3,1'-dihydroxytorulene rhamnoside; 1',2'-Dihydro-4,2'-diketotorulene; 3'-Dihydro-[boxH]-doradecin; 1',2'-Dihydro-1'-glucosyl-3, 4-dehydrotorulene; 1',2'-Dihydro-1'-glucosyl-4-ketotorulene; 1',2'-Dihydro-1'-hydroxy-gamma-carotene; 1',2'-Dihydro-1'-hydroxychiorobactene; 1',2'-Dihydro-2'-hydroxy-3',4'-dehydro-4-keto-gamma-carotene; 1',2'-Dihydro-1'-hydroxy-3,4-dehydrotorulene glucoside; 1',2'-Dihydro-1'-hydroxy-4-keto-gamma-carotene; 1',2'-Dihydro-1'-hydroxy-4-ketotorulene; 1',2'-Dihydro-1'-hydroxy-4-ketotorulene glucoside; 1',2'-Dihydro-1'-hydroxysphe roideneone; 1',2'-Dihydro-1'-hydroxytorulene glucoside; 1',2'-Dihydro-1'-hydroxytorulene rhamnoside; 1, 2-Dihydrolycopene; 1',2'-Dihydrolycopene; 7,8-Dihydrolycopene; 1,2- Dihydro-1-methoxy-lycopen-20-al; Dihydromethoxylycopene; 5,6-Dihydro-4-methoxy-lycopen-6-one; 1,2-Dihydroneurosporene; 1',2'-Dihydroneurosporene; 1,2-Dihydro-1-OH-lycopene; 1',2'-Dihydro-1'-OH-torulene; 2'- Dihydrophillipsiaxanthin; Dihydrophytoene; 1,2-Dihydrophytoene; 1',2'-Dihydrophytoene; 1,2-Dihydrophytofluene; 1',2'-Dihydrophytofluene; 7,8- Dihydro-8,7'-retro-beta,beta-carotene; 7',8'-Dihydrorhodovibrin; 7,8 (or 7',8')-Dihydrosarcinaxanthin; 3,4-Dihydrospheroidene; 11',12'- Dihydrospheroidene; 3,4-Dihydrospirilloxanthin; 3,3'- Dihydroxycanthaxanthin; 3,3'-Dihydroxy-alpha-carotene; 3,4-Dihydroxy-beta-carotene; 2,3-Dihydroxy-beta,beta-carotene-4,4'-dione; 3,3'- Dihydroxy-[epsi]-carotene; 2,3'-Dihydroxy-beta,beta-carotene-4,4'-dione; 3,3'-Dihydroxy-beta,beta-carotene-4,4'-dione; 3,3'-Dihydroxy-beta,[epsi]-carotene-4,2'-dione; 3,3'-Dihydroxy-beta,chi-carotene-4,6'-dione; 3,3'-Dihydroxy-chi,chi-carotene-6,6'-dione; 2,3-Dihydroxy-beta,beta-caroten-4-one; 3,3'-Dihydroxy-beta,beta-caroten-4-one; 3,2'-Dihydroxy-beta,[epsi]-caroten-4-one; 3,3'-Dihydroxy-beta,[epsi]-caroten-4-one; 3,3'-Dihydroxy-beta,chi-caroten-6'-one; 3,8-Dihydroxy- chi,X-caroten-6-one; 3,3'-Dihydroxydehydro-beta-carotene; 3,3'-Dihydroxy-7,8-dehydro-beta-carotene; 3,3'-Dihydroxy-7,8,7',8'-dehydro-beta-carotene; 3,3'-Dihydroxy-7,8-dehydro-beta-carotene-5',6'-epoxide; 3,3'-Dihydroxy-2,3-didehydro-beta,beta-carotene-4,4'-dione; 3,3'-Dihydroxy-7,8-didehydro-beta,beta-carotene-4,4'-dione; 3',8'-Dihydroxy-7,8-didehydro-beta,chi-carotene-3',6'-dione; 3,3'-Dihydroxy-2,3-didehydro-beta,beta-caroten-4-one; 3,3'-Dihydroxy-7',8'-didehydro-beta,beta-caroten-4-one; 3,4'-Dihydroxy-2,3-didehydro-beta,beta-caroten-4-one; 3,3'-Dihydroxy-2,3-didehydro-beta,[epsi]-caroten-4-one; 3,8-Dihydroxy-7',8'-didehydro-chi,X-caroten-6-one; 3,6'-Dihydroxy-7,8-didehydro-6',7'dihydro-beta,[epsi]-carotene-3',8'-dione; 3,3'-Dihydroxy-7,8-didehydro-7',8'dihydro-beta,chi-carotene-6',8'-dione; 3,1'-Dihydroxy-3',4'-didehydro-1',2'-dihydro-beta,psi-caroten-4-one; 1',2'-Dihydroxy-3',4'-didehydro-1',2'-dihydro-beta,psi-caroten-4-one; 3,5-Dihydroxy-6,7-didehydro-5,6,7',8'-tetrahydro-7'-apo-beta-caroten-8'-one; 6,3'-Dihydroxy-7',8'-didehydro-5,6,7,8-tetrahydro-beta,beta-carotene- 3,8-dione; 3,3'-Dihydroxy-5,8,5',8'-diepoxy-beta-carotene; 5,6-Dihydroxy-5,6-dihydro-10'-apo-beta-caroten-10'-al; 5,6-Dihydroxy-5,6-dihydro-10'-apo-beta-caroten-10'-oic acid; 5,6-Dihydroxy-5,6-dihydro 12'-apo-beta-caroten-12'-oic acid; 3,3'-Dihydroxy-7,8-dihydro-beta, beta-carotene-4,4'-dione; 3,1'-Dihydroxy-1',2'-dihydrotorulene; 1',2'-Dihydroxy-1',2'-dihydrotorulene; 3,3'-Dihydroxy-4,4'-diketo-beta-carotene; 3,3'-Dihydroxy-2,2'-dinor-beta,beta-carotene-4,4'-dione-3,3'-diacylate; 3,19-Dihydroxy-3',6'-dioxo-7,8-didehyro-beta,chi-caroten-17-al; 1,1'-Dihydroxy-2,2'-dirhamnosyl-1,2,1',2'-tetrahydro-3,4,3',4'-tetrahydrolycopene; 3,3'-Dihydroxyechinenone; 3,3'-Dihydroxy-5,6-epoxy-alphacarotene; 3,3'-Dihydroxy-5,8-epoxy-alpha-carotene; 3,3'-Dihydroxy-5,6-epoxy-beta-carotene; 3,3'-Dihydroxy-5,8-epoxy-beta- carotene; 2-(Dihydroxyisopentenyl)-2'-isopentenyl-beta-carotene; 3,3'- Dihydroxyisorenieratene; 3,3'-Dihydroxy-4-keto-gcarotene; 3,3'-Dihydroxyluteochrome; Dihydroxylycopene; 3,1'-Dihydroxy-2'-(5-C-methylpentosyloxy)-3',4'-didehydro-1',2'-dihydro-beta,psi-caroten-4-one; Dihydroxyneurosporene; 2',3'-Dihydroxy-2-nor-beta,beta-carotene-3,4-dione; 3,3'-Dihydroxy-2-nor-13-beta,beta-carotene-4,4'-dione-3-acylate; 3,3'-Dihydroxy-2-nor-13-beta,beta-carotene-4,4'-dione-3,3'-di-acylate; 1,2-Dihydroxyphytofluene; Dihydroxypirardixanthin; 3,3'- Dihydroxyretro-beta-carotene; 3,3'-Dihydroxy-2,3,2',3'-tetradehydro-beta,beta-carotene-4,4'-dione; 3,3'-Dihydroxy-7,8,7';8'-tetradehydro-beta,beta-carotene-4,4'-dione; 3,3'-Dihydroxy-2,3,2',3'-teradehydro-beta,beta-carotene-4,4'-dione dipalmitate; 3,3'-Dihydroxy-7,8,7',8'-tetradehydro-beta,beta-caroten-4-one; 1,1'-Dihydroxy-3,4,3',4'-tetradehydro-1,2,1',2'-tetrahydrod-psi,psi-carotene-2,2'-dione; 3,8'-Dihydroxy-5',6',7',8'-tetrahydro-5'-apo-18'-nor-beta-caroten-6'-one; 1,1'-Dihydroxy-1,2,1'2'-tetrahydro-zeta-carotene; 5,5'-Dihydroxy-5,6,5', 6'-tetrahydro-beta,beta-carotene-3,3'-dione; 3,3'-Dihydroxy-7,8,7';8'-tetrahydro-chi,chi-carotene-6,6'-dione; 9',10'-Dihydro-beta- zeacarotene 3,17'-iol; Diketo-, see also Dioxo- or -dione 2,2'- Diketobacterioruberin; 3,4-Diketo-beta-carotene; 4,4'-Diketo-beta-carotene; 4,4'-Diketo-gamma-carotene; 4,4'-Diketocynthiaxanthin; 3,3'- Diketodehydro-beta-carotene; 4,4'-Diketolycopene; Diketopirardixanthin; 3,3'-Diketoretro-beta-carotene; 3,3'-Diketoretrodehydro-beta-carotene; 2,2'-Diketospirilloxanthin; 4,4'-Diketo-7,8,7',8'-tetrade hydrozeaxanthin; 3,3'-Dimethoxy-beta,beta-carotene; 3,3'-Dimethoxy- beta,[epsi]-carotene; 3,3'-Dimethoxy-gamma-carotene; 3,3'-Dimethoxy-3',4'-dehydro-gamma-carotene; 1,1'-Dimethoxy-3,4-didehydro-1,2,1',2',7',8'-hexahydro-psi,psi-carotene; 1,1'-Dimethoxy-3,4-didehydro-1,2,1',2',7',8'-hexahydro-psi,psi-caroten-2-one; 1,1'-Dimethoxy-3,4-didehydro-1,2,1',2'-tetrahydro-psi,psi-carotene; 1,1'-Dimethoxy-3',4'-didehydro-1,2,1',2'-tetrahydro-psi,psi-caroten-4-one; 1,1'-Dimethoxy-1,2,7,8,1',2'-hexahydro-psi,psi-carotene; 1,1'-Dimethoxy-1,2,7,8,11,12,1',2'-octahydro-psi,psi-carotene; 1,1'-Dimethoxy-3,4,3',4'-tetradehydro-1,2, 1',2'-tetrahydro-psi,psi-carotene; 1,1'-Dimethoxy-3,4,3',4'-tetradehydro-1,2,1',2'-tetrahydro-psi,psi-carotene-2,2'-dione; 1,1'-Dimethoxy-1,2,1',2'-tetrahydro-psi,psi-caroten-20-al; 1,1'-Dimethoxy-1,2,1',2'-tetrahydro-psi,psi-carotene; 1,1'-Dimethoxy-1,2,1',2'-tetrahydro-psi,psi-carotene-4,4'-dione; 1,1'-Dimethoxy-1,2,1',2'-tetrahydrolycopene; 1,1'-Dimethoxy-1,1',2,2'-tetrahydroneurosporene; Dimethylcrocetin; Dimethyl-6,6'-diapocarotene-6,6'-dioate; Dimethyl-8,8'- diapocarotene-8,8'-dioate; Dineapolitanosyl-8,8'-diapocarotene-8,8'- dioate; 2,2-Dinor-beta,beta-carotene-3,4,3',4'tetrone; Dinoxanthin; 3,3'-Dioxi-4-oxo-beta-carotene; Dioxo-, see also Diketo- or - dione 5,6- Dioxo-10'-apo-5,6-seco-beta-caroten-10'-al; 5,6,5',6'-Diseco-beta, beta-carotene 5,6,5',6'-tetrone; 7,8,11,12,13,14,15,7',8',11',12',15'-Dodecahydro-13,15':14,15' biscyclo-15,15'-seco-psi,psi-caroten-15-ol; Dodecahydrolycopene; alpha-Doradecin; beta-Doradecin; alpha-Doradexanthin; beta-Doradexanthin;
Echinenone; Echininone; Eloxanthin; 6-Epikarpoxanthin; 3'- Epilutein; 5,6-Epoxy-alpha-carotene; 5,8-Epoxy-alpha-carotene; 5,8- Epoxy-beta-carotene; 1,2-Epoxy-1,2,7,8,11,12,7',8',11',12'-decahydro-psi,psi-carotene; 5,6-Epoxy-7',8'-didehydro-5,6-dihydro-beta,beta-carotene-3,3'-diol; 5,8-Epoxy-7',8'-didehydro-5,8-dihydro-beta,beta-carotene-3,3'-diol; 1',2'-Epoxy-3',4'-didehydro-1,2'-dihydro-beta,psi-caroten-2-ol; 5',6'-Epoxy-6,7-didehydro-5,6,5',6'-tetrahydro-beta,beta-carotene-3,5,19 (or 19'), 3'-tetrol; 5',6'-Epoxy-6,7-didehydro-5,6,5',6'-tetrahydro-beta,beta-carotene-3,5,3'-triol; 5',6'-Epoxy-6,7-didehydro-5,6,5,6'-tetrahydro-beta,beta-carotene-3,5,3'-triol 3-acetate; 5',8'-Epoxy-6,7-didehydro-5,6,5',8'-tetrahydro-beta,beta-carotene-3,5,3'-triol; 5,6-Epoxy-5,6-dihydro-12'-apo-beta-carotene-3,12'-diol; 5,8-Epoxy-5,8-dihydro-10'-apo-beta-carotene-3,10'-diol; 5,8-Epoxy-5,8-dihydro-12'-apo-beta-carotene-3,12'-diol; 5,6-Epoxy-5,6-dihydro-beta, beta-carotene; 5,8-Epoxy-5,8-dihydro-beta,beta-carotene; 5,6-Epoxy-5,6-dihydro-beta,[epsi]-Ecarotene; 5,8-Epoxy-5,8-dihydro-beta,[epsi]-carotene; 1',2'-Epoxy-1',2'-dihydro-beta,psi-carotene; 1',2'-Epoxy-1',2'-dihydro-[epsi],psi-carotene; 1,2-Epoxy-1,2-dihydro-psi,psi-carotene; 5,6-Epoxy-5,6-dihydro-psi,psi-carotene; 5,6-Epoxy-5,6-dihydro-beta,beta-carotene-3,3'-diol; 5,8-Epoxy-5,8-dihydro-beta, beta-carotene-3,3'-diol; 5,6-Epoxy-5,6-dihydro-beta,[epsi]-carotene-3,3'-diol; 5,6-Epoxy-5,6-dihydro-beta,[epsi]-carotene-3,3'-diol dipalmitate; 5,8-Epoxy-5,8-dihydro-beta,[epsi]-carotene-3,3'-diol; 5,6-Epoxy-5,6-dihydro-beta,[epsi]-carotene-3,3',6'-triol; 5,8-Epoxy-5,8-dihydro-beta,[epsi]-carotene-3,3',6'-triol; 5,6-Epoxy-5,6-dihydro-beta, beta-caroten-2-ol; 5,6-Epoxy-5,6-dihydro-beta,beta-caroten-3-ol; 5',8'-Epoxy-5',8'-dihydro-beta,beta-caroten-3-ol; 5,6-Epoxy-5,6-dihydro-beta,[epsi]-caroten-2-ol; 5,6-Epoxy-5,6-dihydro-beta,psi- caroten-3-ol; 5,8-Epoxy-5,8-dihydro-beta,psi-caroten-3-ol; 5,8-Epoxy- 3,3'-dihydroxy-alpha-carotene; 5,6-Epoxy-3,3'-dihydroxy-7',8'didehydro-5,6,7,8-tetrahydrod-beta,beta-caroten-8-one; 5',6'-Epoxy-3,3'-dihydroxy-7,8-didehydro-5',6'-dihydro-10,11,20-trinor-beta,beta-caroten-19',11'-olide; 5',6'-Epoxy-3,3'-dihydroxy-4,7-didehydro-5',6'-dihydro-10,11,20-trinor-beta,beta-caroten-19',11'-olide 3-acetate; 5', 6'-Epoxy-3,3'-dihydroxy-7,8-didehydro-5',6'-dihydro-10,11,20-trinor-beta,beta-caroten-19',11'-olide 3-acetate; 5,6-Epoxy-3,3'-dihydroxy-5,6-dihydro-beta,chicaroten-6'-one; 5,8-Epoxy-3,3'-dihydroxy-5,8-dihydro-beta,chi-caroten-6'-one; 5,6-Epoxy-3,3'-dihydroxy-5,6,7',8'-tetrahydro-beta,[epsi]-caroten-11',19'-olide; 1',2'-Epoxy-2'-(2,3-epoxy-3-methylbutyl)-2-(3-hydroxy-3-methylbutyl)-3',4'-didehydro-1,2,1',2'-tetrahydro-psi,psi-caroten-1-ol; 1,2-Epoxy-1,2,7,8,7',8'-hexahydro-psi,psi-carotene; 5,6-Epoxy-3-hydroxy-8'-apo-beta-caroten-8'-al; 5, 6-Epoxy-5,6-dihydro-10'-apo-beta-carotene-3,10'-diol; 5,8-Epoxy-3-hydroxy-gamma-carotene; 5,8-Epoxy-3-hydroxy-5,8-dihydro-8'-apo-beta- caroten-8'-al; 5,6-Epoxy-3-hydroxy-5,6-dihydro-10'-apo-beta-caroten-10'- al 502; 5,6-Epoxy-3-hydroxy-5,6-dihydro-12'-apo-beta-caroten-12'-al; 5, 6-Epoxy-3-hydroxy-5,6,7',8'-tetrahydro-7'-apo-beta-caroten-8'-one; 5,8- Epoxylutein; 1,2-Epoxy-1,2,7,8,11,12,7',8'octahydro-psi,psi-carotene; 1,2-Epoxy-1,2,7,8,7',8',11',12'octahydro-psi,psi-carotene; 1',2'-Epoxy-7,8,11,12,1',2', 7', 8'-octahydro-beta,psi-caroten-2-ol; 1,2- Epoxyphytoene; 5,8-Epoxyrubixanthin; 5',8'-Epoxy-5,6,5',8'-tetrahydro-beta,beta-carotene-3,5,6,3'-tetrol; 5',6'-Epoxy-5,6,5',6'-tetrahydro-beta,beta-carotene-3,5,6,3'-tetrol; 5,6-Epoxy-3',4',7',8'-tetradehydro-5,6-dihydro-beta,beta-caroten-4-one; 5,6-Epoxy-3,3',5', 19'-tetra-hydroxy-6',7'-didehydro-5,6,7,8,5',6'-hexahydro-beta,beta-caroten-8-one 3'-acetate 19'-hexanoate; 5,6-Epoxy-3,3',5'-trihydroxy-6',7'-didehydro-5,6,7,8,5',6'-hexahydro-beta,beta-caroten-8-one; 5,6- Epoxy-3,3',5'-trihydroxy-6',7'-didehydro-5,6,7,8,5',6'-hexahydro-beta, beta-caroten-8-one 3'-acetate; 5',6'-Epoxy-3,5,3'-trihydroxy-6,7-didehydro-5,6,5',6'-tetrahydro-10,11,20-trinor-beta,beta-caroten-19',11'-olide; 5',6'-Epoxy-3,5,3'-trihydroxy-6,7-didehydro-5,6,5',6'-tetrahydro-10,11,20-trinor-beta,beta-caroten-19',11'-oxide 3-acetate; 4',5'-Epoxy-3,6,3'-trihydroxy-7,8,4',5',7',8'-hexahydro-gamma,[epsi]-caroten-8-one; 5,6-Epoxyzeaxanthin; 5,8-Epoxyzeaxanthin; Eschscholtzxanthin; Eschscholtzxanthone; 4'-Ethoxy-beta,beta-caroten- 4-one; 4'-Ethoxy-4-keto-beta-carotene; Euglenanone; Euglenarhodon; Eutreptiellanone;
Flavacin; Flavochrome; Flavorhodin; Flavoxanthin; Flexixanthin; Foliachrome; Foliaxanthin;
Gazaniaxanthin; beta,D-Gentiobiosyl beta,D-glucosyl 8,8'-diapocarotene-8,8'-dioate; Gentiobiosyl hydrogen-8,8'-dioate; Gentiobiosyl neapolitanosyl 8,8'-diapocarotene-8,8'-dioate; beta,D- Glucosyl hydrogen-4,4'-diapocarotene-4,4'-dioate; 4'-beta,D-Glucosyl 4- hydrogen-7',8'-dihydro-4,4'-diapocarotene-4,4'-dioate; beta,D-Glucosyl hydrogen-8,8'-diapocarotene-8,8'-dioate; beta,D-Glucosyl methyl-8,8'-diapocarotene-8,8'-dioate; Glucopyranosyloxy (see Glucosyloxy); 4- Glucosyloxy-4,4' diaponeurosporene; 1'-Glucosyloxy-3',4'-didehydro-1',2'- dihydro-beta,psi-carotene; 1-Glucosyloxy-3,4-didehydro-1,2-dihydro- psi,psi-carotene; 2'-Glucosyloxy-3',4'-didehydro-1',2'-dihydro-beta, psi-carotene-3,1'-diol; 1'-Glucosyloxy-3',4'-didehydro-1',2'-dihydro-beta,psi-caroten-3-ol; 1'-Glucosyloxy-3',4'-didehydro-1',2'-dihydro-beta,psi-caroten-2'-ol; 1'-Glucosyloxy-3',4'-didehydro-1',2'-dihydro-beta,psi-caroten-4-one; 1-Glucosyloxy-3,4-didehydro-1,2,7',8'-tetrahydro-psi,psi-carotene; 1-Glucosyloxy-1,2-dihydro-psi,psi-caroten-20-al; 1-Glucosyloxy-1',2'-dihydro-beta,psi-carotene; 1'-Glucosyloxy-1',2'-dihydro-[phi],psi-carotene; 1-Glucosyloxy-1,2-dihydro-psi,psi-carotene; 4-Glucosyloxy-7',8'-dihydro-4,4'-diapocarotene; 1'-Glucosyloxy-2'-hydroxy-3',4'-didehydro-1',2'-dihydro-beta,psi-caroten- 4-one; 2-(4-Glucosyloxy-3-methyl-2-butenyl)-2'-(4-hydroxy-3-methyl-2- butenyl)-gamma,gamma-carotene; 2-(4-Glucosyloxy-3-methyl-2-butenyl)-2'-(4-hydroxy-3-methyl-2-butenyl)-[epsi],[epsi]-carotene; 2-(4-Glucosyloxy-3-methyl-2-butenyl)-2'-(4-hydroxy-3-methyl-2-butenyl)-7,8-dihydro-[epsi],[epsi]-carotene; 2'-(4-Glucosyloxy-3-methyl-2-butenyl)-2- (3-methyl-2-butenyl)-[epsi],[epsi]-caroten-18-ol; 2-[3-(Glucosyloxy)-3-methylbutyl]-2'-(3-hydroxy-3-methylbutyl)-3,4,3',4'-tetradehydro-1,2,1',2'-tetrahydro-psi,psi-carotene-1,1'-diol; 1'-Glucosyloxy-3,4,3',4'-tetradehydro-1',2'-dihydro-beta,psi-carotene; Glycymerin; Guaraxanthin;
Halocynthiaxanthin; Helenien; Heteroxanthin; Hexadecahydrolycopene; 2,3,2',3',45'-Hexadehydro-4,5'-retro-beta,beta-carotene; 1,2,7,8,11,12-Hexahydro-psi,psi-carotene; 1,2,7,8,1',2'- Hexahydro-psi,psi-carotene; 1,2,7,8,7',8'-Hexahydro-psi,psi- carotene; 7,8,11,12,7',8'-Hexahydro-psi,psi-carotene; 7,8,11,12,7',8'- Hexahydro-beta,psi-caroten-2-ol; 15,7',8',11',12',15'-Hexahydro-beta,psi-caroten-2-ol; 1,2,7',8',11',12'-Hexahydro-psi,psi-caroten-1-ol; 7,8,11,12,7',8'-Hexahydro-psi,psi-caroten-16-ol; 7,8,11,12,7',8'-Hexahydro-4,4'-diapocarotene; 1,2,7,8,11,12-Hexahydrolycopene; 1',2',7', 8'11',12'-Hexahydrolycopene; 7,8,11,12,7',8'-Hexahydrolycopene; 7,8,1',2', 7',8'-Hexahydrolycopene; 3,4,3',4',7',8'-Hexahydrospirilloxanthin; 19'-Hexanoyloxyfucoxanthin; 19-Hexanoyloxyparacentrone; 1-Hexosyl-1,2-dihydro- 3,4-didehydroapo-8'-lycopenol; O-Hexosyl-1'-hydroxy-1',2'-dihydro-gamma- carotene; O-Hexosy-1-4-keto-1'-hydroxy-1',2'-dihydro-3',4'-didehydro- gamma-carotene; Hopkinsiaxanthin; Hydroxy-, see also Monohydroxy-, OH or -ol3-Hydroxy-beta-apo-2-carotenal; 3-Hydroxy-8'-apo-beta-caroten- 8'-al; 3-Hydroxy-10'-apo-beta-caroten-10'-al: 3-Hydroxy-12'-apo-beta- caroten-12'-al; 3-Hydroxy-8'-apo-[epsi]-caroten-8'-al; 3-Hydroxy-8'-apo- beta-caroten-8'-oic acid; 9'-Hydroxy-9'-apo-beta-caroten-3-one; 9'-Hydroxy-9'-apo-[epsi]-caroten-3-one; Hydroxyasteroidenone; 3-Hydroxycanthaxanthin; 3-Hydroxy-beta,psi-caroten-18'-al; 3-Hydroxy-alpha-carotene; 3'-Hydroxy-alpha-carotene; 4-Hydroxy-alpha-carotene; 2-Hydroxy-beta-carotene; 3-Hydroxy-beta-carotene; 4-Hydroxy-beta- carotene; 3-Hydroxy-gamma-carotene; 4'-Hydroxy-gamma-carotene; 3- Hydroxy-[delta]-carotene; 2-Hydroxy-beta,beta-carotene-4,4'-dione; 3- Hydroxy-beta,beta-carotene-4,4'-dione; 3'-Hydroxy-beta,beta-carotene-3,4-dione; 4'-Hydroxy-beta,beta-carotene-3,4-dione; 3-Hydroxy-beta,[epsi]-carotene-4,3'-dione; 3'-Hydroxy-beta,[epsi]- carotene-3,4-dione; 3-Hydroxy-beta,chi-carotene-3',6'-dione; 3'-Hydroxy-beta,beta-carotene-3,4,4'-trione; 2'-Hydroxy-beta,beta-caroten-2-one; 2-Hydroxy-beta,beta-caroten-4-one; 3-Hydroxy-beta, beta-caroten-4-one; 3'-Hydroxy-beta,beta-caroten-4-one; 4'-Hydroxy-beta,beta-caroten-4-one; 3-Hydroxy-beta,[epsi]-caroten-4-one; 3- Hydroxy-beta,[epsi]-caroten-3'-one; 3'-Hydroxy-beta,chi-caroten-6'-one; 3Hydroxy-beta,psi-caroten-4'-one; 3-Hydroxy-beta,psi-caroten-4-one; 3-Hydroxy-[epsi],[epsi]-caroten-3'-one; 3'-Hydroxy-psi,psi-caroten-4-one; 3-Hydroxycitranaxanthin; 3-Hydroxy-7,8-dehydro-alpha-carotene; 3'-Hydroxy-3,4-dehydro-beta-carotene; 3-Hydroxy-3',4'-dehydro-gamma-carotene; 4-Hydroxy-4,4'-diaponeurosporene; 3-Hydroxy-2,3-didehydro-beta,beta-carotene-4,4'-dione; 2'-Hydroxy-3,4-didehydro-beta,beta-caroten-2-one; 3-Hydroxy-2,3-didehydro-beta,beta- caroten-4-one; 3-Hydroxy-2,3-didehydro-beta,[epsi]-caroten-4-one; 3-Hydroxy-2,3-didehydro-beta,X-caroten-4-one; 3-Hydroxy-2,3-didehydro-beta,[phi]-caroten-4-one; 3-Hydroxy-3',4'-didehydro-beta,psi-caroten-4-one; 3-Hydroxy-7,8-didehydro-7',8'-dihydro-7'-apo-beta-carotene-4,8'-dione; 3-Hydroxy-7,8-didehydro-7',8'-dihydro-7'-apo-beta-caroten-8'-one; 3-Hydroxy-7',8'-didehydro-7,8-dihydro-chi,X-carotene-6, 8-dione; 1'-Hydroxy-3',4'-didehydro-1',2'-dihydro-beta,psi-caroten-4-one; 1'-Hydroxy-3',4'-didehydro-1',2'-dihydro-beta,psi-caroten-2'-one; 2'-Hydroxy-3',4'-didehydro-1',2'-dihydro-beta,psi-caroten-4-one; 5-Hydroxy-4',5'-didehydro-4,5-dihydro-4,5'-retro-beta,beta-carotene-3, 3'-dione; 3'-Hydroxy-2',3'-didehydro-2-nor-beta,beta-carotene-3,4,4'-trione; 3'-Hydroxy-4',5'-didehydro-4,5'-retro-beta,beta-caroten-3-one; 3-Hydroxy-5,8,5',8'-diepoxy-beta-carotene; 3-Hydroxy-7',8'-dihydro-7'-apo-beta-caroten-8'-one; 3-Hydroxy-5',6'-dihydro-5'-apo-18'-nor-beta-caroten-6'-one; 1-Hydroxy-1,2-dihydro-psi,psi-caroten-20-al; 1'-Hydroxy-1',2'-dihydro-gamma-carotene; 3-Hydroxy-7,8-dihydro-chi,X-carotene-6,8-dione; 4'-Hydroxy-5',6'-dihydro-beta,beta-caroten-4-one; 1'-Hydroxy-1',2'-dihydro-beta,psi-caroten-4-one; 8'-Hydroxy-7',8'-dihydrocitranaxanthin; 4-Hydroxy-7',8'-dihydro-4,4'-diapocarotene; 4'-Hydroxy-5',6'-dihydroechinenone; 1'-Hydroxy-1',2'-dihydro-2-isopentenyl-2'-(hydroxyisopentenyl)torulene; 1-Hydroxy-1,2-dihydrolycopene; 1-Hydroxy-1,2-dihydroneurosporene; 1'-Hydroxy-1',2'-dihydroneurosporene; 1-Hydroxy-1,2-dihydrophytoene; 1 (or 1')-Hydroxy-1,2 (or 1',2')-dihydrophytofluene; 8'-Hydroxy-7',8'-dihydroreticulataxanthin; 1'-Hydroxy-1',2'- dihydrospheroidene; 2'-Hydroxy-1',2'-dihydrotorulene; 2-Hydroxy-1',2'-dihydrotorulene-1',2'-oxide; 5-Hydroxy-5,6-dihydrozeaxanthin; 3-Hydroxy-3',4'-diketo-alpha-carotene; 3-Hydroxy-4,4'-diketo-beta-carotene; 3'-Hydroxy-3,4-diketo-beta-carotene; 2'-Hydroxy-3,1'-dimethoxy-3',4'-didehydro-1',2'-dihydro-beta,psi-caroten-4-one; 4-Hydroxy-3',4'-dioxo-beta-carotene; 2-Hydroxyechinenone; 3-Hydroxyechinenone; 3'- Hydroxyechinenone; 4'-Hydroxyechinenone; 3-Hydroxy-5,8-epoxy-beta-carotene; 3'-Hydroxy-3,6-epoxy-5,6-dihydro-beta,[epsi]-caroten-4-one; 3'-Hydroxy-3,6-epoxy-7',8'-didehydro-5,6-dihydro-beta,beta-caroten-4-one; 3'-Hydroxyeuglenanone; 2'-Hydroxyflexixanthin; 1-Hydroxy-1,2,7',8',11',12'-hexahydrolycopene; 1'-Hydroxy-3,4,1',2',11', 12'hexahydrospheroidene; 2-(4-Hydroxy-3-hydroxymethyl-2-butenyl)-2'-(3-methyl-2-butenyl)-beta,beta-carotene; 3-Hydroxyisorenieratene; 3- Hydroxy-4-keto-alpha-carotene; 3-Hydroxy-3'-keto-alpha-carotene; 3-Hydroxy-4-keto-beta-carotene; 3-Hydroxy-4'-keto-beta-carotene; 4-Hydroxy-4'-keto-beta-carotene; 1'-Hydroxy-2'-keto-1',2'-dihydrotorulene; 3-Hydroxy-3'-keto-retrodehydrocarotene; 19-Hydroxylutein; 16- Hydroxylycopene; 3-Hydroxy-3'-methoxy-beta-carotene; 1'-Hydroxy-1-methoxy-3,4-didehydro-1,2,1',2',7',8'-hexahydro-psi,psi-caroten-2-one; 1'-Hydroxy-1-methoxy-1,2,1',2',7',8'-hexahydro-psi,psi-caroten-4-one; 1'-Hydroxy-1-methoxy-3,4,3',4'-tetradehydro-1,2,1',2'-tetrahydro-psi, psi-caroten-2-one; 1'-Hydroxy-1-methoxy-1,2,1',2'-tetrahydro-psi,psi-caroten-4-one; 2-(4-Hydroxy-3-methyl-2-butenyl)-beta,beta-carotene; 2-(4-Hydroxy-3-methyl-2-butenyl)-[epsi],psi-carotene; 2-(3-Hydroxymethyl-but-2-enyl)-7',8'-dihydro-[delta]-carotene; 2-(4-Hydroxy-3-methyl-2-butenyl)-7',8'-dihydro-[epsi],psi-carotene; 2-(4-Hydroxy-3-methyl-2-butenyi)-2'-(3-methyl-2-butenyl)-[epsi],[epsi]-carotene; 2-(4Hydroxy-3-methyl-2-butenyl)-2'-(3-methyl-2-butenyl)-[epsi],[epsi]- caroten-18-ol; 2'-(4-Hydroxy-3-methyl-2-butenyl)-2-(3-methyl-2-butenyl)-3',4'-didehydro-1',2'-dihydro-beta,psi-caroten-1'-ol; 2 (or 2')-(4-Hydroxy-3-methyl-2-butenyl)-2' (or 2)-(3-methyl-2-butenyl)-3',4'- didehydro-1',2'-dihydro-[epsi],psi-caroten-1'-ol; 2'-(4-Hydroxy-3-methyl-2-butenyl)-2-(3-methyl-2-butenyl)-7,8 (or 7',8')-dihydro-[epsi], [epsi]-caroten-18-ol; 2-(4-Hydroxy-3-methyl-2-butenyl)-7,8,7',8'- tetrahydro-[epsi],psi-carotene; 2-(4-Hydroxy-3-methyl-2-butenyl)-7',8',11',12'-tetrahydro-[epsi],psi-carotene; 16-(3-Hydroxy-3-methylbutyl)-16'-(3-methyl-2-butenyl)-7,8,11,12,15,7',8',11',12',15'-decahydro-psi, psi-carotene; 2-(3-Hydroxy-3-methylbutyl)-2'-(3-methyl-2-butenyl)-3,4, 3',4'-tetradehydro-1,2,1',2'-tetrahydro-psi,psi-carotene-1,1'-diol; 2- Hydroxy-monocyclic-phytofluene; 4-Hydroxymyxoxanthophyll; Hydroxyneurosporene; 15-Hydroxy-7',8',9',10',11', 12',13',14'-octahydro- 6'-apo-beta-caroten-7'-one; 1'-Hydroxy-3,4,7,8,1',2',11',12'-octahydrospheroidene; 3'-Hydroxy-4-oxo-beta-carotene; 3-Hydroxy-4-oxo-2,3-dehydro-beta-carotene; 4'-Hydroxy-3-oxoechinenone; Hydroxyphytoene; Hydroxyphytofluene; 4'-Hydroxy-4-oxo-pirardixanthin; 2-Hydroxyplectaniaxanthin; 3-Hydroxy-4,5'-retro-5'-apo-beta-caroten-5'-one; 3-Hydroxy-4',12'-retro-beta,beta-carotene-3',12'-dione; 3'-Hydroxyrubixanthin; 3'-Hydroxy-5,6-seco-beta,beta-carotene-5,6-dione; 3-Hydroxysemi-beta-carotenone; 3-Hydroxysintaxanthin; Hydroxyspheroidene; Hydroxyspheroidenone; Hydroxyspirilloxanthin; 8'-Hydroxy-5',6',7',8'-tetrahydro-5'-apo-18'-nor-beta-caroten-6'-one; 4'-Hydroxy-5,6,5',6'-tetrahydro-beta,beta-caroten-4-one; 1-Hydroxy-3,4, 3',4'-tetradehydro-1,2-dihydro-psi,psi-caroten-2-one; 1-Hydroxy-1,2, 7',8'-tetrahydrolycopene; 1'-Hydroxy-3,4,1',2'-tetrahydrospheroidene; 3- Hydroxytorulene; 16'-Hydroxytorulene; 18'-Hydroxytorulene; 3-Hydroxy-3', 4'-triketo-beta-carotene; 3-Hydroxy-beta-zeacarotene; 5- Hydroxyzeaxanthin;
Idoxanthin; Isoagelaxanthin A; Isobixin; Isocarotene; Iso- zeta-carotene; Iso-zeta-carotene; Isocrocetin; Isocryptoxanthin; Isofucoxanthin; Isofucoxanthinol; Isolutein; Isomethylbixin; Isomytiloxanthin; 2-Isopentenyl-3,4-dehydrorhodopin; Isorenieratene; beta-Isorenieratene; 3,3'-Isorenieratenediol; 3-Isorenieratenol; Isotedaniaxanthin; Isotedanin; Isozeaxanthin;
Karpoxanthin; Keto-, see also oxo or -one Ketocapsanthin; 4-Ketocapsanthin; 4-Keto-alpha-carotene; 4-Keto-beta-carotene; 4-Keto-gamma-carotene; 4-Ketocynthiaxanthin: 4-Keto-3',4'-dehydro-beta-carotene; 4-Keto-1',2'-dihydro-1'-hydroxytorulene; 2-Keto-7',8'-dihydrorhodovibrin; 4-Keto-3,3'-dihydroxy-alpha-carotene; 4'-Keto-3-hydroxy-gamma-carotene; 4-Keto-3'-hydroxylycopene; 4-Ketolutein 332 4- Ketomyxol 2'-(methylpentoside); 4-Ketomyxoxanthophyll; 2-Keto-OH- spirilloxanthin; 4-Ketophleixanthophyll; 2-Ketorhodovibrin; 4'- Ketorubixanthin; 2-Ketospirilloxanthin; 4-Ketotorulene; 4-Ketozeaxanthin;
Lactucaxanthin; Latochrome; Latoxanthin; leprotene; Lilixanthin; Loniceraxanthin; Loroxanthin; Lusomycin; Lutein; Lutein dimethyl ether; Lutein dipalmitate; Lutein epoxide; Luteochrome; Luteol; Luteoxanthin; Lycopenal; Lycopen-20-al; Lycopene; Lycopene-16,16'-diol; Lycopene 1,2-epoxide; Lycopene 5,6-epoxide; Lycopen-16-ol; Lycopen-20-ol; Lycopersene; Lycophyll; Lycoxanthin;
Mactraxanthin; Manixanthin; 1-Mannosyloxy-3,4-didehydro-1,2-dihydro-8'-apo-psi-caroten-8'-ol; 3'-Methoxy-beta,beta-caroten-3-ol; 3-Methoxy-beta,X-carotene; 1-Methoxy-1,2,7,8,11,12,7',8',11',12'-decahydro-psi,psi-carotene; 1'-Methoxy-1,2,7,8,11,12,1',2',7',8'-decahydro-psi,psi-caroten-1-ol; 1-Methoxy-3,4-didehydro-1,2-dihydro-psi,psi-caroten-20-al; 1'-Methoxy-3',4'-didehydro-1',2'-dihydro-beta,psi-carotene; 1-Methoxy-3,4-didehydro-1,2-dihydro-psi,psi-carotene; 1-Methoxy-3,4-didehydro-1,2,7',8',11',12'-hexahydro-psi,psi-carotene; 1'-Methoxy-3',4'-didehydro-1,2,7,8,1',2'-hexahydro-psi,psi-caroten-1-ol; 1-Methoxy-3,4-didehydro-1,2,7',8'-tetrahydro-psi,psi-carotene; 1'-Methoxy-3',4'-didehydro-1,2,1',2'-tetrahydro-psi,psi-caroten-1-ol; 1-Methoxy-3,4-didehydro-1,2,7',8'-tetrahydro-psi,psi-caroten-2-one; 1-Methoxy-1,2-dihydro-psi,psi-caroten-20-al; 1-Methoxy-1,2-dihydro-psi,psi-carotene; 1'-Methoxy-1',2'-dihydro-beta,psi-caroten-4'-one; 1'-Methoxy-1',2'-dihydro-X,psi-caroten-4'-one; 1-Methoxy-1,2-dihydro-psi, psi-caroten-4-one; 1'-Methoxy-1',2'-dihydro-3',4'-dehydro-gamma-carotene; 1-Methoxy-1,2-dihydro-3,4-dehydrolycopene; 1-Methoxy-1,2-dihydro-3,4-didehydrolycopen-20-al; 1-Methoxy-1,2-dihydrolycopene; 4-Methoxy-5,6-dihydrolycopene; 1-Methoxy-1,2-dihydroneurosporene; 1-Methoxy-1,2-dihydrophytoene; 1-Methoxy-1,2-dihydrophytofluene; 1'-Methoxy-1',2'-dihydrospheroidene; 3-Methoxy-19,3'-dihydroxy-7,8-didehydro-beta,chicarotene-6',8'-dione; 1-Methoxy-1,2,7',8',11',12'-hexahydro-psi,psi-carotene; 1'-Methoxy-1,2,7,8,1',2'-hexahydro-psi,psi-caroten-1-ol; 1-Methoxy-1,2,7',8'11',12'-hexahydro-psi,psi-caroten-4-one; 1-Methoxy-1'-hydroxy-1,2,1',2'-tetrahydrophytofluene; 1-Methoxy-2-keto-7',8'-dihydro-3,4-dehydrolycopene; Methoxylycopenal; 1-Methoxy-1,2,7,8,7',8',11', 12'-octahydro-psi,psi-carotene; 1'-Methoxy-1,2,7,8,11,12,1',2'-octahydro-psi,psi-caroten-1-ol; 1-Methoxy-4-oxo-1,2-dihydro-8'-apo-psi-caroten-8'-al; 1-Methoxy-4-oxo-1,2-dihydro-12'-apo-psi-caroten-12'-al; Methoxyphytoene; Methoxyphytofluene; Methoxyspheroidene; 1'-Methoxy-3,4,3',4'-teradehydro-1,2,1',2'-tetrahydro-psi,psi-caroten-1-ol; 1-Methoxy-1,2,7',8'-tetrahydro-psi,psi-carotene; 1-Methoxy-1,2,7',8'-tetrahydro-psi,psi-caroten-4-one; 1-Methoxy-1,2,7',8'-tetrahydro-3, 4-dehydrolycopene; 3Methoxy-19,3',8'-trihydroxy-7,8-didehydro-beta,chi -caroten-6'-one; Methyl 4'-apo-beta-caroten-4'-oate; Methyl 8'-apo- beta-caroten-8'-oate; Methyl 6'-apo-psi-caroten-6'-oate; Methyl apo- 6'-lycopenoate; Methylbixin; 2-(3-Methyl-2-butenyl)-beta,beta-caroten-18(or 18')-ol; 2-(3-Methyl-2-butenyl)-3,4-didehydro-1,2-dihydro-psi, psi-caroten-1-ol; 2-(3-Methyl-2-butenyl)-7,8,7',8'-tetrahydro-[epsi], psi-caroten-18-ol; Methyl 3',4'-didehydro-beta,psi-caroten-16'-oate; Methyl 1-hexosyl-1,2-dihydro-3,4-didehydro-apo-8'-lycopenoate; Methyl hydrogen 6,6'-diapocarotene-dioate; Methyl 1-mannosyloxy-3,4-didehydro-1,2-dihydro-8'-apo-psi-caroten-8'-oate; Methyl 1'-methoxy-4'-oxo-1',2'-dihydro-X,psi-caroten-16 (or 17 or 18)-oate; 2'-(O-Methyl-5-C-methylpentosyloxy)-3',4'-didehydro-1',2'-dihydro-beta,psi-carotene-3,1'-diol; Metridene; Mimulaxanthin; Monadoxanthin; Monoanhydrobacterioruberin; Monodehydro-beta-carotene; Monodehydrolycopene; Monodemethyl(ated) spirilloxanthin; Monoepoxy-, see Epoxy-Monohydroxy cyclophytoene; Monohydroxy cyclophytofluene; Mutatochrome; Mutatoxanthin; Mytiloxanthin; Mytiloxanthinone; Myxobactin; Myxobactone; Myxol 2'-glucoside; Myxol 2'-O-methyl-methylpentoside; Myxol 2'-rhamnoside; Myxoxanthin; Myxoxanthol; Myxoxanthophyll;
Neocarotene; Neochrome; Neo-beta-carotene B; Neo-beta- cryptoxanthin A; Neoxanthin; Neoxanthin 3-acetate; Neurosporaxanthin; Neurosporaxanthin methyl ester; Neurosporene;
Octahydro-beta-carotene; 1,2,7,8,11,12,7,8'-Octahydro-psi,psi-carotene; 7,8,11,12,7',8',11',12'-Octahydro-psi,psi-carotene; 1,2,7,8,11,12,7',8'-Octahydro-psi,psi-carotene-1,2-diol; 1,27,8,1',2',7',8'-Octahydro-psi,psi-carotene-1,1'-diol; 1,2,7,8,11,12, 7',8'-Octahydro-psi,psi-caroten-1-ol; 7,8,11,12,7',8',11',12'-Octahydro-beta,psi-caroten-2-ol; 1,2,7,8,7',8',11',12'-Octahydro-psi,psi-caroten-1-ol; 7,8,11,12,7',8',11',12'-Octahydro-4,4'-diapocarotene; Octahydrolycopene; 5,6,7,8,5',6',7',8'-Octahydrolycopene; 7,8,11,12,7',8',11',12'-Octahydrolycopene; 3,4,3',4',7',8',11',12'-Octahydrospirilloxanthin; OH, see also Hydroxy- or -ol OH-Chlorobactene; OH-Chlorobactene glucoside; OH-Lycopene; 2-OH-Monocyclophytoene; 2-OH- Monocyclophytofluene; OH-Neurosporene; OH-Okenone; OH-P 481; OH-P 482; OH- P 511; OH-R; OH-Rhodopin; OH-Sintaxanthin 5,6-epoxide; OH-Spheroidene; OH- Spheroidenone; OH-7,8,11,12-Tetrahydrolycopene; OH-Y; Okenone; Ophioxanthin; Oscillaxanthin; Oscillol 2,2'-di(O-methyl-methylpentoside); Oscillol 2,2'-dirhamnoside; Ovoester; Oxo-, see also Keto or -one 3- Oxocanthaxanthin; 4'-Oxo-4,4'-diapocaroten-4-oic acid; 8'-Oxo-8,8'- diapocarotenoic acid; 3-Oxoechinenone; 4-Oxosaproxanthin; 16'-Oxotorulene; 6'-Oxychrysanthemaxanthin; P 412; P 444; P 450; P 452; P 481; P 500; P 518; 1'-[(chi-O-Palmitoyl-beta,D-glucosyl)oxy]-3',4'-didehydro-1',2'-dihydro-beta, psi-caroten-2'-ol; Papilioerythrin; Papilioerythrinone; Paracentrone; Parasiloxanthin; Pectenol; Pectenolone; Pectenoxanthin; Pentaxanthin; Peridinin; Peridininol; Persicachrome; Persicaxanthin; Phillipsiaxanthin; Philosamiaxanthin; Phleixanthophyll; Phleixanthophyll palmitate; Phoeniconone; Phoenicopterone; Phoenicoxanthin; Physalien; Physoxanthin; Phytoene; C (30)-Phytoene; Phytoene 1,2-(ep)oxide; Phytoenol; Phytofluene; Phytofluene epoxide; Phytofluenol; Pigment R; Pigment X; Pigment Y; Plectaniaxanthin; Poly-cis-gamma-carotene; Poly-cis-psi- carotene; Poly-cis-lycopene; Prasinoxanthin; Prelycopersene pyrophosphate; Prephytoene pyrophosphate; Pro-gamma-carotene; Prolycopene; Proneurosporene; Protetrahydrolycopene; Pseudo-alpha-carotene; Pyrenoxanthin; Pyrrhoxanthin; Pyrrhoxanthinol;
7-cis: Renieracistene; Renierapurpurin; Renieratene; Reticulaxanthin; Retinylidenetiglic acid; Retrobisdehydro(-beta-) carotene; Retrodehydro(-beta-)carotene; Retrodehydro-gamma-carotene; Retrodehydrozeaxanthin; Rhamnopyranosyloxy-, see Rhamnosyloxy-2'-O-Rhamnosyl-4-ketomyxol; 2'-O-Rhamnosylmyxol; 3'-Rhamnosyloxy-beta,beta-caroten-3-ol; 1-Rhamnosyloxy-3',4'-didehydro-1',2'-dihydro-beta,psi-carotene; 2'-Rhamnosyloxy-3',4'-didehydro-1',2'-dihydro-beta,psi-carotene-3,1'-diol; 2'-Rhamnosyloxy-3',4'-didehydro-1',2'-dihydro-beta, psi-carotene-3,4,1'-triol; 1'-Rhamnosyloxy-3',4'-didehydro-1',2'-dihydro-beta,psi-caroten-3-ol; Rhodoauranxanthin; Rhodopin; Rhodopin(- 20-)al; Rhodopinal glucoside; Rhodopin glucoside; Rhodopinol; Rhodopurpurin; Rhodotorulene; Rhodovibrin; Rhodoviolascin; Rhodoxanthin; Roserythrin; Rubichrome; Rubixanthin; Rubixanthin 5,6-epoxide; Rubixanthone;
Salmon acid; Salmoxanthin; Saproxanthin; Sarcinaxanthin; Sarcinaxanthin diglucoside; Sarcinaxanthin monoglucoside; Sarcinene; 5,6- Seco-beta,beta-carotene-5,6-dione; 5,6-Seco-beta,[epsi]-carotene-5,6-dione; Semi-alpha-carotenone; Semi-beta-carotenone; Sidnyaxanthin; Sintaxanthin; Siphonaxanthin; Siphonein; Sodium-3,19-dihydroxy-7,8-di-dehydro-beta,chi-carotene-3',6'-dione 3-sulfate; Sodium-3,19-dihydroxy-3',6'-dioxo-7,8-didehydro-beta,chi-caroten-17'-al 3-sulfate; Sodium-3,19,3'-trihydroxy-7,8-didehydro-6'-oxo-beta,chi-caroten-17'-oate 3-sulfate; Sodium-3,19,17'-trihydroxy-7,8-didehydro-beta,chi-carotene-3',6'-dione 3-sulfate; Sphaerobolin; Spheroidene; Spheroidenone; Spirilloxanthin; Sulcatoxanthin;
Tangeraxanthin; Taraxanthin; Taraxanthin dipalmitate; Taraxien; Tareoxanthin; Tedaniaxanthin; Tedanin; Ternstroemiaxanthin; Tethyatene; 7,8,7',8'-Tetradehydroastaxanthin; 3,4,3',4'-Tetradehydro-beta,beta-carotene; 3,4,3',4'-Tetradehydro-psi,psi-carotene; 7,8,7',8'-Tetradehydro-beta,beta-carotene-3,3'-diol; 3,4,3',4'-Tetradehydro-beta,beta-carotene-2,2'-dione; 3',4',7',8'-Tetradehydro- beta,beta-caroten-3-ol; 3,4,3',4'Tetradehydrolycopene; 6,7,6',7'-Tetradehydro-5,6,5',6'-tetrahydro-beta,beta-carotene-3,3'-diol; 6,7, 6',7'-Tetradehydro-5,6,5',6'-tetrahydro-beta,beta-carotene-3,5,3',5'-tetrol; 7,8,7',8'-Tetradehydrozeaxanthin; 3,4,3',4'-Tetradehydrobisanhydrobacterioruberin; 5,6,5',6'-Tetrahydrocanthaxanthin; 7,8,7',8'-Tetrahydrocapsorubin; Tetrahydro-beta-carotene; 7,8,7',8'-Tetrahydro-beta,beta-carotene; 7',8',11',12'-Tetrahydro-beta,psi-carotene; 7',8',11',12'-Tetrahydro-gamma-carotene; 7',8',11',12'-Tetrahydro-gamma,psi-carotene; 1,2,7,8-Tetrahydro-psi,psi-carotene; 1,2,1',2'-Tetrahydro-psi,psi-carotene; 7,8,11,12-Tetrahydro-psi,psi-carotene; 7,8,7',8'-Tetrahydro-psi,psi-carotene; 5,6,5',6'-Tetrahydro-beta,beta-carotene-4,4'-diol; 7,8,7',8'-Tetrahydro-beta,beta-carotene-3,3'-diol; 7',8',9',10'-Tetrahydro-beta,psi-carotene-3,17'-diol; 1,2,1',2'-Tetrahydro-psi,psi-carotene-1,1'-diol; 5,6,5',6'-Tetrahydro-beta,beta-carotene-4,4'-dione; 5,6,5',6'- Tetrahydro-beta,beta-carotene-3,5,6,3',5',6'-hexol; 1,2,7,8- Tetrahydro-psi,psi-caroten-1-ol; 1,2,7',8'-Tetrahydro-psi,psi- caroten-1-ol; 7,8,11,12-Tetrahydro-4,4'-diapocarotene; 7,8,7',8'- Tetrahydro-4,4'-diapocarotene; Tetrahydrolycopene; 1,2,1',2'-Tetrahydrolycopene; 5,6,5',6'-Tetrahydrolycopene; 7,8,11,12-Tetrahydrolycopene; 7,8,7',8'-Tetrahydrolycopene; 7',8',11',12'-Tetrahydrolycopene; 1,2,1',2'-Tetrahydrolycopene-1,1'-diol; 1,2,1',2'-Tetrahydroneurosporene; 3,4,11',12'-Tetrahydrospheroidene; 3,4,7,8-Tetrahydrospirilloxanthin; 3,4,3',4'-Tetrahydrospirilloxanthin; 3,4,3',4'-Tetrahydrospirilloxanthin-20-al; 5,6,5',6'-Tetrahydro-3,4,3',4'-tetrol 4, 4'-disulfate; 2,3,2',3'-Tetrahydroxy-beta,beta-carotene-4,4'-dione; 2, 3,2',3'-Tetrahydroxy-beta,beta-caroten-4-one; 3,19,3',17'- Tetrahydroxy-beta,chi-caroten-6'-one 3-sulfate; 3,5,3',5'- Tetrahydroxy-6',7'-didehydro-5,8,5',6'-tetrahydro-beta,beta-caroten-8- one; 3,3',5,5'-Tetrahydroxy-6'-hydro-7-dehydro-beta-carotene; 3,4,3',4'- Tetrahydroxypirardixanthin; 3,4,3',4'-Tetrahydroxy-5,6,5',6'-tetrahydro- beta,beta-carotene; (3,4,3'4')-Tetraketo-beta-carotene; 4,5,4',5'- Tetraketo-beta-carotene; Thiothece-425; Thiothece-460; Thiothece-474; Thiothece-478; Thiothece-484; Thiothece-OH-484; Tilefishxanthin I; Tilefishxanthin II; Tilefishxanthin III; Tilefishxanthin IV; Torularhodin; Torularhodinaldehyde; Torularhodin methyl ester; Torulenal; Torulene; Torulenecarboxylic acid; 2,3,2'-Trihydroxy-beta,beta-caroten-4-one; 3, 3',4'-Trihydroxy-beta,beta-caroten-4-one; 3,4,3'-Trihydroxy-beta, chi-caroten-6'-one; 3,3',5'-Trihydroxy-6',7'-dehydro-alpha-carotene; 3,3',8'-Trihydroxy-7,8-didehydro-beta,chi-carotene-4,6'-dione; 3,3', 8'-Trihydroxy-7,8-didehydro-beta,chi-caroten-6'-one; 3,19,3'- Trihydroxy-7,8-didehydro-beta,chi-caroten-6'-one 3-sulfate; 3,1',2'-Trihydroxy-3',4'-didehydro-1',2'-dihydro-beta,psi-caroten-4-one; 3,5, 19-Trihydroxy-6,7-didehydro-5,6,7',8'-tetrahydro-7'-apo-beta-caroten-8'- one 3-acetate 19-hexanoate; 3,5,6'-Trihydroxy-6,7-didehydro-5,6,7',8'- tetrahydro-beta,[epsi]-carotene-3',8'-dione; 3,5,3'-Trihydroxy-5,6- dihydro-beta-carotene; 3,3',5'-Trihydroxy-5',6'-dihydro-beta-carotene 5',6'-epoxide; 3,19,3'-Trihydroxy-7,8-dihydro-beta,[epsi]-caroten-8-one; 3,19,3'-Trihydroxy-7,8-dihydro-beta,beta-caroten-8-one 19-laurate; 3, 6,3'-Trihydroxy-7,8-dihydro-gamma,[epsi]-caroten-8-one; 3,3',19- Trihydroxy-7,8-dihydro-8-oxo-alpha-carotene; 3,3',6'-Trihydroxy-5,8-epoxy-alpha-carotene; 3,4,4'-Trihydroxypirardixanthin; 1,1',2'- Trihydroxy-3,4,3',4'-tetradehydro-1,2,1',2'-tetrahydro-psi,psi- caroten-2-one; 3,4,4'-Trihydroxy-5,6,5',6'-tetrahydro-beta,beta- carotene; Trikentriorhodin; 3,4,4'-Triketo-beta-carotene; 3,1',2'- Trimethoxy-3',4'-didehydro-1',2' dihydro-beta,psi-caroten-4-one; Triophaxanthin; Triphasiaxanthin; Trisanhydrobacterioruberin; Trollein; Trollichrome; Trolliflavin; Trolliflor; Trollixanthin; Tunaxanthin; Uriolide; Vaucheriaxanthin; Violaxanthin; Violeoxanthin; Violerythrin; Warmingol; Warmingone; Webbiaxanthin; Xanthophyll; Xanthophyll K (1); Xanthophyll K (1)S; Xanthophyll dipalmitate; Xanthophyll epoxide; alpha-Zeacarotene; beta-Zeacarotene; beta (1)- Zeacarotene; alpha-Zeacarotene-3,17'-diol; beta-Zeacarotene-3,17'- diol; beta-Zeacaroten-3-ol; Zeaxanthene; Zeaxanthin; Zeaxanthin diepoxide; Zeaxanthin dimethyl ether; Zeaxanthin dirhamnoside; Zeaxanthin dipalmitate; Zeaxanthin 5,6-epoxide; Zeaxanthin 5,8-epoxide; Zeaxanthin furanoxide; Zeaxanthin monomethyl ether; Zeaxanthin monorhamnoside; Zeaxanthol; and Zeinoxanthin.

The above list of naturally occurring carotenoids is meant to be a non-limiting example of naturally occurring carotenoids. This list is not comprehensive as more naturally occurring molecules are being discovered which will fall within the category of carotenoids.

### Sulfonylureas

Non-limiting examples of sulfonylureas include, but are not limited to acetohexamide, DiaBeta, glibenclamide, gliclazide, glipizide (Glucotrol), glyclopyramide, chlorpropamide, tolazamide, tolbutamide, glimepiride (Amaryl), tolbutamide and meglitinide analogues (for example, repaglinide, nateglinide, meglitinide and mitiglinide (KAD-1229)) and the like.

### Niacin and related derivatives

The term niacin is the generic descriptor for nicotinic acid (pyridine-3-carboxylic acid) and its derivatives. Non-limiting examples of nicotinic acid derivatives include nicofuranose, Acipimox (5-methyl pyrazine-2-carboxylic acid 4-oxide), niceritrol, probucol, isonicotinic acid, Cholexamin, oxiniacic acid, nicoclonate, nicomol, NIASPAN, nicerikol and tocopherol nicotinate.

Further examples of other active agents which may be suitable for this invention include, without limitation: AGI-1067, abecarnil, acamprostate, acavir, acebutolol, aceclofenac, acemetacin, acetaminophen, acetaminosalol, acetanilide, acetohexamide, acetophenazine maleate, acetophenazine, acetoxolone, acetoxypregnenolone, acetretin, acrisorcin, acrivastine, acyclovir, adinazolam, adiphenine hydrochloride, adrafinil, adrenolone, agatroban, ahnitrine, akatinol, alatrofloxacin, albendazole, albuterol, aldioxa, alendronate, alfentanil, alibendol, alitretinoin, allopurinol, allylamines, allylestrenol, alminoprofen, almotriptan, alosetron, aloxiprin, alprazolam, alprenolol, amantadine, ambucetamide, amidephrine, amidinomycin, amiloride, aminoarylcarboxylic acid derivatives, aminoglutethimide, aminoglycosides, aminopentamide, aminopromazine, aminorex, amiodarone, amiphenazole, amiprilose, amisulpride, amitriptyline, amlexanox, amlodipine, amodiaquine, amosulalol, amotriphene, amoxapine, amoxicillin, amphecloral, amphetamine, amphomycin, amphotericin, ampicillin, ampiroxicam, amprenavir, amrinone, amsacrine, amyl nitrate, amylobarbitone, anagestone acetate, anastrozole, andinocillin, androstenediol, androstenediol-17- acetate, androstenediol-17-benzoate, androstenediol-3-acetate, androstenediol-3-acetate-17- benzoate, androstenedione, androsterone acetate, androsterone benzoate, androsterone propionate, androsterone, angiotensin, anidulatungin, aniracetam, apazone, apicycline, apoatropine, apomorphine, apraclonidine, aprepitant, aprotinin, arbaprostil, ardeparin, aripiprazole, arnikacin, arotinolol, arstiinol, arylacetic acid derivatives, arylalkylamines, arylbutyric acid derivatives, arylcarboxylic acids, arylpiperazines, arylpropionic acid derivatives, aspirin, astemizole, atenolol, atomoxetine, atorvastatin, atovaquone, atropine, auranofn, azapropazone, azathioprine, azelastine, azetazolamide, azithromycin, baclofen, bambuterol, bamethan, barbitone, barnidipine, basalazide, beclamide, beclobrate, befimolol, bemegride, benazepril, bencyclane, bendazac, bendazol, bendroflumethiazide, benethamine penicillin, benexate hydrochloride, benfurodil hemisuccinate, benidipine, benorylate, bentazepam, benzhexol, benziodarone, benznidazole, benzoctamine, benzodiazepine derivatives, benzodiazepine, benzonatate, benzphetamine, benzylmorphine, beperiden, bephenium hydroxynaphthoate, bepridil, betahistine, betamethasone, betaxolol, bevantolol, bevonium methyl sulfate, bexarotene, bezadoxifine, bezafibrate, bialamicol, biapenem, bicalutamide, bietamiverine, bifonazole, binedaline, binifibrate, biricodar, bisacodyl, bisantrene, bisoprolol, bitolterol, bopindolol, boswellic acid, bradykinin, bretylium, bromazeparn, bromocriptine, bromperidol, brotizolam, brovincamine, buciclate, bucloxic acid, bucumolol, budralazine, buieniode, bufetolol, buflomedil, bufuralol, bumetanide, bunitrolol, bupranolol, buprenorphine, bupropion, buspirone, busulfan, butalamine, butorphanol, butaverine, butenatme, butidrine hydrochloride, butobarbitone, butoconazole nitrate, butoconazole, butofilol, butropium bromide, cabergoline, calcifediol, calcipotriene, calcitriol, caldibine, cambendazole, camioxirole, camostat, campesterol, camptothecin, candesartan, candoxatril, capecitabine, caprate, capsaicin, captopril, carazolol, carbacephems, carbamates, carbamazepine, carbapenems, carbarsone, Carbatrol, carbenoxolone, carbimazole, carbromal, carbuterol, carisoprodol, carotenes, caroverine, carteolol, carvedilol, cefaclor, cefazolin, cefbuperazone, cefepime, cefoselis, ceftibuten, celecoxib, celiprolol, cephaeline, cephalosporin C, cephalosporins, cephamycins, cerivastatin, certoparin, cetamolol, cetiedil, cetirizine, cetraxate, chloracizine, chlorambucil, chlorbetamide, chlordantoin, chlordiazepoxide, chlormadinone acetate, chlormethiazole, chloroquine, chlorothiazide, chlorpheniramine, chlorphenoxamide, chlorphentermine, chlorproguanil, chlorpromazine, chlorpropamide, chlorprothixene, chlortetracycline, chlorthalidone, cholecalciferol, chromonar, ciclesonide, ciclonicate, cidofovir, ciglitazone, cilansetron, cilostazol, cimetidine, cimetropium bromide, cinepazet maleate, cinnamedrine, cinnarizine, cinolazepam, cinoxacin, ciprofibrate, ciprofloxacin, cisapride, cisplatin, citalopram, citicoline, clarithromycin, clebopride, clemastine, clenbuterol, clidanac, clinofibrate, clioquinol, clobazam, clobenfurol, clobenzorex, clofazimine, clofibrate, clofibric acid, cloforex, clomipramine, clonazepam, clonidine, clonitrate, clopidogrel, clopirac indomethacin, cloranolol, cloricromen, clorprenaline, clortermine, clotiazepam, clotrimazole, cloxacillin, clozapine, cmepazide, codeine methyl bromide, codeine phosphate, codeine sulfate, codeine, colloidal bismuth subcitrate, cromafiban, cromolyn, cropropamide, crotethamide, curcumin, cyclandelate, cyclarbamate, cyclazocine, cyclexedrine, cyclizine, cyclobenzaprine, cyclodrine, cyclonium iodide, cyclopentamine, cyclosporin, cypionate, cyproheptadine, cyproterone acetate, cytarabine, dacarbazine, dalfopristine, dantrolene sodium, dapiprazole, darodipine, decanoate, decitabine, decoquinate, dehydroemetine, delavirdine, delaviridine, demeclo cycline, denopamine, deramciclone, descitalopram, desipramine, desloratadine, 3- ketodesogeskel, desomorphine, desoxymethasone, detomidine, dexamphetamine, dexanabinol, dexchlorpheniramine, dexfenfluramine, dexmethylphenidate, dexrazoxane, dextroamphetamine sulfate, dextroamphetamine, dextropropoxyphene, DHEA, diacetate, diamorphine, diazemine, diazepam, diaziquinone, diazoxide, dibromopropamidine, dichlorophen, diclofenac, dicoumarol, didanosine, dideoxyadenosine, diethylpropion, difemerine, difenamizole, diflunisal, digitoxin, digoxin, dihydroergotamine, dihydrocodeine, dihydrocodeinone enol acetate, dihydroergotamine mesylate, dihydroergotamine, dihydrogesterone, dihydromorphine, dihydropyridine derivatives, dihydrostreptomycin, dihydrotachysterol, dihydroxyaluminum acetylsalicylate, diiodohydroxyquinoline, diisopromine, dilazep, dilevalol, diltiazem, diloxanide furoate, diloxanide, diltiazem, dimefline, dimenhydrinate, dimethisterone, dimetotrine, dimorpholamine, dinitolmide, dioxaphetyl butyrate, dioxethedrine, diphemethoxidine, diphenhydramine, diphenoxylate, diphetarsone, dipivefrin, diponium bromide, dipyridamole, dirithromycin, disopyramide, divalproex sodium, dofetilide, domperidone, donepezil, dopexamine, dopradil, dosmalfate, doxapram, doxazosin, doxefazepam, doxepin, doxycycline, drofenine, dromostanolone propionate, dromostanolone, dronabinol, droperidol , droprenilamine, d-threo - methylphenidate, duloxetine, ebrotidine, eburnamonine, ecabet, ecenofloxacin, econazole nitrate, edavarone, edoxudine, efavirenz, effivarenz, efloxate, eledoisin, eletriptan, elgodipine, ellipticine, emepronium bromide, emetine, enalapril, enanthate, encainide, enlopitat, enoximone, enprostil, entacapone, epanolol, ephedrine, epinastine, epinephrine, epirubicin, eplerenone, eprosartan, ergocalciferol, ergoloid mesylates, ergotamine, ertapenum, erythromycin, erytlirityl tetranitrate, esaprazole, escitalopram, esmolol, esomeprazole, esonarimod, estazolam, estradiol benzoate, estramustine, eskiol succinate, estrone acetate, estrone sulfate, etafedrine, etafenone, ethacrynic acid, ethamivan, ethinamate, ethinyleskadiol 3-acetate, ethinyleskadiol 3-benzoate, ethinylestradiol, ethionamide, ethisterone (17a-ethinyltestosterone), ethopropazine, ethotoin, ethoxyphenamine, ethylestrenol, ethylmorphine, ethylnorepinephrine, ethynodiol diacetate, etodolac, etofibrate, etoposide, etoricoxib, etretinate, everolimus, exalamide, examestane, examorelin, ezemitibe, falecalcitriol, famciclovir, famotidine, fantofarone, farapenum, farglitazar, fasudil, felbamate, felodipine, fenalamide, fenbuLen, fenbutrazate, fendiline, fenfluramine, fenoldopam, fenoprofen, fenoterol, fenoverine, fenoxazoline, fenoxedil, fenpiprane, fenproporex, fenspiride, fentanyl, fexofenadine, flavoxate, flecainide, flopropione, floredil, floxuridine, fluconazole, flucytosine, fludarabine, fludiazepam, fludrocortisone, flulenamic acid, flunanisone, flunarizine, flunisolide, flunitrazepam, fluocortolone, fluoxetine, flupenthixol decanoate, fluphenazine decanoate, fluphenazine enanthate, fluphenazine, fluproquazone, flurazepam, flurbiprofen, flurogestone acetate, fluticasone propionate, fluvastatin, fluvoxamine, fominoben, formoterol, foscarnet, foscarnet, fosinopril, fosphenytoin, frovatriptan, fudosteine, fumagillin, furazolidone, furazolidone, furfurylmethyl amphetamine, furosemide, gabapentin, gabexate, gaboxadol, galanthamine, gallopamil, gammaparin, ganciclovir, ganglefene, gefarnate, gemcitabine, gemfibrozil, gepirone, gestadene, ghrelin, glatiramer, glaucarubin, glibenclamide, gliclazide, glimepiride, glipizide, gluconic acid, glutamic acid, glyburide, glyceryl trinitrate, glimepiride, granisetron, grepafloxacin, griseofulvin, guaiazulene, guanabenz, guanfacine, halofankine, haloperidol decanoate, haloperidol, haloxazolam, hepronicate, heptanoate, hexobendine, hexoprenaline, hydramitrazine, hydrazides, hydrocodone, hydrocortisone, hydromorphone, hydroxyamphetamine, hydroxymethylprogesterone acetate, hydroxymethylprogesterone, hydroxyprogesterone acetate, hydroxyprogesterone caproate, hydroxyprogesterone, hymecromone, hyoscyamine, ibopamine, ibudilast, ibutenac, ibuprofen, ibutilide, idoxuridine, ifenprodil, igmesine, iloprost, imatinib, imidapril, imidazoles, imipenem, imipramine, imolamine, incadronic acid pergolide, indanazoline, indenolol, indinavir, indomethacin, indoramin, inosine pranobex, inositol niacinate, iodoquinol, ipidracine, iproniazid, irbesartan, irinotecan, irsogladine, isobutyrate, isocaprate esters, isoetharine, isometheptene, isoproterenol, isosorbide dinitrate, isosorbide mononitrate, isosorbide dinitrate, isoxsuprine, isradipine, itasetron, itraconazole, itramintosylate, ivermectin, kallidin, kallikrein, kanamycin, ketamine, ketoconazole, ketoprofen, ketorolac, ketotifen, labetalol, lafutidine, lamifiban, lamivudine, lamotrigine, lanatoside c, lansoprazole, lasofoxifene, leflunomide, leminoprazole, lercanidipine, lesopitron, letrozole, leucovorin, levalbuterol, levallorphan, levetiracetam, levetriacetam, levobunolol, levodopa, levofloxacin, levophacetoperane, levorphanol, lidocaine, lidoflazine, lifibrol, limaprost, linezolid, lintitript, liranaftate, lisinopril, lisuride, lobeline, lobucavir, lodoxamide, lomefloxacin, lomerizine, lomustine, loperamide, lopinavir, loprazolam, loracarbef, loratadine, lorazepam, lorefloxacin, lormetazepam, losartan, lovastatin, lovastatin, loxapine succinate, loxapine, 1-threo methylphenidate, lumiracoxib, lysine acetylsalicylate, lysozyme, lisuride, mabuterol, mafenide, magnesium acetylsalicylate, malgramostin, mannitol hexanitrate, maprotiline, mazindol, mebendazole, meclizine, meclofenamic acid, mecloxaminepentapiperide, medazepam, medibazine, medigoxin, medrogestone, medroxyprogesterone acetate, mefenamic acid, mefenorex, mefloquin, mefloquine, megestrol acetate, melengestrol acetate, melphalan, memantine, mepenzolate bromide, meperidine, mephenoxalone, mephentermine, mepindolol, mepixanox, meprobamate, meptazinol, mercaptopurine, meropenum, mesalamine, mesalazine, mesoridazine besylate, mesoridazine, metaclazepam, metamfepramone, metampicillin, metaproterenol, metaraminol, methacycline, methadone hydrochloride, methadone, methamphetamine, methaqualone, metharnphetamine, methoin, methotrexate, methoxamine, methsuximide, methylhexaneamine, methylphenidate d-threo-methylphenidate, methylphenidate, methylphenobarbitone, methylprednisolone, methysergide, metiazinic acid, metizoline, metoclopramide, metolazone, metoprolol, metoxalone, metripranolol, metronidazole, mexiletine, metaxalone, mianserin, mibefradil, miconazole, midazolam, midodrine, miglitol, milnacipran, milrinone, minoxidil, mirtazapine, misoprostol, mitomycin, mitotane, mitoxantrone, mizolastine, modafinil, mofebutazone, mofetil, molindone hydrochloride, molindone, molsidomine, monatepil, montelukast, Monteplase, moprolol, moricizine, morphine hydrochloride, morphine sulfate, morphine, morpholine salicylate, mosapramine, moxifloxacin, moxisylyte, moxonidine, mycophenolate, nabumetone, nadolol, nadoxolol, nadroparin, nafamostat, nafronyl, naftopidil, nalbuphine, nalidixic acid, nalmefene, nalorphine, naloxone, naltrexone, nandrolone benzoate, nandrolone cyclohexanecarboxylate, nandrolone cyclohexane-propionate, nandrolone decanoate, nandrolone furylpropionate, nandrolone phenpropionate, naphazoline, naproxen, naratriptan, natamycin, nateglinide, nedocromil, nefazodone, nefopam, nelfinavir, nemonapride, neomycin undecylenate, neomycin, neokofin, nesiritide, n- ethylamphetamine, nevirapine, nexopamil, nicametate, nicardipine, nicergoline, nicofibrate, nicofuranose, nicomorphine, nicorandil, nicotinyl alcohol, nicoumalone, nifedipine, nifenalol, nikethamide, nilutamide, nilvadipine, nimodipine, nimorazole, nipradilol, nisoldipine, nitisonone, nitrazepam, nitrofurantoin, nitrofurazone, nitroglycerin, nizatidine, norastemizole, norepinephrine, norethynodrel, norfenefrine, norfloxacin, norgestimate, norgeskel, norgestrienone, normethadone, normethisterone, normorphine, norpseudoephedrine, nortriptyline, novantrone, nylidrin, nystatin, octamylamine, octodrine, octopamine, ofloxacin, olanzapine, olanzapine, olapatadine, olmesartan, Olapatadine, olsalazine, omapatrilat, omeprazole, ondansetron, opium, oprevelkin, orlistat, ornidazole, ornoprostil, oseltamivir, oxaliplatin, oxamniquine, oxandrolone, oxantel embonate, oxaprozin, oxatomide pemirolast, oxatomide, oxazepam, oxcarbazepine, oxfendazole, oxiconazole, oxiracetam, oxolinic acid, oxprenolol, oxycodone, oxyfedrine, oxymetazoline, oxymorphone, oxyphenbutazone, oxyphencyclimine, ozagrel, paclitaxel, palonosetron, pantoprazole, papaverine, paricalcitol, paramethadione, parecoxib, pariprazole, paromomycin, paroxetine, parsalmide, pazinaclone, pemoline, penbutolol, penciclovir, penicillin G benzathine, penicillin G procaine, penicillin V, penicillins, pentaerythritol tetranitrate, pentaerythritol tetranitrate, pentapiperide, pentazocine, pentifylline, pentigetide, pentobarbitone, pentorex, pentoxifylline, pentrinitrol, pirbuterol, pirenzepine, pergolide, perhexiline, perindopril erbumine, perospone, perphenazine pimozide, perphenazine, phanquinone, phenacemide, phenacetin, phenazopyridine, phencarbamide, phendimetrazine, phenelzine, phenindione, phenmetrazine, phenobarbitone, phenoperidine, phenothiazines, phenoxybenzamine, phensuximide, phentermine, phentolamine, phenylsalicylate, phenylacetate, phenylbutazone, phenylephrine hydrochloride, phenylpropanolamine hydrochloride, phenylpropanolamine hydrochloride, phenylpropyl-methylamine, phenytoin, phloroglucinol, pholedrine, physostigmine salicylate, physostigmine, phytonadiol, phytosterols, piapenum, picilorex, piclamilast, picrotoxin, picumast, pifarnine, pilsicainide, pimagedine, pimeclone, pimecrolimus, pimefylline, pimozide, pinaverium bromide, pindolol, pioglitazone, piperacillin, piperazine estrone sulfate, piperazine derivatives, piperilate, piracetam, piribedil, pirifibrate, piroxicam, pitavastatin, pizotyline, plaunotol, polaprezinc, polybenzarsol, polyestrol phosphate, practolol, pralnacasan, pramipexole, pranlukast, pravastatin, prazepam, praziquantel, prazosin, pregabalin, prenalterol, prenylamine, pridinol, prifinium bromide, primidone, primipramine, probenecid, probucol, procainamide, procarbazine, procaterol, prochlorperazine, proguanil, pronethalol, propafenone, propamidine, propatyl nitrate, propentoffyline, propionate, propiram, propoxyphene, propranolol, propylhexedrine, propylthiouracil, protokylol, protriptyline, proxazole, pseudoephedrine, purines, pyrantel embonate, pyrazoles, pyrazolones, pyridofylline, pyrimethamine, pyrimidines, pyrrolidones, quazepam, quetiapine, quetuapine, quinagolide, quinapril, quinestrol, quinfamide, quinidine, quinine sulfate, quinolones, quinupritin, rabalzotan, rabeprazole sodium, rabeprazole, racefimine, ramahroban, ramipril, ranitidine, ranolazine, ransoprazole, rasagiline, rebamipide, refludan, repaglinide, repinotan, repirinast, reproterol, reserpine, retinoids, ribavirin, rifabutine, rifampicin, rifapentine, rilmenidine, riluzole, rimantadine, rimiterol, rioprostil, risperidone, ritanovir, ritapentine, ritipenem, ritodrine, ritonavir, rivastigrnine, rizatriptan, rociverine, rofecoxib, rohypnol, rolipram, remoxipride, ronifibrate, ropinirole, ropivacaine, rosaprostol, rosiglitazone, rosuvastatin, rotinolol, rotraxate, roxatidine acetate, roxindole, rubitecan, salacetamide, salicin, salicylamide, salicylic acid derivatives, salmeterol, saquinavir, saquinavir, scopolamine, secnidazole, selegiline, semotiadil, sertindole, sertraline, sibutramine, sildenafil, simvastatin, siramesine, sirolimus, sitaxsentan, sofalcone, somotiadil, sorivudine, sotalol, soterenol, sparfloxacin, spasmolytol, spectinomycin, spiramycin, spizofurone, stavudine, streptomycin, succinylsulfathiazole, sucralfate, sufentanil, sulconazole nitrate, sulfacetamide, sulfadiazine, sulfaloxicacid, sulfarside, sulfinalol, sulindac, suloctidil, sulphabenzamide, sulphacetamide, sulphadiazine, sulphadoxine, sulphafurazole, sulphamerazine, sulphamethoxazole, sulphapyridine, sulphasalazine, sulphinpyrazone, sulpiride, sulthiame, sultopride, sulbroponium, sumanirole, sumahriptan, sunepihon, superoxide dismutase, suplatast, suramin sodium, synephrine, tacrine, tacrolimus, tacrolimus, tadalafil, talinolol, talipexole, tamoxifen, tamsulosin, targretin, tazanolast, tazarotene, tazobactam, tecastimezole, teclozan, tedisamil, tegaserod, telenzepine, telmisartan, temazepam, teniposide, teprenone, terazosin, terbinafine, terbinafine, terbutaline sulfate, terbutaline, terconazole, terfenadine, terodiline, terofenamate, tertatolol, testolactone, 4-dihydrotestosterone, tetracyclics, tetracycline, tetrahydrocannabinol, tetrahydrozoline, thalidomide, theofibrate, thiabendazole, thiazinecarboxamides, thiocarbamates, thiocarbamizine, thiocarbarsone, thioridazine, thiothixene, tiagabine, tiamenidine, tianeptine, tiaprofenic acid, tiaramide, ticlopidine, tigloidine, tilisolol, timolol, tinidazole, tinofedrine, tinzaparin, tioconazole, tipranavir, tirapazamine, tirofiban, tiropramide, titanicene, tizanadine, tizanidine, tizinadine, tocainide, tolazamide, tolazoline, tolbutamide, tolcapone, tolciclate, tolfenamic acid, toliprolol, tolteridine, tolterodine, tonaberstat, topiramate, topotecan, torsemide, toremifene cibrate, toremifene, tosufloxacin, tramadol, tramazoline, trandolapril, tranilast, tranylcypromine, trapidil, traxanox, trazodone, tretoquinol, triacetin, triamcinolone, triamterine, triamterene, triazolam, trifluoperazine hydrochloride, trifluoperazine, triflupromazine, trihexyphenidyl, trimazosin, trimebutine, trimetazidine, trimethoprim, trimgestone, trimipramine, trimoprostil, trithiozine, troglitazone, trolnibrate phosphate, tromethamine, tropicamide, trovafloxacin, troxipide, tuaminoheptane, tulobuterol, tymazoline, tyramine, undecanoate, undecenoic acid, urinastatin, valacyclovir, valdecoxib, valerate, valganciclovir, valproic acid, valsartan, vancomycin, vardenafil, venlafaxine, venorelbine, verapamil, vidarabine, vigabakin, vincamine, vinpocetine, viomycin, viquidil, visnadine, vitamin a derivatives, vitamin a, vitamin b₂, vitamin d, vitamin e, vitamin k, voglibose, voriconazole, xaliproden, xamoterol, xanthinol niacinate, xenytropium bromide, xibenolol, ximelagatran, xylometazoline, yohimbine, zacopride, zafirlukast, zalcitabine, zaleplon, zanamivir, zatebradine, ziconotide, zidovudine, zileuton, zimeldine, zinc propionate, ziprasidone, zolimidine, zolmitriptan, zolpidem, zonisamide, zopiclone, and mixtures thereof.

The compounds according to the invention may be formulated as pharmaceutical compositions suitable for oral, buccal, parenteral, transdermal or rectal administration or in a form suitable for administration by inhalation or insufflation. Parenteral administration includes subcutaneous injections, intravenous, intramuscular, intrasternal injection, or infusion techniques.

The pharmaceutical compositions may also be formulated as a therapeutically effective amount in a dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof. Such dosage forms can also comprise, as in conventional practice, additional substances other than inert diluents, e.g., lubricating agents such as magnesium stearate. In the case of capsules, tablets, effervescent tablets, and pills, the dosage forms can also comprise buffering agents. Soft gelatin capsules can be prepared to contain a mixture of the active compounds or compositions of the invention and vegetable oil. Hard gelatin capsules can contain granules of the active compound in combination with a solid, pulverulent carrier such as lactose, saccharose, sorbitol, mannitol, potato starch, corn starch, amylopectin, cellulose derivatives of gelatin. Tablets and pills can be prepared with enteric coatings.

Liquid dosage forms for oral administration can include pharmaceutically acceptable emulsions, solutions, suspensions, syrups, and elixirs containing inert diluents commonly used in the art, such as water. Such compositions can also comprise adjuvants, such as wetting agents, emulsifying and suspending agents, and sweetening, flavoring, and perfuming agents.

Suppositories for vaginal or rectal administration of the compounds and compositions of the invention can be prepared by mixing the compounds or compositions with a suitable nonirritating excipient such as cocoa butter and polyethylene glycols which are solid at room temperature but liquid at body temperature, such that they will melt and release the drug.

Injectable preparations, for example, sterile injectable aqueous or oleaginous suspensions can be formulated according to the known art using suitable dispersing agents, wetting agents and/or suspending agents. The sterile injectable preparation can also be a sterile injectable solution or suspension in a nontoxic parenterally acceptable diluent or solvent, for example, as a solution in 1,3-butanediol. Among the acceptable vehicles and solvents that can be used are water, Ringer's solution, and isotonic sodium chloride solution. Sterile fixed oils are also conventionally used as a solvent or suspending medium.

The compositions of this invention can further include conventional excipients, i.e., pharmaceutically acceptable organic or inorganic carrier substances suitable for oral, buccal, parenteral, transdermal or rectal administration or in a form suitable for administration by inhalation or insufflation, which do not deleteriously react with the active compounds. Suitable pharmaceutically acceptable carriers include, for example, water, salt solutions, alcohol, vegetable oils, polyethylene glycols, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like. The pharmaceutical preparations can be sterilized and if desired, mixed with auxiliary agents, e.g., lubricants, preservatives, stabilizers, wetting agents, emulsifiers, salts for influencing osmotic pressure, buffers, colorings, flavoring and/or aromatic substances and the like which do not deleteriously react with the active compounds. For parenteral application, particularly suitable vehicles consist of solutions, preferably oily or aqueous solutions, as well as suspensions, emulsions, or implants. Aqueous suspensions may contain substances that increase the viscosity of the suspension and include, for example, sodium carboxymethyl cellulose, sorbitol and/or dextran. Optionally, the suspension may also contain stabilizers.

Both tablets and capsules may also be manufactured in the form of sustained release formulations, such that they provide a controlled continuous release of the compounds according to the invention over a period of hours.

Administration of the compositions of the present invention will be in an amount sufficient to achieve a therapeutic effect as recognized by one of ordinary skill in the art.

The dosage of any compositions of the present invention will vary depending on the symptoms, age and body weight of the patient, the nature and severity of the disorder to be treated or prevented, the route of administration, and the form of the subject composition. Any of the subject formulations may be administered in a single dose or in divided doses. Dosages for the compositions of the present invention may be readily determined by techniques known to those of skill in the art or as taught herein.

The dosage range for nebivolol ranges from about 0.1 mg to about 100 mg per day. In another embodiment, the dosage range may be from about 0.75 mg to about 50 mg per day. In yet another embodiment, the dosage range may be from about 1.25 mg to about 10 mg per day.

In certain embodiments, the dosage of the co-active compounds will generally be in the range of about 0.01 ng to about 1 g per kg body weight, specifically in the range of about 1 ng to about 0.1 g per kg, and more specifically in the range of about 100 ng to about 10 mg per kg body weight.

An effective dose or amount, and any possible affects on the timing of administration of the formulation, may need to be identified for any particular composition of the present invention. This may be accomplished by routine experiment as described herein, using one or more groups of animals (preferably at least 5 animals per group), or in human trials if appropriate. The effectiveness of any subject composition and method of treatment or prevention may be assessed by administering the composition and assessing the effect of the administration by measuring one or more applicable indices, and comparing the post-treatment values of these indices to the values of the same indices prior to treatment.

The precise time of administration and amount of any particular subject composition that will yield the most effective treatment in a given patient will depend upon the activity, pharmacokinetics, and bioavailability of a subject composition, physiological condition of the patient (including age, sex, disease type and stage, general physical condition, responsiveness to a given dosage and type of medication), route of administration, and the like. The guidelines presented herein may be used to optimize the treatment, e.g., determining the optimum time and/or amount of administration, which will require no more than routine experimentation consisting of monitoring the subject and adjusting the dosage and/or timing.

While the subject is being treated, the health of the patient may be monitored by measuring one or more of the relevant indices at predetermined times during the treatment period. Treatment, including composition, amounts, times of administration and formulation, may be optimized according to the results of such monitoring. The patient may be periodically reevaluated to determine the extent of improvement by measuring the same parameters. Adjustments to the amount(s) of subject composition administered and possibly to the time of administration may be made based on these reevaluations.

Treatment may be initiated with smaller dosages which are less than the optimum dose of the compound. Thereafter, the dosage may be increased by small increments until the optimum therapeutic effect is attained.

The use of the subject compositions may reduce the required dosage for any individual agent contained in the compositions (e.g., the steroidal anti inflammatory drug) because the onset and duration of effect of the different agents may be complimentary.

Toxicity and therapeutic efficacy of subject compositions may be determined by standard pharmaceutical procedures in cell cultures or experimental animals, e.g., for determining the LD₅₀ and the ED₅₀.

The data obtained from the cell culture assays and animal studies may be used in formulating a range of dosage for use in humans. The dosage of any subject composition lies preferably within a range of circulating concentrations that include the ED₅₀ with little or no toxicity. The dosage may vary within this range depending upon the dosage form employed and the route of administration utilized. For compositions of the present invention, the therapeutically effective dose may be estimated initially from cell culture assays.

In general, the doses of an active agent will be chosen by a physician based on the age, physical condition, weight and other factors known in the medical arts.

Those of skill in treating subjects suffering from a vascular disease, for instance, an increased blood pressure, could easily determine the therapeutically effective amount from the test results presented hereinafter. In general it is contemplated that an effective daily dose of the compounds of formula (I) or their pharmaceutically acceptable acid-addition salts would be from about 0.01 mg/kg to about 50 mg/kg body weight, in particular from about 0.1 mg/kg to about 10 mg/kg body weight, and preferably from about 0.1 mg/kg to about 1 mg/kg body weight.

Pharmaceutical kits are also provided comprising one or more containers filled with one or more of the ingredients of the pharmaceutical compounds and/or compositions of the invention, including, glucuronidated nebivolol metabolites. Such kits can also include, for example, other compounds and/or compositions (e.g., diuretics, digoxin, compounds used to treat cardiovascular diseases, therapeutic agents, permeation enhancers, lubricants, and the like), a device(s) for administering the compounds and/or compositions, and written instructions in a form prescribed by a governmental agency regulating the manufacture, use or sale of pharmaceuticals or biological products, which instructions can also reflects approval by the agency of manufacture, use or sale for human administration.

In another aspect, the present disclosure relates to the local administration of at least one glucuronidated metabolite of nebivolol, and, optionally, at least one additional therapeutic agent to treat injured tissue, such as damaged blood vessels.

The compounds of the present disclosure and, optionally, other therapeutic agents, can be incorporated into a natural or synthetic matrix which can then be applied with specificity to a biological site of interest. Accordingly the nebivolol metabolites are "bound to the matrix" which means that the nebivolol metabolite(s) along with other therapeutic agents are physically and/or chemically associated with part of, incorporated with, attached to, or contained within the natural or synthetic matrix.

Any of a wide variety of natural or synthetic polymers can be used as the matrix in the context of the present disclosure. It is only necessary for the matrix to be biologically acceptable. Exemplary matrixes are polymers including, for example, polyolefins (such as polystyrene, polypropylene, polyethylene, high density polyethylene, polytetrafluorethylene, polyvinylidene diflouride and polyvinylchloride), polyethylenimine or derivatives thereof, polyethers (such as polyethylene glycol), polyesters (such as poly-L-lactic acid, poly-D, L-lactic, poly-D-lactic, polyglycolic, poly-(lactide/glycolide)), polyanhydrides, polyhydroxybutyrates, polyamides (such as nylon), polyurethanes, polyurethane copolymers (such as pellethane polymers), polyacrylates (such as polymethacrylate, poly (2-(methacryloyloxyethyl)-2'-(trimethylammonium)ethyl phosphate inner salt-con-dodecyl methacrylate), mixtures of polymers (such as polylactic acid/polylysine copolymers, polyurethane/polyester copolymers, polyurethane/polyether copolymers, nylon/polyether copolymers, such as vestamid), biopolymers (such as peptides, proteins, oligonucleotides, antibodies, peptide hormones, glycoproteins, glycogen and nucleic acids), starburst dendrimers, natural fibrous matrix (such as filter paper), synthetic fibrous matrix materials (such as three-dimensional lattice of synthetic polymers and copolymers) and the like. Exemplary polymers are described in U.S. Pat. Nos. 5,705,583, 5,770,645 and 5,994,444.

The physical and structural characteristics of the matrixes are not critical, but depend on the application. It will be appreciated by one skilled in the art that where the matrix-metabolite of nebivolol composition is intended for local, relatively short term administration or similar administration they need not be biodegradable. For some uses, such as postangioplasty, coronary bypass surgery or intimal hyperplasia associated with vascular graft implants or the like, it may be desirable for the matrix to slowly dissolve in a physiological environment or to be biodegradable or bioresorbable.

In preventing and/or treating cardiovascular diseases or disorders, the nebivolol metabolites of the present disclosure and optionally at least one therapeutic agent can be administered directly to the damaged vascular surface intravenously by using an intraarterial or intravenous catheter, suitable for delivery of the compounds to the desired location. The location of damaged arterial surfaces is determined by conventional diagnostic methods, such as X-ray angiography, performed using routine and well-known methods available to one skilled in the art.

When administered *in vivo*, the compounds and compositions of the disclosure can be administered in combination with pharmaceutically acceptable carriers and in a therapeutically effective dosage, as described herein.

When the compounds and compositions of the present disclosure are administered as a mixture of at least one glucuronidated nebivolol metabolite, they can also be used in combination with one or more additional therapeutic agents which are known to be effective against the specific disease state targeted for treatment, such as for example diuretics and/or different antihypertensive agents such as beta-blockers, calcium channel blockers or ACE inhibitors. It is to be understood that such combination therapy constitutes a further aspect of the present disclosure.

"Concurrent administration" encompasses simultaneous or sequential treatment with the components of the combination, as well as regimens in which the drugs are alternated, or wherein one component is administered long-term and the other(s) are administered intermittently. Components can be administered in the same or in separate compositions, and by the same or different routes of administration.

The nebivolol metabolites of the present disclosure may be used in combination with other drugs that are used in the treatment/prevention/suppression or amelioration of the diseases or conditions for which the glucuronidated nebivolol metabolites are useful. Other drugs include, for example, an antithrombogenic agent, a thrombolytic agent, a fibrinolytic agent, a vasospasm inhibitor, a potassium channel activator, a calcium channel blocker, an antihypertensive agent, an antimicrobial agent, an antibiotic, an antiplatelet agent, an antimitotic agent, an antiproliferative agent, a microtubule inhibitor, an antisecretory agent, a remodelling inhibitor, an antisense nucleotide, an anti-cancer chemotherapeutic agent, a steroid, a non-steroidal antiinflammatory agent, a selective COX-2 inhibitor, an immunosuppressive agent, a growth factor antagonist or antibody, a dopamine agonist, a radiotherapeutic agent, a biologic agent, an angiotensin converting enzyme inhibitor, an angiotensin II receptor antagonist, a renin inhbitior, a free radical scavenger, an iron chelator, an antioxidant, a sex hormone, an antipolymerase, an antiviral agent, a photodynamic therapy agent, an antibody targeted therapy agent, a gene therapy agent, biphosphonates or other osteoporosis agents, cholesterol lowering agents, including but not limited to statins, niacin, or gemfibrozil, or mixtures thereof.

Such other drugs may be administered, by a route and in an amount commonly used therefore, contemporaneously or sequentially with a compound of the glucuronidated nebivolol metabolites. When a nebivolol metabolite of the present disclosure is used contemporaneously with one or more other drugs, a pharmaceutical composition containing such other drugs in addition to the compound of the present disclosure is preferred. Accordingly, the pharmaceutical compositions of the present disclosure include those that also contain one or more other active ingredients, in addition to the nebivolol metabolite of the present disclosure. Examples of other active ingredients that may be combined with a nebivolol metabolite of the present disclosure, either administered separately or in the same pharmaceutical compositions, include, but are not limited to: (1) morphine and other opiate receptor agonists including propoxyphene (Darvon); (2) non-steroidal anti-inflammatory drugs (NSAIDs) including COX-2 inhibitors such as propionic acid derivatives (alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid, and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (flufenamic acid, meclofenamic acid, mefenamic acid, niflumic acid and tolfenamic acid), biphenylcarboxylic acid derivatives (diflunisal and flufenisal), oxicams (isoxicam, piroxicam, sudoxicam and tenoxican), salicylates (acetyl salicylic acid, sulfasalazine) and the pyrazolones (apazone, bezpiperylon, feprazone, mofebutazone, oxyphenbutazone, phenylbutazone), and the coxibs (celecoxib, valecoxib, rofecoxib and etoricoxib); (3) corticosteroids such as betamethasone, budesonide, cortisone, dexamethasone, hydrocortisone, methylprednisolone, prednisolone, prednisone and triamcinolone; (4) histamine H1 receptor antagonists such as bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine pyrilamine, astemizole, terfenadine, loratadine, cetirizine, desloratadine, fexofenadine and levocetirizine; (5) histamine H2 receptor antagonists such as cimetidine, famotidine and ranitidine; (6) proton pump inhibitors such as omeprazole, lansoprazole, pantoprazole and esomeprazole; (7) leukotriene antagonists and 5-lipoxygenase inhibitors such as zafirlukast, montelukast, pranlukast and zileuton; (8) drugs used for angina, myocardial ischemia including nitrates such as nitroglycerin and isosorbide nitrates, beta blockers such as atenolol, metoprolol, propranolol, acebutolol, betaxolol, bisoprolol, carteolol, labetalol, nadolol, oxprenolol, penbutolol, pindolol, sotalol and timolol, and calcium channel blockers such as diltiazam, verapamil, nifedipine, bepridil, felodipine, flunarizine, isradipine, nicardipine and nimodipine; (9) incontinence medications such as antimuscarinics, (e.g., tolterodine and oxybutinin); (10) gastrointestinal antispasmodics (such as atropine, scopolamine, dicyclomine, antimuscarinics, as well as diphenoxylate); skeletal muscle relaxants (cyclobenzaprine, carisoprodol, chlorphenesin, chlorzoxazone, metaxalone, methocarbamol, baclofen, dantrolene, diazepam, or orphenadrine); (11) gout medications such as allopurinol, probenicid and colchicine; (12) drugs for rheumatoid arthritis such as methotrexate, auranofin, aurothioglucose and gold sodium thiomalate; (13) drugs for osteoporosis such as alendronate and raloxifene; decongestants such as pseudoephedrine and phenylpropanolamine; (14) local anesthetics; (15) anti-herpes drugs such as acyclovir, valacyclovir and famcyclovir; (16) anti-emetics such as ondansetron and granisetron; (17) advanced glycosylation end-product crosslink breakers (AGE crosslink breakers), for example, alagebrium; and (18) advanced glycosylation end-product formation inhibitors (AGE formation inhibitors), for example, pimagebine.

The term "nitric oxide" encompasses uncharged nitric oxide (NO) and charged nitrogen monoxide species, preferably charged nitrogen monoxide species, such as nitrosonium ion (NO⁺) and nitroxyl ion (NO⁻). The nitrogen monoxide releasing, delivering or transferring compounds have the structure F--NO, wherein F is a nitrogen monoxide releasing, delivering or transferring moiety, and include any and all such compounds which provide nitrogen monoxide to its intended site of action in a form active for its intended purpose.

### Administration of a nebivolol metabolite with a blood pressure reducing agent

The nebivolol metabolites of the present disclosure and the acid addition salts thereof may be administered before, during or after the administration of a blood pressure reducing agent provided that the time of the administration of the compounds of the present disclosure in relation to the administration of the blood pressure reducing agent allows the compound of the present disclosure to be effective in potentiating the effects of the blood pressure reducing agent.

As blood pressure reducing agents of which the activity is potentiated there may be mentioned agents having adrenergic and/or vasodilating activity. In particular such agents may be the compounds mentioned in U.S. Pat. Nos. 3,663,607 and 3,836,671, in particular atenolol; U.S. Pat. Nos. 3,337,628 and 3,520,919, in particular propranolol; U.S. Pat. No. 3,873, 600, in particular metoprolol; U.S. Pat. No. 3,511,836, in particular prazosin; U.S. Pat. No. 2,484,029, in particular hydralazine; U.S. Pat. No. 2,928,829 in particular guanethidine; U.S. Pat. No. 2,503,059, in particular phentolamine; U.S. Pat. No. 3,261,859, in particular verapamil; U.S. Pat. No. 3,485,847 in particular nifedipine; U.S. Pat. No. 3,910, 924, in particular carteolol; German Pat. Nos. 2,458,624 and 2,458,625, in particular celiprolol.

Preferably a nebivolol metabolite of the present disclosure and the blood pressure reducing agent are administered in the form of suitable compositions. Said compositions are meant to also comprise products containing a nebivolol metabolite as defined herein and a blood-pressure reducing agent as a combined preparation for simultaneous, separate, timed-release of either or both components, or sequential use in blood-pressure reducing therapy. Such products may, for example, comprise a kit comprising a container with a suitable composition containing a nebivolol metabolite of the present disclosure and another container containing a composition with a blood pressure reducing agent. Such a product may have the advantage that the physician wishing to administer blood pressure reducing therapy can select, based on the diagnosis of the patient to be treated, the appropriate amounts of both components and the sequence of administration.

When administered during the administration of the blood pressure reducing agent, a composition containing both the blood pressure reducing agent and the active ingredient of a nebivolol metabolite of the present disclosure may particularly be convenient.

In a further aspect of the present disclosure there may be provided a composition comprising an amount capable of potentiating the effects of blood pressure reducing agents of a nebivolol metabolite of the present disclosure as defined herein and a blood pressure reducing agent. In the said composition, the molar ratio between the nebivolol metabolite and the blood pressure reducing agent may be other than about 1:1, or may be about 1:1. The amount of the active ingredient of the nebivolol metabolite in such a composition will be such that a potentiation of the effects of the blood-pressure reducing agent is obtained; the amount of the blood pressure reducing agent will be such that when potentiated, a blood pressure reducing effect is obtained upon administration. In particular, it is contemplated that the molar ratio of the compound of formula (I) to the blood pressure reducing compound may be situated between about 50:1 and about 1:50, in particular between about 20:1 and about 1:20, or between about 10:1 and about 1:10, or between about 5:1 and about 1:5, more particularly between about 2:1 and about 1:2. Particular such compositions are those wherein the blood pressure reducing agent is one of the agents pertaining to the patents cited hereinabove, and more particularly the agents specifically mentioned herein.

The present disclosure also provides a composition comprising a pharmaceutically acceptable carrier and, as active ingredient, an amount capable of potentiating the effects of blood pressure reducing agents of a nebivolol metabolite of the present disclosure or a pharmaceutically acceptable acid-addition salt thereof, as defined herein.

To prepare such pharmaceutical compositions, an effective amount of the particular compound or compounds, in base or acid-addition salt form, as the active ingredient or active ingredients is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, maybe included. Injectable solutions, for example, maybe prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of the nebivolol metabolites of the present disclosure, due to their increased water solubility over the corresponding base form, are obviously more suitable in the preparation of aqueous compositions.

The present disclosure also concerns a method of potentiating the effects of blood pressure reducing agents in warm-blooded animals in need of blood pressure reducing medication, said method comprising administering to said warm-blooded animals of an effective amount of a blood pressure reducing agent and a nebivolol metabolite of the present disclosure, as defined hereinabove.

Or alternatively, the present disclosure concerns a method of lowering the blood pressure in warm-blooded animals suffering therefrom, said method comprising administering to said warm-blooded animals of an effective amount of a blood pressure reducing agent and a nebivolol metabolite as defined hereinabove.

Those of skill in treating subjects suffering from an increased blood pressure could easily determine the safe and effective amount from the test results presented hereinafter. In general it is contemplated that an effective daily dose of the nebivolol metabolites of the present disclosure or their pharmaceutically acceptable acid-addition salts would be from 0.01 mg/kg to 50 mg/kg body weight, in particular from 0.1 mg/kg to 10 mg/kg body weight, and preferably from 0.1 mg/kg to 1 mg/kg body weight.

### Administration of an oral nebivolol metabolite for delayed-release dosing application

The glucuronidated nebivolol metabolites have a unique absorption profile due to the considerable delay until the metabolite transits the GI-tract to the colon, the site at which the glucuronidated nebivolol metabolite would be liberated. This situation provides an inherent delayed delivery of the metabolite into the blood stream. Therefore, with this in mind, such a formulation could be administered at bedtime, on chronotherapeutic grounds, such that effects of maximum blood levels of the glucuronidated metabolites and associated metabolites would be present upon waking, since this is the time of day associated with greater cardiac-related incidents. Such a dosing regime and rationale is used for verapamil formulations and are envisioned for use with this nebivolol metabolite.

### Nebivolol glucuronidated metabolites and nitric oxide release from normotensive Black American and White Donors

Nebivolol is a β₁-selective agent that stimulates endothelial-dependent nitric oxide (NO). This is an important property as loss in NO bioavailability has been linked to hypertension and increased susceptibility to cardiovascular disease, especially among black Americans. ("Black" refers to a person of African descent or an African-American person but is not necessarily limited to those of African origin (e.g., Carribean)).

With a higher prevalence of cardiovascular risk factors, especially hypertension observed among African Americans in the United States,¹ it has been proposed that these increased CV risk among African Americans may be attributed to differences in vascular physiology, including reduced NO bioavailability. In support of this concept, a clinical evaluation of brachial artery activity demonstrated reduced responsiveness of conductance vessels to both endogenous and exogenous NO in healthy black Americans, as compared with age-matched whites.² To understand the basis for this difference, it was reported that there is low bioavailability of NO from endothelium of black Americans, despite much higher levels of endothelial-dependent NO synthase (eNOS).³ The cellular basis for this paradox was the finding that excessive O₂⁻generation by NAD(P)H -oxidase and uncoupled eNOS resulted in the loss of functional NO due to its reactivity with O₂⁻, resulting in peroxynitrite (ONOO⁻) formation, a potent oxidant.

The acute effects of nebivolol racemate, its separate enantiomers, and four stereoselective glucuronide metabolites on endothelial-dependent NO release were tested in Human Umbilical Vein Endothelial Cells (HUVEC). The tissue is isolated from normotensive white and black American donors with matched risk factor profiles. Production of NO is recorded using electrochemical nanosensors following *acute* treatment with the drugs at 1.0 µM (n=5).

Acute treatment with four stereoselective glucuronide metabolites of nebivolol show a marked improvement in NO release in endothelial cells from both white and black American donors. All four stereoselective glucuronide metabolites have activity. In general, the NO release activities of these metabolites is relatively similar with acute treatment.

These data demonstrate that stereoselective glucuronide metabolites of nebivolol are stimulants of NO release in human endothelial cells from both white and black American donors and these findings indicate that glucuronide metabolites of nebivolol have significant and highly reproducible effects on acute NO release from human endothelial cells.

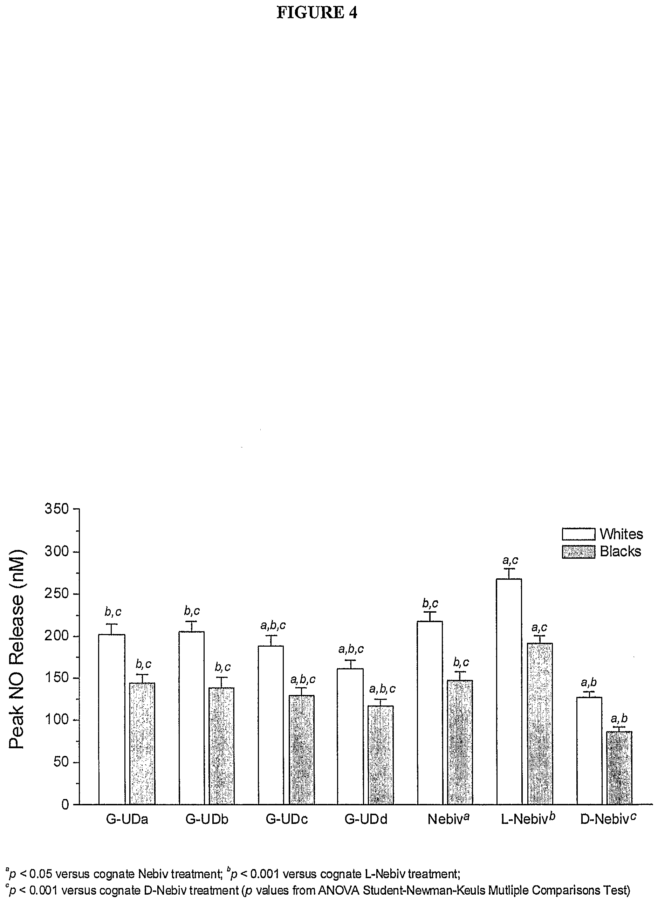

Table 1 and Figure 4 show a marked improvement in NO release following *acute* treatment with nebivolol, *d*-nebivolol, *l*-nebivolol and four stereoselective glucuronide metabolites in endothelial cells from both white and black American donors. (the data in Table I was obtained with HUVEC samples isolated into primary cultures from 12 white and 12 black American female donors by Clonetics (San Diego, California) and purchased as proliferating cells. All cell culture donors were healthy, with no pregnancy or prenatal complications. There were not meaningful differences in the contraceptives used between black and white: approximately 50% of blacks and 60% of white donors did not use contraceptives at least 2 months prior to conception. None of the donors took any drugs regularly and all were nonsmokers and consumed regular caloric/content diet. The donors were between 20-25 years of age, with a mean body mass index (in kg/m²), of 22.6 ± 1.4 for white donors and 23.1 ± 1.7 for black donors. The identification of race was defined by self report. For Figures 1-3, the HUVEC samples were obtained from American Type Culture Collection (Manassas, VA).

**Table 1: Acute NO Release with Compounds**

| **Name** | **White Donors NO (nM)** | **Black Donors NO (nM)** |
|---|---|---|
| G-UDa, *d*-Nebivolol-11-O-β-D-glucuronide | **202 ± 12** | **144 ± 10** |
| G-UDb, *l*-Nebivolol-11-O-β-D-glucuronide | **206 ± 12** | **139 ± 12** |
| G-UDc, *d*-Nebivolol-11'-O-β-D-glucuronide | **188 ± 12** | **129 ± 10** |
| G-UDd, *l*-Nebivolol-11'-O-β-D-glucuronide | **161 ± 11** | **117 ± 8** |
| *l*-nebivolol | **268 ± 12** | **192 ± 9** |
| nebivolol | **218 ± 11** | **148 ± 10** |
| *d*-nebivolol | **127 ± 17** | **87 ± 6** |

**Table 2 Nomenclature for Metabolites**

| **Name** | **Assigned ID** |
|---|---|
| G-UDa, *d*-Nebivolol-11-O-β-D-glucuronide | **G-UDa** |
| G-UDb, *l*-Nebivolol-11-O-β-D-glucuronide | **G-UDb** |
| G-UDc, *d*-Nebivolol-11'-O-β-D-glucuronide | **G**-**UDc** |
| G-UDd, *l*-Nebivolol-11'-O-β-D-glucuronide | **G**-**UDd** |

**Table 3** shows the effects of nebivolol and its glucuronide metabolites on the release of OONO- (peroxynitrite) from human endothelial cells. The findings are similar to the results with NO release, with the greatest activity associated with G-UDa, *d*-Nebivolol-11-O-β-D-glucuronide and G-UDb, *l*-Nebivolol-11-O-β-D-glucuronide. However, the amount of OONO- produced for any of the metabolites was less than that observed for nebivolol. The ratio of NO to OONO⁻ produced by nebivolol and its glucuronide metabolites was similar following acute administration (**Table 4**).

**Table 3: Acute ONOC⁻ Release with Compounds**

| **Name** | **White Donors ONOO⁻ (nM)** | **Black Donors ONOO⁻ (nM)** |
|---|---|---|
| G-UDa, *d*-Nebivolol-11-O-β-D-glucuronide | **180 ± 16** | **238 ± 22** |
| G-UDb, *l*-Nebivolol-11-O-β-D-glucuronide | **180 ± 13** | **225 ± 16** |
| G-UDc, *d*-Nebivolol-11'-O-β-D-glucuronide | **166 ± 13** | **204 ± 13** |
| G-UDd, *l*-Nebivolol-11'-O-β-D-glucuronide | **135 ± 13** | **193 ± 17** |
| *l*-nebivolol | **223 ± 17** | **267 ± 13** |
| nebivolol | **193 ± 16** | **242 ± 17** |
| *d*-nebivolol | **87 ± 6** | **116 ± 6** |

**Table 4: Acute [NO]/[ONOO⁻] Release Ratio**

| **Name** | **White Donors** | **Black Donors** |
|---|---|---|
| G-UDa, *d*-Nebivolol-11-O-β-D-glucuronide | **1.122** | **0.605** |
| G-UDb, *l*-Nebivolol-11-O-β-D-glucuronide | **1.144** | **0.618** |
| G-UDc, *d*-Nebivolol-11'-O-β-D-glucuronide | **1.133** | **0.632** |
| G-UDd, *l*-Nebivolol-11-O-β-D-glucuronide | **1.193** | **0.606** |
| *l*-nebivolol | **1.202** | **0.719** |
| nebivolol | **1.130** | **0.612** |
| *d*-nebivolol | **1.460** | **0.750** |

### Other Pharmacological Examples

### Methods and Materials: Measurements of NO Release from Human Endothelium

All measurements presented are *recorded in vitro* using a sensitive porphyrinic probe. NO release is measured directly from Human Umbilical Vein Endothelial Cells (HUVEC). HUVEC are obtained from American Type Culture Collection (Manassas, VA) and grown in Ham's F12K medium with 2mM L-glutamine adjusted to contain 1.5g/l sodium bicarbonate and supplemented with 0.1mg/ml heparin and 0.03-0.05mg/ml endothelial cell growth supplement (ECGS) = 10% fetal bovine serum. The HUVEC were kept in an atmosphere of elevated CO₂ concentration (5%). Nebivolol, its enantiomers, and nebivolol metabolites including the nebivolol glucuronides, 4-hydroxy-5'phenol metabolite, 4-hydroxy-8'-phenol metabolite, 4-hydroxy, 5-hydroxy, 8-hydroxy metabolites, N-DAD, N-DACA and N-DAAA, are obtained from Mylan Laboratories (Morgantown, WV.)

All measurements of endothelial NO release are conducted in Hank's balance solution at 37°C. Cell wells are transferred to a Faraday cage and a porphyrinic sensor (diameter 0.5mm) is positioned at a distance of 5±2 µm from the surface of the endothelial cells using an inverted microscope (Leica Microsystems, Wetzler, Germany) and a computer-assisted micromanipulator. The sensor operates with a three-electrode system: nanosensor (working electrode), saturated calomel electrode (reference electrode) and platinum wire (counter electrode, 0.5mm diameter). The three electrodes are connected to a potentiostat/galvanostat PAR273. The baseline is stabilized after about 20 seconds. The test compounds are injected with a nanoinjector on to the surface of the cells following solubilization in buffer. The current proportional to the NO concentration is measured with the sensor, which operates in amperometric mode at a constant potential of 0.63V. Data are acquired with the use of an IBM computer with custom software and amperograms (current vs. time curves) and are recorded with a Guniry FAS1 Femtostat (Warminster, PA).

Nanosensors are prepared from carbon fibers. The size of the tip of carbon fiber is reduced from 6 µm to less than 1 µm by temperature controlled burning. The sensors are sensitized to NO by deposition of electrically conductive polymeric porphyrin and covered with a thin layer of Nafion. The prophyrinic microsensor has a response time of 0.1ms at a micromolar NO concentration and 10ms at the detection limit of 1 nM.

The nanosensor for NO is calibrated using saturated solution (concentration 1.82mM verified with the coulometric method). Linear calibration curves are constructed for each sensor from 5 x to 3 x NO before and after measurements of cell activity. The data are presented as the mean ±SEM for each of the triplicate measurements. The data (calculation and plotting) are transferred to Microcal Origin Software (OriginLab Corp., Northampton, MA).

The HUVEC preparation is stable over the course of these experiments with the cells remaining viable in culture for >24 hours. Under non-stimulating conditions, basal levels of NO release are very low (<30nM). Measurement of NO release as a function of treatment is conducted in individual endothelial cells. Multiple measurements of NO release can be conducted on single cells following a brief refractory period. For robust statistical analysis, separate cells are used for each concentration and type of drug used in these analyses. Any apparent inconsistencies in the data may be attributable to the nebivolol sample used. Earlier studies employed a mixture of the four nebivolol glucuronide metabolites, not necessarily in the ratios or concentrations observed in the body. Later studies incorporate the individually isolated/purified nebivolol glucuronide metabolites.

### Comparative Effects of Acute Treatment with Nebivolol Metabolites on NO Release from Human Endothelial Cells

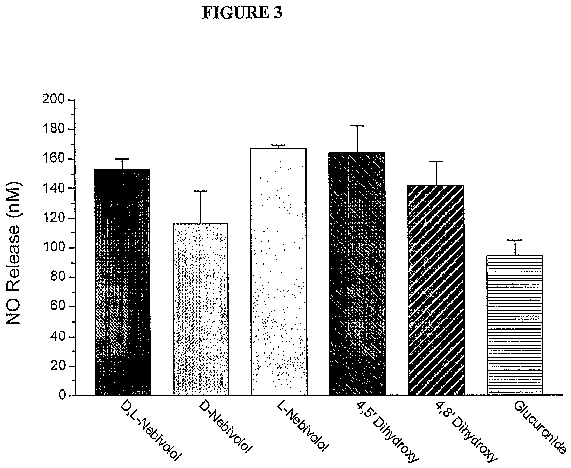

The results of these analyses demonstrate that six metabolites of the β₁-adrenoceptor-selective antagonist nebivolol have different effects on NO release from human endothelial cells over a range of concentrations. FIGURES 1 and 3 compare peak NO release from HUVEC preparations for the three metabolites following addition of the agents at concentrations of 50µM and 500 nM, respectively. The dihydroxy nebivolol metabolites, including the 4-hydroxy-5'-phenol and 4-hydroxy-8'-phenol as well as glucuronidated metabolites are all active in regards to NO releasing capacity.

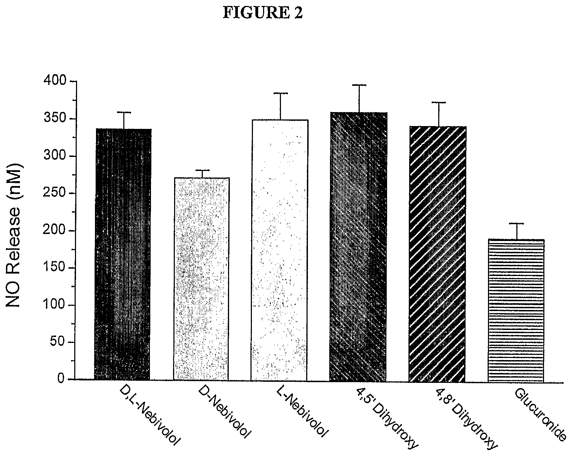

The NO releasing effects of certain nebivolol metabolites on NO release following acute treatment are compared in FIGURE 2. Note that Figure measures the dose dependency of the metabolites. These data indicate that while the dihydroxy metabolites increase the concentration of NO to levels similar to that of nebivolol and its enantiomers at pharmacologic-like levels, the glucuronidated metabolites also show activity, albeit these are less active than the glucuronidated counterparts.

### References:

1. Burt VL, Whelton P, Roccella EJ, Brown C, Cutler JA, Higgins M, Horan MJ, Labarthe D. Prevalence of hypertension in the US adult population: Results from the Third National Health and Nutrition Examination Survey, 1988-1991. Hypertension. 1995;25:305-313.
2 Campia U, Choucair WK, Bryant MB, Waclawiw MA, Cardillo C, Panza JA. Reduced endothelium-dependent and -independent dilation of conductance arteries in African Americans. J. Am. Coll. Cardiol. 2002;40:754-760.
3. Kalinowski L, Dobrucki I, Malinski T. Race-specific differences in endothelial function: Predisposition of African Americans to vascular diseases. Circulation. 2004;109:2511-2517.

## Claims

1. At least one diastereomer of a glucuronidated nebivolol metabolite having a formula below: 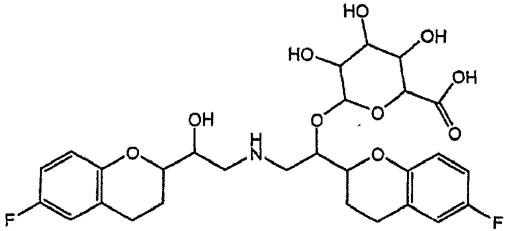 for use as a medicament in treating and/or preventing cardiovascular disease.

2. The diastereomer for use of claim 1 wherein said cardiovascular disease is selected from the group consisting of congestive heart failure, hypertension, pulmonary hypertension, myocardial and cerebral infarctions, atherosclerosis, atherogenesis, thrombosis, ischemic heart disease, post- angioplasty restenosis, coronary artery diseases, renal failure, stable, unstable and variant (Prinzmetal) angina, cardiac edema, renal insufficiency, nephrotic edema, hepatic edema, stroke, transient ischemic attacks, cerebrovascular accidents, restenosis, controlling blood pressure in hypertension, platelet adhesion, platelet aggregation, smooth muscle cell proliferation, pulmonary edema, vascular complications associated with the use of medical devices including stents, pulmonary thromboembolism, cerebral thromboembolism, thrombophlebitis, thrombocytopenia and bleeding disorders.

3. The diastereomer for use of claim 1 or 2 wherein the cardiovascular disease is selected from the group consisting of congestive heart failure, hypertension, restenosis and atherosclerosis.

4. The diastereomer for use of any of claims 1 to 3 wherein the medicament is administered intravenously, orally, bucally, parenterally, by inhalation, or transdermally.

5. A pharmaceutical composition comprising at least one diastereoisomer of a glucuronidated nebivolol metabolite having a formula below: 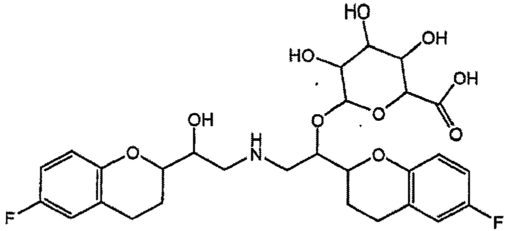 or a pharmaceutically acceptable salt thereof and a pharmaceutically-acceptable carrier.

6. The pharmaceutical composition of claim 5, further comprising at least one additional cardiovascular agent.

7. The pharmaceutical composition of claim 6 comprising only one cardiovascular agent.

8. The pharmaceutical composition of claims 6 and 7 wherein the cardiovascular agent is selected from the group consisting of angiotensin II receptor antagonists (ARB's).

9. The pharmaceutical composition of claim 8 wherein the ARB is selected from the group consisting of olmesartan, candesartan, eprosartan, irbesartan, losartan, valsartan, and mixtures thereof.

10. The pharmaceutical composition of any of claims 5 to 9 for use as a medicament in treating and/or preventing cardiovascular disease.

11. The pharmaceutical composition for use of claim 10 wherein said cardiovascular disease is selected from the group consisting of congestive heart failure, hypertension, pulmonary hypertension, myocardial and cerebral infarctions, atherosclerosis, atherogenesis, thrombosis, ischemic heart disease, post- angioplasty restenosis, coronary artery diseases, renal failure, stable, unstable and variant (Prinzmetal) angina, cardiac edema, renal insufficiency, nephrotic edema, hepatic edema, stroke, transient ischemic attacks, cerebrovascular accidents, restenosis, controlling blood pressure in hypertension, platelet adhesion, platelet aggregation, smooth muscle cell proliferation, pulmonary edema, vascular complications associated with the use of medical devices including stents, pulmonary thromboembolism, cerebral thromboembolism, thrombophlebitis, thrombocytopenia and bleeding disorders.

12. The pharmaceutical composition for use of claim 10 or 11 wherein the cardiovascular disease is selected from the group consisting of congestive heart failure, hypertension, restenosis and atherosclerosis.

13. The pharmaceutical composition for use of claim 10 to 12 wherein the medicament is in dosage unit form, wherein each unit contains a predetermined quantity of the nebivolol metabolite or metabolites to produce the desired therapeutic effect, in association with a pharmaceutical carrier wherein the composition is administered intravenously, orally, bucally, parenterally, by inhalation; or transdermally.

## Patentansprüche

1. Mindestens ein Diastereomer eines glukuronidierten Nebivolol-Metabolits mit einer Formel: 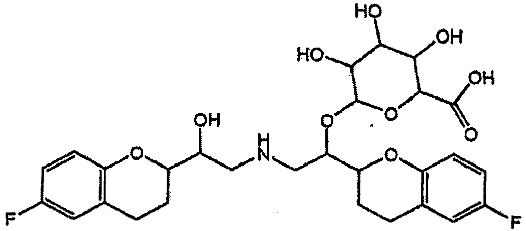 zur Verwendung als ein Medikament beim Behandeln und/oder Verhüten einer Herz-Kreislauf-Erkrankung.

2. Diastereomer zur Verwendung nach Anspruch 1, wobei die Herz-Kreislauf-Erkrankung ausgewählt ist aus der Gruppe bestehend aus hydropischer Herzdekompensation, Bluthochdruck, pulmonale Hypertonie, Herz- und Hirninfarkte, Arteriosklerose, Atherogenese, Thrombose, ischämische Herzerkrankung, Post-Angioplastie-Restenose, koronare Arterienkrankheiten, Niereninsuffizienz, stabile, instabile und Variant- (Prinzmetal) -Angina, kardiales Ödem, Niereninsuffizienz, nephrotisches Ödem, hepatogenes Ödem, Schlaganfall, transitorische ischämische Attacken, Schlaganfälle, Restenose, Kontrollieren von Blutdruck bei Bluthochdruck, Blutplättchenadhäsion, Blutplättchenaggregation, Proliferation glatter Muskelzellen, Lungenödem, vaskuläre Komplikationen, die mit der Verwendung von medizinischen Vorrichtungen einschließlich Stents verbunden sind, pulmonale Thromboembolie, Gehirnthromboembolie, Thrombophlebitis, Thrombozytopenie und Blutungserkrankungen.

3. Diastereomer zur Verwendung nach Anspruch 1 oder 2, wobei die Herz-Kreislauf-Erkrankung ausgewählt ist aus der Gruppe bestehend aus hydropischer Herzdekompensation, Bluthochdruck, Restenose und Arteriosklerose.

4. Diastereomer zur Verwendung nach einem der Ansprüche 1 bis 3, wobei das Medikament intravenös, oral, bukkal, parenteral, durch Inhalation oder transdermal verabreicht wird.

5. Pharmazeutische Zusammensetzung, die mindestens ein Diastereoisomer eines glukuronidierten Nebivolol-Metabolits umfasst mit einer Formel: 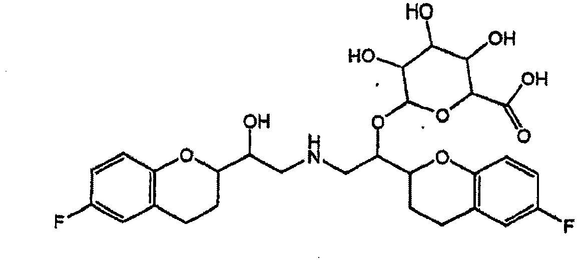 oder ein pharmazeutisch verträgliches Salz davon und ein pharmazeutisch verträglicher Träger.

6. Pharmazeutische Zusammensetzung nach Anspruch 5, weiter umfassend mindestens ein zusätzliches kardiovaskuläres Mittel.

7. Pharmazeutische Zusammensetzung nach Anspruch 6, umfassend nur ein kardiovaskuläres Mittel.

8. Pharmazeutische Zusammensetzung nach den Ansprüchen 6 und 7, wobei das kardiovaskuläre Mittel ausgewählt ist aus der Gruppe bestehend aus Angiotensin-II-Rezeptor-Antagonisten (ARB's).

9. Pharmazeutische Zusammensetzung nach Anspruch 8, wobei der ARB ausgewählt ist aus der Gruppe bestehend aus Olmesartan, Candesartan, Eprosartan, Irbesartan, Losartan, Valsartan und Mischungen davon.

10. Pharmazeutische Zusammensetzung nach einem der Ansprüche 5 bis 9 zur Verwendung als ein Medikament beim Behandeln und/oder Verhüten einer Herz-Kreislauf-Erkrankung.

11. Pharmazeutische Zusammensetzung zur Verwendung nach Anspruch 10, wobei die Herz-Kreislauf-Erkrankung ausgewählt ist aus der Gruppe bestehend aus hydropischer Herzdekompensation, Bluthochdruck, pulmonale Hypertonie, Herz- und Hirninfarkte, Arteriosklerose, Atherogenese, Thrombose, ischämische Herzerkrankung, Post-Angioplastie-Restenose, koronare Arterienkrankheiten, Niereninsuffizienz, stabile, instabile und Variant- (Prinzmetal) -Angina, kardiales Ödem, Niereninsuffizienz, nephrotisches Ödem, hepatogenes Ödem, Schlaganfall, transitorische ischämische Attacken, Schlaganfälle, Restenose, Kontrollieren von Blutdruck bei Bluthochdruck, Blutplättchenadhäsion, Blutplättchenaggregation, Proliferation glatter Muskelzellen, Lungenödem, vaskuläre Komplikationen, die mit der Verwendung von medizinischen Vorrichtungen einschließlich Stents verbunden sind, pulmonale Thromboembolie, Gehirnthromboembolie, Thrombophlebitis, Thrombozytopenie und Blutungserkrankungen.

12. Pharmazeutische Zusammensetzung zur Verwendung nach Anspruch 10 oder 11, wobei die Herz-Kreislauf-Erkrankung ausgewählt ist aus der Gruppe bestehend aus hydropischer Herzdekompensation, Hypertension, Restenose und Arteriosklerose.

13. Pharmazeutische Zusammensetzung zur Verwendung nach Anspruch 10 bis 12, wobei das Medikament Dosierungseinheitsform aufweist, und wobei jede Einheit eine vorgegebene Menge an Nebivolol-Metabolit oder -Metaboliten enthält, um die gewünschte Heilwirkung in Verbindung mit einem pharmazeutischen Träger zu erzeugen, wobei die Zusammensetzung intravenös, oral, bukkal, parenteral, durch Inhalation; oder transdermal verabreicht wird.

## Revendications

1. Au moins un diastéréomère d'un métabolite de nébivolol glycuroconjugué ayant la formule ci-dessous : 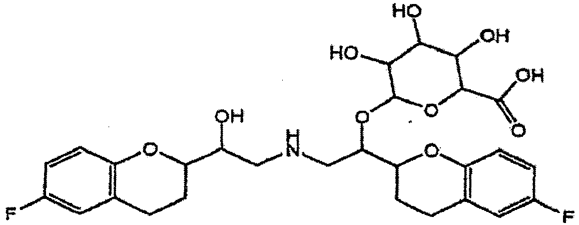 destiné à être utilisé en tant que médicament dans le traitement et/ou la prévention d'une maladie cardiovasculaire.

2. Diastéréomère destiné à être utilisé selon la revendication 1, dans lequel ladite maladie cardiovasculaire est sélectionnée dans le groupe constitué par l'insuffisance cardiaque congestive, l'hypertension, l'hypertension pulmonaire, l'infarctus du myocarde et l'infarctus cérébral, l'athérosclérose, l'athérogénèse, la thrombose, la cardiopathie ischémique, la resténose post-angioplastie, les maladies coronariennes, l'insuffisance rénale, l'angine stable, l'angine instable et l'angor spastique (de Prinzmetal), l'oedème cardiaque, l'insuffisance rénale, l'oedème néphrotique, l'oedème hépatique, l'attaque d'apoplexie, les accidents ischémiques transitoires, les accidents cérébrovasculaires, la resténose, la régulation de la pression artérielle en cas d'hypertension, l'adhésion des plaquettes, l'agrégation des plaquettes, la prolifération des cellules musculaires lisses, l'oedème pulmonaire, les complications vasculaires associées à l'utilisation de dispositifs médicaux comportant des endoprothèses, le thromboembolisme pulmonaire, le thromboembolisme cérébral, la thrombophlébite, la thrombocytopénie et les troubles hémostatiques.

3. Diastéréomère destiné à être utilisé selon la revendication 1 ou 2, dans lequel la maladie cardiovasculaire est sélectionnée dans le groupe constitué par l'insuffisance cardiaque congestive, l'hypertension, la resténose et l'athérosclérose.

4. Diastéréomère destiné à être utilisé selon l'une quelconque des revendications 1 à 3, dans lequel le médicament est administré par voie intraveineuse, orale, buccale, parentérale, par inhalation ou par voie transdermique.

5. Composition pharmaceutique comprenant au moins un diastéréomère d'un métabolite de nébivolol glycuroconjugué ayant la formule ci-dessous : 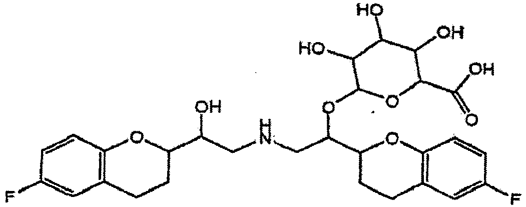 ou un sel pharmaceutiquement acceptable de ce dernier et un vecteur pharmaceutiquement acceptable.

6. Composition pharmaceutique selon la revendication 5, comprenant en outre au moins un agent cardiovasculaire supplémentaire.

7. Composition pharmaceutique selon la revendication 6, comprenant seulement un agent cardiovasculaire.

8. Composition pharmaceutique selon les revendications 6 et 7, dans laquelle l'agent cardiovasculaire est sélectionné dans le groupe constitué par les antagonistes des récepteurs de l'angiotensine II (ARB).

9. Composition pharmaceutique selon la revendication 8, dans laquelle l'antagoniste ARB est sélectionné dans le groupe constitué par l'olmésartan, le candésartan, l'éprosartan, l'irbésartan, le losartan, le valsartan et des mélanges de ces derniers.

10. Composition pharmaceutique selon l'une quelconque des revendications 5 à 9 destinée à être utilisée en tant que médicament dans le traitement et/ou la prévention d'une maladie cardiovasculaire.

11. Composition pharmaceutique destinée à être utilisée selon la revendication 10, dans laquelle ladite maladie cardiovasculaire est sélectionnée dans le groupe constitué par l'insuffisance cardiaque congestive, l'hypertension, l'hypertension pulmonaire, l'infarctus du myocarde et l'infarctus cérébral, l'athérosclérose, l'athérogénèse, la thrombose, la cardiopathie ischémique, la resténose post-angioplastie, les maladies coronariennes, l'insuffisance rénale, l'angine stable, l'angine instable et l'angor spastique (de Prinzmetal), l'oedème cardiaque, l'insuffisance rénale, l'oedème néphrotique, l'oedème hépatique, l'attaque d'apoplexie, les accidents ischémiques transitoires, les accidents cérébrovasculaires, la resténose, la régulation de la pression artérielle en cas d'hypertension, l'adhésion des plaquettes, l'agrégation des plaquettes, la prolifération des cellules musculaires lisses, l'oedème pulmonaire, les complications vasculaires associées à l'utilisation de dispositifs médicaux comportant des endoprothèses, le thromboembolisme pulmonaire, le thromboembolisme cérébral, la thrombophlébite, la thrombocytopénie et les troubles hémostatiques.

12. Composition pharmaceutique destinée à être utilisée selon la revendication 10 ou 11, dans laquelle la maladie cardiovasculaire est sélectionnée dans le groupe constitué par l'insuffisance cardiaque congestive, l'hypertension, la resténose et l'athérosclérose.

13. Composition pharmaceutique destinée à être utilisée selon la revendication 10 à 12, dans laquelle le médicament est sous une forme posologique unitaire, dans laquelle chaque unité contient une quantité prédéterminée de métabolite, ou de métabolites, de nébivolol pour produire l'effet thérapeutique souhaité, en association avec un vecteur pharmaceutique dans lequel la composition est administrée par voie intraveineuse, orale, buccale, parentérale, par inhalation ou par voie transdermique.
